# EUROPEAN PATENT APPLICATION

(11) **EP 2 395 109 A1**
(43) Date of publication of application: **14.12.2011**
(21) Application number: 11003918.7
(22) Date of filing: 01.12.2005
(51) Int. Cl.: C12Q 1/68, G01N 33/574

(54) **Method for measuring resistance or sensitivity to docetaxel**

(30) Priority: 08.12.2004 US 634298 P
(62) Divisional of application: 05852717.7
(71) Applicant: Aventis Pharmaceuticals Inc., Bridgewater, New Jersey 08807 (US)
(72) Inventor: Grueneberg, Dorre, Newtonville MA 02460 (US); Huang, Xi, Arlington MA 02474 (US); Natesan, Sridaran, Ashland MA 01721 (US); August, Paul, Boxford MA 01921 (US)
(74) Representative: Körner, Kathrin

(57) **Abstract**

The present invention relates to novel and useful methods that predict or monitor a patient's response to a molecule of the taxoid family by measuring the increase or decrease of specific genetic markers, in particular STK11, as compared to controls. The present invention also provides kits that predict of monitor patient's response to a molecule of the taxoid family by measuring nucleic acid or protein levels of particular genetic markers and comparing their levels to controls or reference markers.

## Description

### FIELD OF THE INVENTION

The present invention relates to novel, useful and heretofore unknown methods that may predict or monitor a patient's response to a molecule of the taxoid family by measuring the increase or decrease of specific genetic markers as compared to controls. The present invention also provides kits that predict or monitor patients' response to a molecule of the taxoid family by measuring nucleic acid or protein levels of particular genetic markers and comparing their levels to controls or reference markers.

### BACKGROUND OF THE INVENTON

Docetaxel is an anti-mitotic drug widely used for the treatment of breast, lung and ovarian cancer, and to a lesser extent used for treating head and neck, gastric and prostate carcinomas (Hong, Oncology 16:9, 2002). Docetaxel inhibits microtubule dynamics by binding to beta-tubulin and blocking disassembly of alpha- and beta-tubulin heterodimers thus abrogating tumor growth (Ringel and Horwitz, J Natl Cancer Inst 83:288, 1991). The anti-tumor activities of docetaxel and a related taxane, paclitaxel arise from targeting microtubules of the mitotic spindle to impede chromosome alignment and segregation, block cell cycle progression and activate apoptosis pathways (Wang et al., Cancer 88:2619, 2000). The proapoptotic activities of paclitaxel have been linked with the phosphorylation and inactivation of Bc1-2 through cell signaling events that involve p53/p21waf1/Cip1, raf/ras and mitogen-activated protein kinases (MAPKs) (Wang et al., Cancer 88:2619, 2000). Although docetaxel is a more potent anti-cancer agent than paclitaxel, the pathways involved in its cytotoxicity are less well defined (Katsumata, Br J Cancer 89:S9, 2003). Docetaxel-induced apoptosis has been observed in select cell lines through a mechanism involving Bcl-2 phosphorylation and caspase-3 activation (Kolfschoten et al., Biochem Pharmacol 63:733, 2002). Other proteins that modulate taxane-induced cell killing in different cultured lines include Aurora-A in HeLa cells (Anand et al., Cancer Cell 3:51, 2003), HER-2 in breast cancer cells (Tanabe et al., Int J Oncol 22:875, 2003), p21waf1/Cip1 in glioblastoma cells (Li et al., J Biol Chem 277:11352, 2002) and JNK/MKK1 in ovarian cancer cells (Lee et al., J Biol Chem 273:28253, 1998).

There remains a need in the field to identify drugable proteins that mediate docetaxel resistance, which could lead to their utilization in drug discovery to identify reagents (small molecules, siRNA, etc.) that abrogate their in vivo function and potentially sensitize cells to anti-tumor drug chemotherapy. There also remains a need in the field for an assay which could confidently predict in advance of chemotherapy treatment which patients would and would not respond to anti-tumor docetaxel drug based chemotherapy. Applicants describe herein a novel assay that can predict resistance and or sensitivity to docetaxel and provide screening assays for the development of new therapies to abrogate drug resistance in cancer.

### SUMMARY OF THE INVENTION

In accordance with the present invention, there is provided novel and useful methods to monitor and/or predict a patient's response to a molecule of the taxoid family by comparing the levels of activation and/or expression of specific genetic markers in patients and controls.

In one embodiment, the present invention provides a method for predicting or monitoring a cancer patient's response to a molecule of the taxoid family, comprising the steps of:
a) obtaining a test sample from a cancerous area of said patient;
b) obtaining a control sample;
c) measuring the level of one or more genetic markers; and
d) comparing the measured levels of said one or more genetic markers in the test sample and the control sample;
   wherein a decrease in the level of said one or more genetic markers measured in the test sample as compared to the control sample indicates an increased resistance to a molecule of the taxoid family.

Particular genetic markers provided in this aspect of the present invention include:
BubR1, Homo sapiens similar to protein kinase (BUBR1) mRNA, complete cds (GenBank accession number: AF046079);
Mad2, Homo sapiens mRNA for MAD2 protein (GenBank accession number: AJ000186);
Mps1, Homo sapiens TTK protein kinase (TTK), mRNA (GenBank accession number: NM_003318);
GEFT for Rac1/CDC42, Homo sapiens RAC/CDC42 exchange factor (GEFT), transcript variant 2, mRNA (GenBank accession number: NM_133483);
Bub1, Homo sapiens BUB1 budding uninhibited by benzimidazoles 1 homolog (yeast) (BUB1), mRNA (GenBank accession number: NM_004336);
hSepharase, Homo sapiens extra spindle poles like 1 (S. cerevisiae) (ESPL1), mRNA (GenBank accession number: NM_012291);
CamKIId, Homo sapiens calcium/calmodulin-dependent protein kinase (CaM kinase) II delta (CAMK2D), transcript variant 3, mRNA (GenBank accession number: NM_001221);
CDK6, Homo sapiens cyclin-dependent kinase 6 (CDK6), mRNA (GenBank accession number: NM_001259); and
GRB2, Homo sapiens growth factor receptor-bound protein 2 (GRB2), transcript variant 1, mRNA (GenBank accession number: NM_002086).

In another embodiment, the present invention relates to a method for predicting or monitoring a cancer patient's response to a molecule of the taxoid family, comprising the steps of:
a) obtaining a test sample from a cancerous area of said patient;
b) obtaining a control sample;
c) measuring the level of one or more genetic markers; and
d) comparing the measured levels of said one or more genetic markers in the test sample and the control sample;
   wherein a decrease in the level of said one or more genetic markers measured in the test sample as compared to the control sample indicates an increased sensitivity to a molecule of the taxoid family.

In this particular aspect of the invention one or more genetic markers may be selected from the group consisting of:
P21(Waf1), Homo sapiens cyclin-dependent kinase inhibitor 1A (p21, Cip1) (CDKN1A), transcript variant 1, mRNA (GenBank accession number: NM_000389);
Pim-1, Homo sapiens pim-1 oncogene (PIM1), mRNA (GenBank accession number: NM_002648);
GBP-1, Homo sapiens guanylate binding protein 1, interferon-inducible, 67kDa (GBP1), mRNA (GenBank accession number: NM_002053);
RXRA, Homo sapiens retinoid X receptor, alpha (RXRA), mRNA (GenBank accession number: NM_002957);
SPF45, Homo sapiens RNA binding motif protein 17 (RBM17), mRNA (GenBank accession number: NM_032905);
Hec1, Homo sapiens kinetochore associated 2 (KNTC2), mRNA (GenBank accession number: NM_006101);
Raf1, Human mRNA for raf oncogene (GenBank accession number: X03484);
Aurora A, Homo sapiens aurora-related kinase 1 (ARK1) mRNA, complete cds (GenBank accession number: AF008551);
TACC3, Homo sapiens transforming, acidic coiled-coil containing protein 3 (TACC3), mRNA (GenBank accession number: NM_006342);
RelB, Homo sapiens v-rel reticuloendotheliosis viral oncogene homolog B, nuclear factor of kappa light polypeptide gene enhancer in B-cells 3 (avian) (RELB), mRNA (GenBank accession number: NM_006509);
PRKCD, Homo sapiens protein kinase C, delta (PRKCD), transcript variant 1, mRNA (GenBank accession number: NM_006254);
BRAF35, Homo sapiens high-mobility group 20B (HMG20B), mRNA (GenBank accession number: NM_006339);
HSPA1L, Homo sapiens heat shock 70kDa protein 1A (HSPA1A), mRNA (GenBank accession number: NM_005345);
STK11, Homo sapiens serine/threonine kinase 11 (Peutz-Jeghers syndrome) (STK11), mRNA (GenBank accession number: NM_000455); and
MKK3, Homo sapiens MAP kinase kinase 3 (MKK3) mRNA, complete cds (GenBank accession number: L36719).

In yet a further embodiment, the present invention relates to a method for predicting or monitoring a cancer patient's response to a molecule of the taxoid family, comprising the steps of:
a) obtaining a test sample from a cancerous area of said patient;
b) measuring the level of one or more genetic markers selected from the group consisting of:
   BubR1, Homo sapiens similar to protein kinase (BUBR1) mRNA, complete cds (GenBank accession number: AF046079);
   Mad2, Homo sapiens mRNA for MAD2 protein (GenBank accession number: AJ000186);
   Mps1, Homo sapiens TTK protein kinase (TTK), mRNA (GenBank accession number: NM_003318);
   GEFT for Rac1/CDC42, Homo sapiens RAC/CDC42 exchange factor (GEFT), transcript variant 2, mRNA (GenBank accession number: NM_133483);
   Bub1 Homo sapiens BUB1 budding uninhibited by benzimidazoles 1 homolog (yeast) (BUB1), mRNA (GenBank accession number: NM_004336);
   hSepharase, Homo sapiens extra spindle poles like 1 (S. cerevisiae) (ESPL1), mRNA (GenBank accession number: NM_012291);
   CamKIId, Homo sapiens calcium/calmodulin-dependent protein kinase (CaM kinase) II delta (CAMK2D), transcript variant 3, mRNA (GenBank accession number: NM_001221);
   CDK6, Homo sapiens cyclin-dependent kinase 6 (CDK6), mRNA (GenBank accession number: NM_001259); and
   GRB2, Homo sapiens growth factor receptor-bound protein 2 (GRB2), transcript variant 1, mRNA (GenBank accession number: NM_002086)
c) measuring the level of one or more reference genetic markers selected from the group consisting of:
   GAPDH, Homo sapiens glyceraldehyde-3-phosphate dehydrogenase (GAPD), mRNA (GenBank accession number: NM_002046); and
   RPS9, Homo sapiens cDNA clone IMAGE:6647283, partial cds (GenBank accession number: BC071941);
d) comparing the measured levels of said one or more genetic markers and said one or more reference genetic markers in the test sample;
   wherein a decrease in the level of said one or more genetic markers as compared to the level of said one or more reference genetic markers indicates an increased resistance to a molecule of the taxoid family.

Still yet a further embodiment of the present invention relates to a method for predicting or monitoring a cancer patient's response to a molecule of the taxoid family, comprising the steps of:
a) obtaining a test sample from a cancerous area of said patient;
b) measuring the level of one or more genetic markers selected from the group consisting of:
   P21(Waf1), Homo sapiens cyclin-dependent kinase inhibitor 1A (p21, Cip1) (CDKN1A), transcript variant 1, mRNA (GenBank accession number: NM_000389);
   Pim-1, Homo sapiens pim-1 oncogene (PIM1), mRNA (GenBank accession number: NM_002648);
   GBP-1, Homo sapiens guanylate binding protein 1, interferon-inducible, 67kDa (GBP1), mRNA (GenBank accession number: NM_002053);
   RXRA, Homo sapiens retinoid X receptor, alpha (RXRA), mRNA (GenBank accession number: NM_002957);
   SPF45, Homo sapiens RNA binding motif protein 17 (RBM17), mRNA (GenBank accession number: NM_032905);
   Hec1, Homo sapiens kinetochore associated 2 (KNTC2), mRNA (GenBank accession number: NM_006101);
   Raf1, Human mRNA for raf oncogene (GenBank accession number: X03484);
   Aurora A, Homo sapiens aurora-related kinase 1 (ARK1) mRNA, complete cds (GenBank accession number: AF008551);
   TACC3, Homo sapiens transforming, acidic coiled-coil containing protein 3 (TACC3), mRNA (GenBank accession number: NM_006342);
   RelB, Homo sapiens v-rel reticuloendotheliosis viral oncogene homolog B, nuclear factor of kappa light polypeptide gene enhancer in B-cells 3 (avian) (RELB), mRNA (GenBank accession number: NM_006509);
   PRKCD, Homo sapiens protein kinase C, delta (PRKCD), transcript variant 1, mRNA (GenBank accession number: NM_006254);
   BRAF35, Homo sapiens high-mobility group 20B (HMG20B), mRNA (GenBank accession number: NM_006339);
   HSPA1L, Homo sapiens heat shock 70kDa protein 1A (HSPA1A), mRNA (GenBank accession number: NM_005345);
   STK11, Homo sapiens serine/threonine kinase 11 (Peutz-Jeghers syndrome) (STK11), mRNA (GenBank accession number: NM_000455); and
   MKK3, Homo sapiens MAP kinase kinase 3 (MKK3) mRNA, complete cds (GenBank accession number: L36719);
c) measuring the level of one or more reference genetic markers selected from the group consisting of:
   GAPDH, Homo sapiens glyceraldehyde-3-phosphate dehydrogenase (GAPD), mRNA (GenBank accession number: NM_002046); and
   RPS9, Homo sapiens cDNA clone IMAGE:6647283, partial cds (GenBank accession number: BC071941);
d) comparing the measured levels of said one or more genetic markers and said one or more reference genetic markers in the test sample;
   wherein a decrease in the level of said one or more genetic markers as compared to the level of said one or more reference genetic markers indicates an increased susceptibility to a molecule of the taxoid family.

The present invention relates to methods for predicting or monitoring a cancer patient's response to a molecule of the taxoid family. This, a further embodiment of the present inventions includes the taxoid family molecules paclitaxel, docetaxel XRP9881 and XRP6258

The genetic markers described in the present inventions may be evaluated by measuring the levels of RNA, DNA or protein using a variety of techniques that are described in further detail below. Other embodiments of the present invention relate to kits for predicting or monitoring a patient's response to a molecule of the taxoid family.

The present invention, in particular, relates to the following:
1. A method for predicting or monitoring a cancer patient's response to a molecule of the taxoid family, comprising the steps of:
   a) obtaining a test sample from a cancerous area of said patient;
   b) obtaining a control sample;
   c) measuring the level of one or more genetic markers; and
   d) comparing the measured levels of said one or more genetic markers in the test sample and the control sample;
   wherein a decrease in the level of said one or more genetic markers measured in the test sample as compared to the control sample indicates an increased resistance to a molecule of the taxoid family.
2. The method of aspect 1, wherein said one or more genetic markers is selected from the group consisting of:
   BubR1, Homo sapiens similar to protein kinase (BUBR1) mRNA, complete cds (GenBank accession number: AF046079);
   Mad2, Homo sapiens mRNA for MAD2 protein (GenBank accession number: AJ000186);
   Mps1, Homo sapiens TTK protein kinase (TTK), mRNA (GenBank accession number: NM_003318);
   GEFT for Rac1/CDC42, Homo sapiens RAC/CDC42 exchange factor (GEFT), transcript variant 2, mRNA (GenBank accession number: NM_133483);
   Bub1, Homo sapiens BUB1 budding uninhibited by benzimidazoles 1 homolog (yeast) (BUB1), mRNA (GenBank accession number: NM_004336);
   hSepharase, Homo sapiens extra spindle poles like 1 (S. cerevisiae) (ESPL1), mRNA (GenBank accession number: NM_012291);
   CamKIId, Homo sapiens calcium/calmodulin-dependent protein kinase (CaM kinase) II delta (CAMK2D), transcript variant 3, mRNA (GenBank accession number: NM_001221);
   CDK6, Homo sapiens cyclin-dependent kinase 6 (CDK6), mRNA (GenBank accession number: NM_001259); and
   GRB2, Homo sapiens growth factor receptor-bound protein 2 (GRB2), transcript variant 1, mRNA (GenBank accession number: NM_002086).
3. The method of aspect 1 wherein said molecule of the taxoid family is selected from the group consisting of paclitaxel, docetaxel XRP9881 and XRP6258.
4. The method of aspect 1 wherein said level of one or more genetic markers is measured by mRNA, DNA or protein.
5. The method of aspect 4 wherein said mRNA is measured by using a technique selected from the group consisting of in situ hybridization, reverse-transcriptase polymerase chain reaction, nucleic acid hybridization, electrophoresis, Northern blotting and mass spectrometry.
6. The method of aspect 4 wherein said DNA is measured by using a technique selected from the group consisting of quantitative polymerase chain reaction, genomic DNA-chips, in situ hybridization, electrophoresis, Southern blotting and mass spectrometry.
7. The method of aspect 4 wherein said protein is measured by using a technique selected from the group consisting of immunoassay, Western blots, ELISA, and mass spectrometry.
8. A method for predicting or monitoring a cancer patient's response to a molecule of the taxoid family, comprising the steps of:
   a) obtaining a test sample from a cancerous area of said patient;
   b) obtaining a control sample;
   c) measuring the level of one or more genetic markers; and
   d) comparing the measured levels of said one or more genetic markers in the test sample and the control sample;
   wherein a decrease in the level of said one or more genetic markers measured in the test sample as compared to the control sample indicates an increased sensitivity to a molecule of the taxoid family.
9. The method of aspect 8, wherein said one or more genetic markers is selected from the group consisting of:
   P21(Wafl), Homo sapiens cyclin-dependent kinase inhibitor 1A (p21, Cip1) (CDKN1A), transcript variant 1, mRNA (GenBank accession number: NM_000389);
   Pim-1, Homo sapiens pim-1 oncogene (PIM1), mRNA (GenBank accession number: NM_002648);
   GBP-1, Homo sapiens guanylate binding protein 1, interferon-inducible, 67kDa (GBP1), mRNA (GenBank accession number: NM_002053);
   RXRA, Homo sapiens retinoid X receptor, alpha (RXRA), mRNA (GenBank accession number: NM_002957);
   SPF45, Homo sapiens RNA binding motif protein 17 (RBM17), mRNA (GenBank accession number: NM_032905);
   Hec1, Homo sapiens kinetochore associated 2 (KNTC2), mRNA (GenBank accession number: NM_006101);
   Raf1, Human mRNA for raf oncogene (GenBank accession number: X03484);
   Aurora A, Homo sapiens aurora-related kinase 1 (ARK1) mRNA, complete cds ((GenBank accession number: AF008551);
   TACC3, Homo sapiens transforming, acidic coiled-coil containing protein 3 (TACC3), mRNA (GenBank accession number: M_006342) ;
   RelB, Homo sapiens v-rel reticuloendotheliosis viral oncogene homolog B, nuclear factor of kappa light polypeptide gene enhancer in B-cells 3 (avian) (RELB), mRNA (GenBank accession number: NM_006509);
   PRKCD, Homo sapiens protein kinase C, delta (PRKCD), transcript variant 1, mRNA (GenBank accession number: NM_006254);
   BRAF35, Homo sapiens high-mobility group 20B (HMG20B), mRNA (GenBank accession number: NM_006339);
   HSPA1L, Homo sapiens heat shock 70kDa protein 1A (HSPA1A), mRNA (GenBank accession number: NM_005345);
   STK11, Homo sapiens serine/threonine kinase 11 (Peutz-Jeghers syndrome) (STK11), mRNA (GenBank accession number: NM_000455); and
   MKK3, Homo sapiens MAP kinase kinase 3 (MKK3) mRNA, complete cds (GenBank accession number: L36719).
10. The method of aspect 8 wherein said molecule of the taxoid family is selected from the group consisting of paclitaxel, docetaxel XRP9881 and XRP6258.
11. The method of aspect 8 wherein said level of one or more genetic markers is measured by mRNA, DNA or protein.
12. The method of aspect 11 wherein said mRNA is measured by using a technique selected from the group consisting of in situ hybridization, reverse-transcriptase polymerase chain reaction, nucleic acid hybridization, electrophoresis, Northern blotting and mass spectrometry.
13. The method of aspect 11 wherein said DNA is measured by using a technique selected from the group consisting of quantitative polymerase chain reaction, genomic DNA-chips, in situ hybridization, electrophoresis, Southern blotting and mass spectrometry.
14. The method of aspect 11 wherein said protein is measured by using a technique selected from the group consisting of immunoassay, Western blots, ELISA, and mass spectrometry.
15. A kit for predicting or monitoring a cancer patient's response to molecule of the taxoid family according to the method of aspect 1 wherein the kit comprises detectable-labeled antibodies, detectable-labeled antibody fragments or detectable-labeled oligonucleotides comprising nucleic acids capable of hybridizing under stringent conditions to said one or more genetic markers selected from the group consisting of:
   BubR1, Homo sapiens similar to protein kinase (BUBR1) mRNA, complete cds (GenBank accession number: AF046079);
   Mad2, Homo sapiens mRNA for MAD2 protein (GenBank accession number: AJ000186);
   Mps1, Homo sapiens TTK protein kinase (TTK), mRNA (GenBank accession number: NM_003318);
   GEFT for Rac1/CDC42, Homo sapiens RAC/CDC42 exchange factor (GEFT), transcript variant 2, mRNA (GenBank accession number: NM_133483);
   Bub1, Homo sapiens BUB1 budding uninhibited by benzimidazoles 1 homolog (yeast) (BUB1), mRNA (GenBank accession number: NM_004336);
   hSepharase, Homo sapiens extra spindle poles like 1 (S. cerevisiae) (ESPL1), mRNA (GenBank accession number: NM_012291);
   CamKIId, Homo sapiens calcium/calmodulin-dependent protein kinase (CaM kinase) II delta (CAMK2D), transcript variant 3, mRNA (GenBank accession number: NM_001221);
   CDK6, Homo sapiens cyclin-dependent kinase 6 (CDK6), mRNA (GenBank accession number: NM_001259); and
   GRB2, Homo sapiens growth factor receptor-bound protein 2 (GRB2), transcript variant 1, mRNA (GenBank accession number: NM_002086).
16. A kit for predicting or monitoring a cancer patient's response to molecule of the taxoid family according to the method of aspect 8 wherein the kit comprises detectable-labeled antibodies, detectable-labeled antibody fragments or detectable-labeled oligonucleotides comprising nucleic acids capable of hybridizing under stringent conditions to said one or more genetic markers selected from the group consisting of:
   P21(Wafl), Homo sapiens cyclin-dependent kinase inhibitor 1A (p21, Cip1) (CDKN1A), transcript variant 1, mRNA (GenBank accession number: NM_000389);
   Pim-1, Homo sapiens pim-1 oncogene (PIM1), mRNA (GenBank accession number: NM_002648);
   GBP-1, Homo sapiens guanylate binding protein 1, interferon-inducible, 67kDa (GBP1), mRNA (GenBank accession number: NM_002053);
   RXRA, Homo sapiens retinoid X receptor, alpha (RXRA), mRNA (GenBank accession number: NM_002957);
   SPF45, Homo sapiens RNA binding motif protein 17 (RBM17), mRNA (GenBank accession number: NM_032905);
   Hec1, Homo sapiens kinetochore associated 2 (KNTC2), mRNA (GenBank accession number: NM_006101);
   Raf1 Human mRNA for raf oncogene (GenBank accession number: X03484); Aurora A, Homo sapiens aurora-related kinase 1 (ARK1) mRNA, complete cds (GenBank accession number: AF008551);
   TACC3, Homo sapiens transforming, acidic coiled-coil containing protein 3 (TACC3), mRNA (GenBank accession number: NM_006342);
   RelB, Homo sapiens v-rel reticuloendotheliosis viral oncogene homolog B, nuclear factor of kappa light polypeptide gene enhancer in B-cells 3 (avian) (RELB), mRNA (GenBank accession number: NM_006509);
   PRKCD, Homo sapiens protein kinase C, delta (PRKCD), transcript variant 1, mRNA (GenBank accession number: NM_006254);
   BRAF35, Homo sapiens high-mobility group 20B (HMG20B), mRNA (GenBank accession number: NM_006339);
   HSPA1L, Homo sapiens heat shock 70kDa protein 1A (HSPA1A), mRNA (GenBank accession number: NM_005345);
   STK11, Homo sapiens serine/threonine kinase 11 (Peutz-Jeghers syndrome) (STK11), mRNA (GenBank accession number: NM_000455); and
   MKK3, Homo sapiens MAP kinase kinase 3 (MKK3) mRNA, complete cds (GenBank accession number: L36719).
17. A kit for predicting or monitoring a cancer patient's response to molecule of the taxoid family according to the method of aspect 1 wherein the kit comprises the means of:
   a) obtaining total RNA from a bodily sample;
   b) reverse transcribing the total RNA to obtain cDNA; and
   c) subjecting the cDNA to a polymerase chain reaction using a set of primers
      wherein one or both primers are detectably-labeled and both primers are derived from a nucleotide sequence of one or more genetic markers selected from the group consisting of:
      BubR1, Homo sapiens similar to protein kinase (BUBR1) mRNA, complete cds (GenBank accession number: AF046079);
      Mad2, Homo sapiens mRNA for MAD2 protein (GenBank accession number: AJ000186);
      Mps1, Homo sapiens TTK protein kinase (TTK), mRNA (GenBank accession number: NM_003318);
      GEFT for Rac1/CDC42, Homo sapiens RAC/CDC42 exchange factor (GEFT), transcript variant 2, mRNA (GenBank accession number: NM_133483);
      Bub1, Homo sapiens BUB1 budding uninhibited by benzimidazoles 1 homolog (yeast) (BUB1), mRNA (GenBank accession number: NM_004336);
      hSepharase, Homo sapiens extra spindle poles like 1 (S. cerevisiae) (ESPL1), mRNA (GenBank accession number: NM_012291);
      CamKIId, Homo sapiens calcium/calmodulin-dependent protein kinase (CaM kinase) II delta (CAMK2D), transcript variant 3, mRNA (GenBank accession number: NM_001221);
      CDK6, Homo sapiens cyclin-dependent kinase 6 (CDK6), mRNA (GenBank accession number: NM_001259); and
      GRB2, Homo sapiens growth factor receptor-bound protein 2 (GRB2), transcript variant 1, mRNA (GenBank accession number: NM_002086).
18. A kit for predicting or monitoring a cancer patient's response to molecule of the taxoid family according to the method of aspect 8 wherein the kit comprises the means of:
   a) obtaining total RNA from a bodily sample;
   b) reverse transcribing the total RNA to obtain cDNA; and
   c) subjecting the cDNA to a polymerase chain reaction using a set of primers
      wherein one or both primers are detectably-labeled and both primers are derived from a nucleotide sequence of one or more genetic markers selected from the group consisting of:
      P21(Waf1), Homo sapiens cyclin-dependent kinase inhibitor 1A (p21, Cip1) (CDKN1A), transcript variant 1, mRNA (GenBank accession number: NM_000389);
      Pim-1, Homo sapiens pim-1 oncogene (PIM1), mRNA (GenBank accession number: NM_002648);
      GBP-1, Homo sapiens guanylate binding protein 1, interferon-inducible, 67kDa (GBP1), mRNA (GenBank accession number: NM_002053);
      RXRA, Homo sapiens retinoid X receptor, alpha (RXRA), mRNA (GenBank accession number: NM_002957);
      SPF45, Homo sapiens RNA binding motif protein 17 (RBM17), mRNA (GenBank accession number: NM_032905);
      Hec1, Homo sapiens kinetochore associated 2 (KNTC2), mRNA (GenBank accession number: NM_006101);
      Raf1, Human mRNA for raf oncogene (GenBank accession number: X03484); Aurora A, Homo sapiens aurora-related kinase 1 (ARK1) mRNA, complete cds (GenBank accession number: AF008551);
      TACC3, Homo sapiens transforming, acidic coiled-coil containing protein 3 (TACC3), mRNA (GenBank accession number: NM_006342);
      RelB, Homo sapiens v-rel reticuloendotheliosis viral oncogene homolog B, nuclear factor of kappa light polypeptide gene enhancer in B-cells 3 (avian) (RELB), mRNA (GenBank accession number: NM_006509);
      PRKCD, Homo sapiens protein kinase C, delta (PRKCD), transcript variant 1, mRNA (GenBank accession number: NM_006254);
      BRAF35, Homo sapiens high-mobility group 20B (HMG20B), mRNA (GenBank accession number: NM_006339);
      HSPA1L, Homo sapiens heat shock 70kDa protein 1A (HSPA1A), mRNA (GenBank accession number: NM_005345);
      STK11, Homo sapiens serine/threonine kinase 11 (Peutz-Jeghers syndrome) (STK11), mRNA (GenBank accession number: NM_000455); and
      MKK3, Homo sapiens MAP kinase kinase 3 (MKK3) mRNA, complete cds (GenBank accession number: L36719).
19. The detectable label of any one of aspects 15 - 18, wherein the detectable label is selected from the group consisting of an enzyme, a radioactive isotope, or a chemical which fluoresces, a chemiluminescent molecule, radiopaque substances, liposomes, and haptenic molecules.
20. A method for predicting or monitoring a cancer patient's response to a molecule of the taxoid family, comprising the steps of:
   a) obtaining a test sample from a cancerous area of said patient;
   b) measuring the level of one or more genetic markers selected from the group consisting of:
      BubR1, Homo sapiens similar to protein kinase (BUBR1) mRNA, complete cds (GenBank accession number: AF046079);
      Mad2, Homo sapiens mRNA for MAD2 protein (GenBank accession number: AJ000186);
      Mps1, Homo sapiens TTK protein kinase (TTK), mRNA (GenBank accession number: NM_003318);
      GEFT for Rac1/CDC42, Homo sapiens RAC/CDC42 exchange factor (GEFT), transcript variant 2, mRNA (GenBank accession number: NM_133483);
      Bub1, Homo sapiens BUB1 budding uninhibited by benzimidazoles 1 homolog (yeast) (BUB1), mRNA (GenBank accession number: NM_004336);
      hSepharase, Homo sapiens extra spindle poles like 1 (S. cerevisiae) (ESPL1), mRNA (GenBank accession number: NM_012291);
      CamKIId, Homo sapiens calcium/calmodulin-dependent protein kinase (CaM kinase) II delta (CAMK2D), transcript variant 3, mRNA (GenBank accession number: NM_001221);
      CDK6, Homo sapiens cyclin-dependent kinase 6 (CDK6), mRNA (GenBank accession number: NM_001259); and
      GRB2, Homo sapiens growth factor receptor-bound protein 2 (GRB2), transcript variant 1, mRNA (GenBank accession number: NM_002086)
   c) measuring the level of one or more reference genetic markers selected from the group consisting of:
      GAPDH, Homo sapiens glyceraldehyde-3-phosphate dehydrogenase (GAPD),
      mRNA (GenBank accession number: M_002046) ; and
      RPS9, Homo sapiens cDNA clone IMAGE:6647283, partial cds (GenBank accession number: BC071941);
   d) comparing the measured levels of said one or more genetic markers and said one or more reference genetic markers in the test sample;
      wherein a decrease in the level of said one or more genetic markers as compared to the level of said one or more reference genetic markers indicates an increased resistance to a molecule of the taxoid family.
21. A method for predicting or monitoring a cancer patient's response to a molecule of the taxoid family, comprising the steps of:
   a) obtaining a test sample from a cancerous area of said patient;
   b) measuring the level of one or more genetic markers selected from the group consisting of:
      P21(Waf1), Homo sapiens cyclin-dependent kinase inhibitor 1A (p21, Cip1) (CDKN1A), transcript variant 1, mRNA (GenBank accession number: NM_000389);
      Pim-1, Homo sapiens pim-1 oncogene (PIM1), mRNA (GenBank accession number: NM_002648);
      GBP-1, Homo sapiens guanylate binding protein 1, interferon-inducible, 67kDa (GBP1), mRNA (GenBank accession number: NM_002053);
      RXRA, Homo sapiens retinoid X receptor, alpha (RXRA), mRNA (GenBank accession number: NM_002957);
      SPF45, Homo sapiens RNA binding motif protein 17 (RBM17), mRNA (GenBank accession number: NM_032905);
      Hec1, Homo sapiens kinetochore associated 2 (KNTC2), mRNA (GenBank accession number: NM_006101);
      Raf1, Human mRNA for raf oncogene (GenBank accession number: X03484);
      Aurora A, Homo sapiens aurora-related kinase 1 (ARK1) mRNA, complete cds (GenBank accession number: AF008551);
      TACC3, Homo sapiens transforming, acidic coiled-coil containing protein 3 (TACC3), mRNA (GenBank accession number: NM_006342);
      RelB, Homo sapiens v-rel reticuloendotheliosis viral oncogene homolog B, nuclear factor of kappa light polypeptide gene enhancer in B-cells 3 (avian) (RELB), mRNA (GenBank accession number: NM_006509);
      PRKCD, Homo sapiens protein kinase C, delta (PRKCD), transcript variant 1, mRNA (GenBank accession number: NM_006254);
      BRAF35, Homo sapiens high-mobility group 20B (HMG20B), mRNA (GenBank accession number: NM_006339);
      HSPA1L, Homo sapiens heat shock 70kDa protein 1A (HSPA1A), mRNA (GenBank accession number: NM_005345);
      STK11, Homo sapiens serine/threonine kinase 11 (Peutz-Jeghers syndrome) (STK11), mRNA (GenBank accession number: NM_000455); and
      MKK3, Homo sapiens MAP kinase kinase 3 (MKK3) mRNA, complete cds (GenBank accession number: L36719);
   c) measuring the level of one or more reference genetic markers selected from the group consisting of:
      GAPDH, Homo sapiens glyceraldehyde-3-phosphate dehydrogenase (GAPD), mRNA (GenBank accession number: NM_002046); and
      RPS9, Homo sapiens cDNA clone IMAGE:6647283, partial cds (GenBank accession number: BC071941);
   d) comparing the measured levels of said one or more genetic markers and said one or more reference genetic markers in the test sample;
      wherein a decrease in the level of said one or more genetic markers as compared to the level of said one or more reference genetic markers indicates an increased susceptibility to a molecule of the taxoid family.
22. The method of aspect 20 or 21 wherein said molecule of the taxoid family is selected from the group consisting of paclitaxel, docetaxel XRP9881 and XRP6258.
23. The method of aspect 20 or 21 wherein said level of one or more genetic markers is measured by mRNA, DNA or protein.
24. The method of aspect 23 wherein said mRNA is measured by using a technique selected from the group consisting of in situ hybridization, reverse-transcriptase polymerase chain reaction, nucleic acid hybridization, electrophoresis, Northern blotting and mass spectrometry.
25. The method of aspect 23 wherein said DNA is measured by using a technique selected from the group consisting of quantitative polymerase chain reaction, genomic DNA-chips, in situ hybridization, electrophoresis, Southern blotting and mass spectrometry.
26. The method of aspect 23 wherein said protein is measured by using a technique selected from the group consisting of immunoassay, Western blots, ELISA, and mass spectrometry.
27. A kit for predicting or monitoring a cancer patient's response to a molecule of the taxoid family according to the method of aspect 20 wherein the kit comprises detectable-labeled antibodies, detectable-labeled antibody fragments or detectable-labeled oligonucleotides comprising nucleic acids capable of hybridizing under stringent conditions to said one or more genetic markers and said one or more reference genetic markers selected from the group consisting of:
   BubR1, Homo sapiens similar to protein kinase (BUBR1) mRNA, complete cds (GenBank accession number: AF046079);
   Mad2, Homo sapiens mRNA for MAD2 protein (GenBank accession number: AJ000186);
   Mps1, Homo sapiens TTK protein kinase (TTK), mRNA (GenBank accession number: NM_003318);
   GEFT for Rac1/CDC42, Homo sapiens RAC/CDC42 exchange factor (GEFT), transcript variant 2, mRNA (GenBank accession number: NM_133483);
   Bub1 Homo sapiens BUB1 budding uninhibited by benzimidazoles 1 homolog (yeast) (BUB1), mRNA (GenBank accession number: NM_004336);
   hSepharase, Homo sapiens extra spindle poles like 1 (S. cerevisiae) (ESPL1), mRNA (GenBank accession number: NM_012291);
   CamKIId, Homo sapiens calcium/calmodulin-dependent protein kinase (CaM kinase) II delta (CAMK2D), transcript variant 3, mRNA (GenBank accession number: NM_001221);
   CDK6, Homo sapiens cyclin-dependent kinase 6 (CDK6), mRNA (GenBank accession number: NM_001259);
   GRB2, Homo sapiens growth factor receptor-bound protein 2 (GRB2), transcript variant 1, mRNA (GenBank accession number: NM_002086);
   GAPDH, Homo sapiens glyceraldehyde-3-phosphate dehydrogenase (GAPD), mRNA (GenBank accession number: NM_002046); and
   RPS9, Homo sapiens cDNA clone IMAGE:6647283, partial cds (GenBank accession number: BC071941).
28. A kit for predicting or monitoring a cancer patient's response to a molecule of the taxoid family according to the method of aspect 21 wherein the kit comprises detectable-labeled antibodies, detectable-labeled antibody fragments or detectable-labeled oligonucleotides comprising nucleic acids capable of hybridizing under stringent conditions to said one or more genetic markers and said one or more genetic markers selected from the group consisting of:
   P21(Waf1 Homo sapiens cyclin-dependent kinase inhibitor 1A (p21, Cip1) (CDKN1A), transcript variant 1, mRNA (GenBank accession number: NM_000389); Pim-1, Homo sapiens pim-1 oncogene (PIM1), mRNA (GenBank accession number:
   NM_002648);
   GBP-1, Homo sapiens guanylate binding protein 1, interferon-inducible, 67kDa (GBP1), mRNA (GenBank accession number: NM_002053);
   RXRA, Homo sapiens retinoid X receptor, alpha (RXRA), mRNA (GenBank accession number: NM_002957);
   SPF45, Homo sapiens RNA binding motif protein 17 (RBM17), mRNA (GenBank
   accession number: NM_032905); Hec1, Homo sapiens kinetochore associated 2 (KNTC2), mRNA (GenBank accession number: NM_006101);
   Raf1, Human mRNA for raf oncogene (GenBank accession number: X03484); Aurora A, Homo sapiens aurora-related kinase 1 (ARK1) mRNA, complete cds (GenBank accession number: AF008551);
   TACC3, Homo sapiens transforming, acidic coiled-coil containing protein 3 (TACC3), mRNA (GenBank accession number: NM_006342);
   RelB, Homo sapiens v-rel reticuloendotheliosis viral oncogene homolog B, nuclear factor of kappa light polypeptide gene enhancer in B-cells 3 (avian) (RELB), mRNA (GenBank accession number: NM_006509);
   PRKCD, Homo sapiens protein kinase C, delta (PRKCD), transcript variant 1, mRNA (GenBank accession number: NM 006254);
   BRAF35, Homo sapiens high-mobility group 20B (HMG20B), mRNA (GenBank accession number: NM_006339);
   HSPA1L, Homo sapiens heat shock 70kDa protein 1A (HSPA1A), mRNA (GenBank accession number: NM_005345);
   STK11, Homo sapiens serine/threonine kinase 11 (Peutz-Jeghers syndrome) (STK11), mRNA (GenBank accession number: NM_000455); and
   MKK3, Homo sapiens MAP kinase kinase 3 (MKK3) mRNA, complete cds (GenBank accession number: L36719);
   GAPDH, Homo sapiens glyceraldehyde-3-phosphate dehydrogenase (GAPD), mRNA (GenBank accession number: NM_002046); and
   RPS9, Homo sapiens cDNA clone IMAGE:6647283, partial cds (GenBank accession number: BC071941).
29. A kit for predicting or monitoring a cancer patient's response to a molecule of the taxoid family according to the method of aspect 20 wherein the kit comprises the means of:
   a) obtaining total RNA from a bodily sample;
   b) reverse transcribing the total RNA to obtain cDNA; and
   c) subjecting the cDNA to a polymerase chain reaction using a set of primers wherein one or both primers are detectable-labeled and both primers are derived from a nucleotide sequence of one or more genetic markers selected from the group consisting of:
      BubR1, Homo sapiens similar to protein kinase (BUBR1) mRNA, complete cds (GenBank accession number: AF046079);
      Mad2, Homo sapiens mRNA for MAD2 protein (GenBank accession number: AJ000186);
      Mps1, Homo sapiens TTK protein kinase (TTK), mRNA (GenBank accession number: NM_003318);
      GEFT for Rac1/CDC42, Homo sapiens RAC/CDC42 exchange factor (GEFT), transcript variant 2, mRNA (GenBank accession number: NM_133483);
      Bub1 Homo sapiens BUB1 budding uninhibited by benzimidazoles 1 homolog (yeast) (BUB1), mRNA (GenBank accession number: NM_004336);
      hSepharase, Homo sapiens extra spindle poles like 1 (S. cerevisiae) (ESPL1), mRNA (GenBank accession number: NM_012291);
      CamKIId, Homo sapiens calcium/calmodulin-dependent protein kinase (CaM kinase) II delta (CAMK2D), transcript variant 3, mRNA (GenBank accession number: NM_001221);
      CDK6, Homo sapiens cyclin-dependent kinase 6 (CDK6), mRNA (GenBank accession number: NM_001259); and
      GRB2, Homo sapiens growth factor receptor-bound protein 2 (GRB2), transcript variant 1; mRNA (GenBank accession number: NM_002086);
      GAPDH, Homo sapiens glyceraldehyde-3-phosphate dehydrogenase (GAPD), mRNA (GenBank accession number: NM_002046); and
      RPS9, Homo sapiens cDNA clone IMAGE:6647283, partial cds (GenBank accession number: BC071941).
30. A kit for predicting or monitoring a cancer patient's response to a molecule of the taxoid family according to the method of aspect 19 wherein the kit comprises the means of:
   a) obtaining total RNA from a bodily sample;
   b) reverse transcribing the total RNA to obtain cDNA; and
   c) subjecting the cDNA to a polymerase chain reaction using a set of primers wherein one or both primers are detectable-labeled and both primers are derived from a nucleotide sequence of one or more genetic markers selected from the group consisting of:
      P21(Wafl), Homo sapiens cyclin-dependent kinase inhibitor 1A (p21, Cip1) (CDKN1A), transcript variant 1, mRNA (GenBank accession number: NM_000389);
      Pim-1, Homo sapiens pim-1 oncogene (PIM1), mRNA (GenBank accession number: NM_002648);
      GBP-1, Homo sapiens guanylate binding protein 1, interferon-inducible, 67kDa (GBP1), mRNA (GenBank accession number: NM_002053);
      RXRA, Homo sapiens retinoid X receptor, alpha (RXRA), mRNA (GenBank accession number: NM_002957);
      SPF45, Homo sapiens RNA binding motif protein 17 (RBM17), mRNA (GenBank accession number: NM_032905);
      Hec1, Homo sapiens kinetochore associated 2 (KNTC2), mRNA (GenBank accession number: NM_006101);
      Raf1, Human mRNA for raf oncogene (GenBank accession number: X03484);
      Aurora A, Homo sapiens aurora-related kinase 1 (ARK1) mRNA, complete cds (GenBank accession number: AF008551);
      TACC3, Homo sapiens transforming, acidic coiled-coil containing protein 3 (TACC3), mRNA (GenBank accession number: NM_006342);
      RelB, Homo sapiens v-rel reticuloendotheliosis viral oncogene homolog B, nuclear factor of kappa light polypeptide gene enhancer in B-cells 3 (avian) (RELB), mRNA (GenBank accession number: NM_006509);
      PRKCD, Homo sapiens protein kinase C, delta (PRKCD), transcript variant 1, mRNA (GenBank accession number: NM_006254);
      BRAF35, Homo sapiens high-mobility group 20B (HMG20B), mRNA (GenBank accession number: NM_006339);
      HSPA1L, Homo sapiens heat shock 70kDa protein 1A (HSPA1A), mRNA (GenBank accession number: NM_005345);
      STK11, Homo sapiens serine/threonine kinase 11 (Peutz-Jeghers syndrome) (STK11), mRNA (GenBank accession number: NM_000455); and
      MKK3, Homo sapiens MAP kinase kinase 3 (MKK3) mRNA, complete cds (GenBank accession number: L36719);
      GAPDH, Homo sapiens glyceraldehyde-3-phosphate dehydrogenase (GAPD), mRNA (GenBank accession number: NM_002046); and
      RPS9, Homo sapiens cDNA clone IMAGE:6647283, partial cds (GenBank accession number: BC071941).
31. The detectable label of any one of aspects 24 - 27, wherein the detectable label comprises an enzyme, a radioactive isotope, or a chemical which fluoresces, a chemiluminescent molecule, radiopaque substances, liposomes, and haptenic molecules.

The above aspects and other aspects, features, and advantages of the present invention will be better understood from the following detailed description taken in conjunction with the accompanying figures.

### BRIEF DESCRIPTION OF THE DRAWINGS

Figure 1 shows the characterization of genes whose downregulation confer docetaxel resistance. Docetaxel generated dose response curves shown for HCT116 cells transfected with RB1, BubR1, Mad2 and Mps1 siRNAs (solid squares) compared to control siRNA (open squares). RB 1 was an example of a siRNA that did not score in the screen. Cell viability was measured at an absorbance of 450 nM following WST-1 addition. The minimum ratio (MR) is the WST-1 value for a gene over the control at 40 nM docetaxel.
Figure 2 shows dose response curves of BubR1 and Mps1 shRNAs (solid squares) compared to control shRNA (open squares).
Figure 3 shows TaqMan real-time PCR analysis of BubR1 and Mps1 mRNA levels in cells containing BubR1 and Mps1 shRNA, respectively.
Figure 4 shows cell viability and morphology shown for HCT116 cells transfected with Mad2, BubR1 and Mps1 siRNAs with or without docetaxel treatment. Twenty-four hours after transfection, cells were left untreated (-Docetaxel) or treated with 200 nM docetaxel (+Docetaxel) and stained with Calcein-AM to visualize live cells 16 and 72 hours after treatment.
Figure 5 shows mitotic indices for HCT116 cells transfected with Mad2, BubR1, Mps1 and control siRNAs following docetaxel treatment. Twenty-four hours after transfection cells were treated with 200 nM docetaxel for 16 and 72 hours and processed. Mitotic cells were detected by a phosphorylated histone H3 antibody, shown here as red punctuate staining over the nucleus of cells (Mitotic Index Kit, Cellomics). Nuclei were stained blue with Hoechst dye.
Figure 6 shows TaqMan real-time PCR analysis of Mps1, BubR1 and Mad2 mRNA levels in HCT116 cells transfected with Mps1, BubR1 and Mad2 siRNA, respectively.
Figure 7 shows cell cycle analysis of HCT116 cells transfected with three mitotic checkpoint gene siRNAs. After transfection with Mps1, BubR1 and Mad2 siRNAs, cells were untreated (-Docetaxel) or treated with 200 nM docetaxel (+Docetaxel) for 24 hours. Seventy-two hours after the addition of docetaxel, cells were harvested and analyzed for cell cycle. Numbers above peaks indicate DNA content such as 2N, 4N, 8N, 16N or 32N.
Figure 8 shows clonogenic cell survival assay using stable knockdown cell lines. HCT116 cells containing BubR1 or vector control shRNAs were plated on 10 cm dishes and maintained in 5 nM docetaxel for 10 days. Colonies were washed with PBS and stained with crystal violet.
Figure 9 shows several dose response curves of genes whose down-regulation confer increased sensitivity to docetaxel. Doectaxel generated dose response curves shown for HCT116 cells transfected with RB1, Pim-1, p21, Aurora A and TACC3 siRNAs (solid squares) compared to control siRNA (open squares). The minimum ration (MR) is the ratio of WST-1 readout of a gene over that of the control at 40 nM docetaxel concentration. The IC50 (IR) ratio is the ratio of IC50 of a gene over that of the control.
Figure 10 shows a dose response curves of Aurora A shRNA (solid squares) compared to vector control (open squares).
Figure 11 shows TaqMan real-time PCR analysis of Aurora A mRNA levels in cells containing Aurora A shRNA.
Figure 12 shows differences in proliferation of HCT116 cells transfected with Pim-1 and TACC3 siRNAs (solid squares) compared to control siRNA (open squares). Number of cells remaining in 6-well plates at different time points after transfection with Pim-1, TACC3 and control siRNAs.
Figure 13 shows TaqMan analysis of Pim-1 and TACC3 mRNA levels after siRNA transfection.
Figure 14 shows active caspase-3 levels in HCT116 cells transfected with Pim-1 and BubR1 siRNAs. Active caspase-3 levels were shown as fluorescent intensity using the active caspase-3 beadmates kit (Upstate). Three docetaxel concentrations, 0, 5 and 40 nM, and three time points, 24, 48 and 72 hours after docetaxel addition were examined.

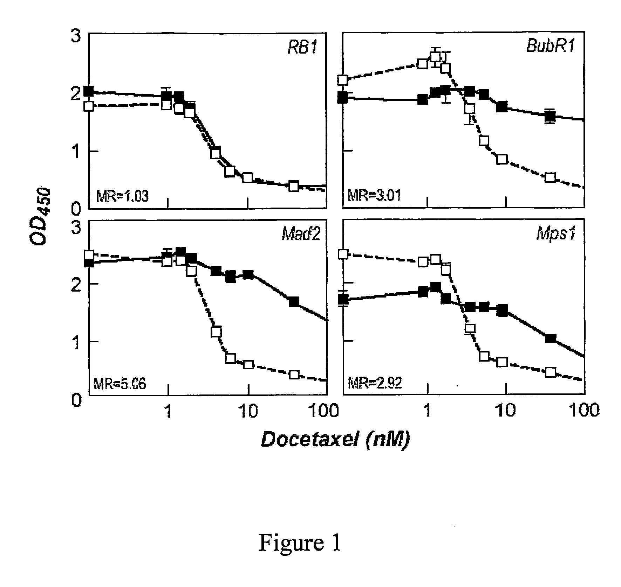

Figure 1 shows AKT phosphorylation levels in HCT116 cells transfected with Pim-1 and BubR1 siRNAs. The ratio of phosphorylated and total AKT levels was determined using a bead-based assay (Biosource). Twenty-four hours after transfection cells were left untreated or treated with 5 and 40 nM docetaxel. Forty-eight hours after docetaxel treatment cells lysates were analyzed. The phosphorylation level was calculated as the ratio of phosphorylated AKT (p-AKT) over total AKT (t-AKT).

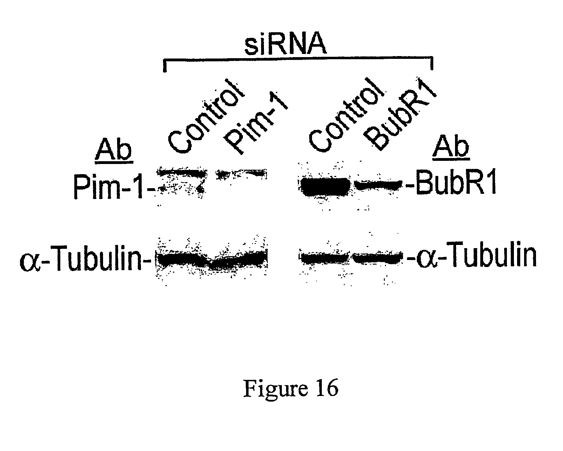

Figure 16 shows Western blots showing the reduced levels of Pim-1 and BubR1 48 hours after transfection.

### DETAILED DESCRIPTION OF THE INVENTION

The present invention is broadly based upon applicants' identification of gene markers that can predict whether a test subject is resistant or sensitive to drugs in the taxoid family. Using a cell-based RNA interference (RNAi) screen, applicants observed that drug resistance or sensitivity to docetaxel was associated with particular gene markers.

It is noted that numerous terms and phases are used throughout the instant specification and claims. Definitions of these terms and phrases are provided below:
As used herein, the term "prognosis" refers to the prediction or the likelihood of cancer-attributable death or progression, including drug resistance as well as recurrence, metastatic spread, and neoplastic disease.
As used herein, the term "prediction" refers to the likelihood that a patient will respond either favorably or unfavorably to a drug or set of drugs, and also the extent of those responses Such as whether the patient will survive, following surgical removal of the primary tumor and/or chemotherapy for a certain period of time without cancer recurrence. Predictive methods envisioned by the present invention can be used clinically to make treatment decisions by choosing the most appropriate treatment modalities, in particular a chemotherapeutic of the taxoid family, for any particular patient. The predictive methods of the present invention are valuable tools in predicting if a patient is likely to respond favorably to a treatment regimen, such as chemotherapy with a given drug or drug combination, and/or radiation therapy, or whether long-term survival of the patient following termination of chemotherapy or other treatment modalities is likely.
As used herein, the term "tumor," refers to all neoplastic cell growth and proliferation, whether malignant or benign, and all pre-cancerous and cancerous cells and tissues.
As used herein, the term "cancer" and "cancerous" refers to a physiological condition in mammals typically characterized by unregulated cell growth. Examples of cancer include but are not limited to the following: breast cancer; colon cancer; lung cancer; prostate cancer; hepatocellular cancer; gastric cancer; pancreatic cancer; cervical and ovarian cancer; liver cancer; bladder cancer; cancer of the urinary tract; carcinoma; melanoma; brain cancer including glioblastomas and medulloblastomas; biliary tract cancer; choriocarcinoma; esophageal cancer; gastric cancer; hematological neoplasms including acute lymphocytic and myelogenous leukemia; multiple myeloma; AIDS-associated leukemia and adult T-cell leukemia lymphoma; intraepithelial neoplasms including Bowen's disease and Paget's disease; lymphomas including Hodgkin`s disease and lymnphocytic lymphomas; neuroblastomas; oral cancer including squamous cell carcinoma, sarcomas including leiomyosarcoma, rhabdomyosarcoma, liposarcoma, fibrosarcoma, and osteosarcoma; skin cancer including melanoma, Kaposi's sarcoma, basocellular cancer, and squamous cell cancer; testicular cancer including germinal tumors such as seminoma, non-seminoma (teratomas, choriocarcinomas), stromal tumors, and germ cell tumors; thyroid cancer including thyroid adenocarcinoma and modullar carcinoma; and renal cancer including adenocarcinoma and Wilms tumor..
As used herein, the term "patient" refers preferably to a human but may also include a non-human primate, cow, horse, pig, sheep, goat, dog, cat or rodent. Preferably the subject is a human either suspected of having cancer, or having been diagnosed with cancer or in a high-risk category for developing cancer, for example, having a family history of cancer. In a preferred embodiment of the invention the cancer is breast cancer. Methods for identifying subjects suspected of having cancer may include manual examination, biopsy, subject's family medical history, subject's medical history, or a number of imaging technologies such as mammography, magnetic resonance imaging, magnetic resonance spectroscopy, or positron emission tomography. Diagnostic methods for cancer and the clinical characterizations of cancer diagnoses are well-known to those of skill in the medical arts.
As used herein, the term "sample" is tissue obtained using methods well-known to those of ordinary skill in the related medical arts. Methods such as biopsy include gross apportioning of a mass, microdissection, laser-based microdissection, or other art-known cell-separation methods. Because of the variability of the cell types in diseased-tissue biopsy material, and the variability in sensitivity of the diagnostic methods used, the sample size required for analysis may range from 1, 10, 50, 100, 200, 300, 500, 1000, 5000, 10,000, to 50,000 or more cells. The appropriate sample size may be determined based on the cellular composition and condition of the biopsy and the standard preparative steps for this determination and subsequent isolation of the nucleic acid for use in the invention are well known to one of ordinary skill in the art. For example, a sample from a biopsy may be sufficient for assessment of RNA expression without amplification. Conversely, the lack of a suitable number of cells in a small biopsy region may require use of RNA conversion and/or amplification methods or other methods to enhance resolution of the nucleic acid molecules. Such methods, which allow use of limited biopsy materials, are well known to those of ordinary skill in the art. Some examples include but are not limited to direct RNA amplification, reverse transcription of RNA to cDNA, amplification of cDNA, or the generation of radio-labeled nucleic acids.
As used herein, the term "test " with respect to a sample refers to a sample taken from a cancerous area of the body or from an area of the body exhibiting a stage of or characteristics of cancer.
As used herein, the term "control" with respect to a sample refers to samples which are used for comparative purposes. Preferably, these samples are "control" in the sense that the samples do not exhibit any indication of, or are believed to have, any disease or condition that would affect gene expression, particularly in respect of the disease for which they are to be used as the standard. Alternatively, it may be appreciated that different stages of a disease or condition can be compared and in such cases, the "control" sample corresponds to the earlier stage of the disease or condition. For example, a control sample can be a sample taken from a non-cancerous but comparable area of the body. Additionally, a control sample may be a comparable area of the body from the same subject or a non-cancerous area of the body from a second subject substantially similar to the first subject (same or similar species, age, weight, sex, etc.). Finally, a control sample could also be from a cancerous area of a second subject who respond well to treatment using a molecule of the taxoid family.
A "nucleic acid molecule" refers to the phosphate ester polymeric form of ribonucleosides (adenosine, guanosine, uridine or cytidine; "RNA molecules") or deoxyribonucleosides (deoxyadenosine, deoxyguanosine, deoxythymidine, or deoxycytidine; "DNA molecules"), or any phosphoester analogs thereof, such as phosphorothioates and thioesters, in either single stranded form, or a double-stranded helix. Double stranded DNA-DNA, DNA-RNA and RNA-RNA helices are possible. The term nucleic acid molecule, and in particular DNA or RNA molecule, refers only to the primary and secondary structure of the molecule, and does not limit it to any particular tertiary forms. Thus, this term includes double-stranded DNA found, *inter alia,* in linear or circular DNA molecules (e.g., restriction fragments), plasmids, and chromosomes. In discussing the structure of particular double-stranded DNA molecules, sequences may be described herein according to the normal convention of giving only the sequence in the 5' to 3' direction along the non-transcribed strand of DNA (*i.e.,* the strand having a sequence homologous to the mRNA). A "recombinant DNA molecule" is a DNA molecule that has undergone a molecular biological manipulation.
As used herein, the term "portion" of an isolated nucleic acid molecule that encodes for a particular protein, refers to a part or fragment of the isolated nucleic acid molecule that comprises a sufficient number of contiguous nucleotides that encode for a peptide or polypeptide. Naturally, a "portion" of an isolated nucleic acid molecule is greater than one nucleotide, and the peptide or polypeptide encoded by the portion contains numerous amino acid residues, as described in the definitions of peptide and polypeptide below.
As used herein, the term "peptide" refers to two or more amino acids covalently joined by peptide binds. In a particular embodiment, a peptide comprises at least 10, preferably at least 20, more preferably at least 30, even more preferably at least 40, and most preferably 50 or more amino acids.
As used herein, the term "polypeptide" refers to a linear polymer composed of multiple contiguous amino acids. In particular, a polypeptide may possess a molecular weight greater than 100 kD.
As used herein, the term "genetic marker" refers to a physiological composition whose measured RNA, DNA or protein level within a sample serves to predict whether a test subject is resistant or sensitive to drugs in the taxoid family. Moreover, a genetic marker may encode the particular protein or alternatively, may serve as a "surrogate" marker for a protein whose activity is related to the level of the genetic marker in a bodily sample. This relationship may be direct, wherein a decrease in the level of protein activity corresponds to a decrease in the level of the genetic marker, or alternatively, the relationship may be inverse, wherein a decrease in the level of protein activity corresponds to an increase in the level of the genetic marker. Such physiological compositions include, but certainly are not limited to, cells (e.g., progenitor stem cells) proteins, polypeptides, DNA, RNA, carbohydrates, or fatty acids, to name only a few. In a particular embodiment of the present invention, the measured levels of certain gene markers can predict whether a test subject is resistant or sensitive to drugs in the taxoid family. Examples of such genetic markers include, but certainly are not limited to:
   BubR1, Homo sapiens similar to protein kinase (BUBR1) mRNA, complete cds (GenBank accession number: AF046079);
   Mad2, Homo sapiens mRNA for MAD2 protein (GenBank accession number: AJ000186);
   Mps1, Homo sapiens TTK protein kinase (TTK), mRNA (GenBank accession number: NM_003318);
   GEFT for Rac1/CDC42, Homo sapiens RAC/CDC42 exchange factor (GEFT), transcript variant 2, mRNA (GenBank accession number: NM_133483);
   Bub1, Homo sapiens BUB1 budding uninhibited by benzimidazoles 1 homolog (yeast) (BUB1), mRNA (GenBank accession number: NM_004336);
   hSepharase, Homo sapiens extra spindle poles like 1 (S. cerevisiae) (ESPL1), mRNA, (GenBank accession number: NM_012291);
   CamKIId, Homo sapiens calcium/calmodulin-dependent protein kinase (CaM kinase) II delta (CAMK2D), transcript variant 3, mRNA (GenBank accession number: NM_001221);
   CDK6, Homo sapiens cyclin-dependent kinase 6 (CDK6), mRNA (GenBank accession number: NM_001259); and
   GRB2, Homo sapiens growth factor receptor-bound protein 2 (GRB2), transcript variant 1, mRNA (GenBank accession number: NM_002086)
   P21(Wafl), Homo sapiens cyclin-dependent kinase inhibitor 1A (p21, Cip1) (CDKN1A), transcript variant 1, mRNA (GenBank accession number: NM_000389); Pim-1, Homo sapiens pim-1 oncogene (PIM1), mRNA (GenBank accession number: NM_002648);
   GBP-1, Homo sapiens guanylate binding protein 1, interferon-inducible, 67kDa (GBP1), mRNA (GenBank accession number: NM_002053);
   RXRA, Homo sapiens retinoid X receptor, alpha (RXRA), mRNA (GenBank accession number: NM_002957);
   SPF45, Homo sapiens RNA binding motif protein 17 (RBM17), mRNA (GenBank accession number: NM_032905);
   Hec1, Homo sapiens kinetochore associated 2 (KNTC2), mRNA (GenBank accession number: NM_006101);
   Raf1, Human mRNA for raf oncogene (GenBank accession number: X03484);
   Aurora A, Homo sapiens aurora-related kinase 1 (ARK1) mRNA, complete cds (GenBank accession number: AF008551);
   TACC3, Homo sapiens transforming, acidic coiled-coil containing protein 3 (TACC3), mRNA (GenBank accession number: NM_006342);
   RelB, Homo sapiens v-rel reticuloendotheliosis viral oncogene homolog B, nuclear factor of kappa light polypeptide gene enhancer in B-cells 3 (avian) (RELB), mRNA (GenBank accession number: NM_006509);
   PRKCD, Homo sapiens protein kinase C, delta (PRKCD), transcript variant 1, mRNA (GenBank accession number: NM_006254);
   BRAF35, Homo sapiens high-mobility group 20B (HMG20B), mRNA (GenBank accession number: NM_006339);
   HSPA1L, Homo sapiens heat shock 70kDa protein 1A (HSPA1A), mRNA (GenBank accession number: NM_005345);
   STK11, Homo sapiens serine/threonine kinase 11 (Peutz-Jeghers syndrome) (STK11), mRNA (GenBank accession number: NM_000455); and
   MKK3, Homo sapiens MAP kinase kinase 3 (MKK3) mRNA, complete cds (GenBank accession number: L36719)
As used herein, the term "reference genetic marker" refers to a physiological composition whose measured RNA, DNA or protein levels remain unchanged before, during or after exposure to drugs in the taxoid family. "Reference genetic markers" are referred to as housekeeping genes. These are genes that are selected based on the relatively invariable levels of expression in the system which is being examined, for example, the in a particular disease such as cancer. Housekeeping genes are used to normalize results of expression. Examples of such genetic markers include, but certainly are not limited to:
   GAPDH, Homo sapiens glyceraldehyde-3-phosphate dehydrogenase (GAPD),
   mRNA (GenBank accession number: NM_002046); and
   RPS9, Homo sapiens cDNA clone IMAGE:6647283, partial cds (GenBank accession number: BC071941).
As used herein, the term "a molecule of the taxoid family" refers to a class of chemotherapeutic compounds to belonging to the taxane family. Specific members of the taxoid family include but are not limited to paclitaxel (taxol), docetaxel (taxotere) and analogs thereof (*i.e.*,XRP9881 and XRP6258; see Ojima and Geney, Curr Opin Investig Drugs 4:737, 2004). Since this class of molecules are beta-tubulin binders and stabilize the polymerized form of the microtubule similar to docetaxel, it is anticipated that the clinical expresson of the biomarker herein described will reflect similar response states to these drugs.
As used herein, the terms "molecule", "compound" or "agent" refer to any composition presently known or subsequently discovered. Examples of compounds or agents having applications herein include organic compounds (e.g., man made, naturally occurring and optically active), peptides (man made, naturally occurring, and optically active, i.e., either D or L amino acids), carbohydrates, nucleic acid molecules, etc.
As used herein, "stringency of hybridization" or "hybridization under stringent conditions" refers to conditions readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).
As used herein, "stringent conditions" or "high stringency conditions", refers to those parameters that: (1) employ low ionic strength and high temperature for washing, for example 0.015 M sodium chloride/0.0015 M sodium citrate/0.1% sodium dodecyl sulfate at 50° C.; (2) employ during hybridization a denaturing agent, such as formamide, for example, 50% (v/v) formamide with 0.1% bovine serum albumin/0.1% Ficoll/0.1% polyvinylpyrrolidone/50 mM sodium phosphate buffer at pH 6.5 with 750 mM sodium chloride, 75 mM sodium citrate at 42° C.; or (3) overnight hybridization in a solution that employs 50% formamide, 5x SSC (0.75 M NaCl, 0.075 M sodium citrate), 50 mM sodium phosphate (pH 6.8), 0.1% sodium pyrophosphate, 5x Denhardt's solution, sonicated salmon sperm DNA (50 .mu.g/ml), 0.1% SDS, and 10% dextran sulfate at 42° C, with a 10 minute wash at 42°C in 0.2x SSC (sodium chloride/sodium citrate) followed by a 10 minute high-stringency wash consisting of 0.1x SSC containing EDTA at 55° C. Moderately stringent conditions may be identified as described by Sambrook et al., Molecular Cloning: A Laboratory Manual, New York: Cold Spring Harbor Press, 1989, and include the use of washing solution and hybridization conditions (e.g., temperature, ionic strength and % SDS) less stringent that those described above. An example of moderately stringent conditions is overnight incubation at 37°C in a solution comprising: 20% formamide, 5x SSC (150 mM NaCl, 15 mM trisodium citrate), 50 mM sodium phosphate (pH 7.6), 5x Denhardt's solution, 10% dextran sulfate, and 20 mg/ml denatured sheared salmon sperm DNA, followed by washing the filters in 1x SSC at about 37-50°C. The skilled artisan will recognize how to adjust the temperature, ionic strength, etc. as necessary to accommodate factors such as probe length and the like. The appropriate stringency for hybridizing nucleic acids depends on the length of the nucleic acids and the degree of complementation, variables well known in the art. The greater the degree of similarity or homology between two nucleotide sequences, the greater the value of Tm for hybrids of nucleic acids having those sequences. The relative stability (corresponding to higher Tm) of nucleic acid hybridizations decreases in the following order: RNA:RNA, DNA:RNA, DNA:DNA. Preferably a minimum length for a hybridizable nucleic acid is at least about 12 nucleotides; preferably at least about 16 nucleotides; and more preferably the length is at least about 24 nucleotides; and most preferably at least 36 nucleotides.
As used herein, "label" or "detectable-label" refers to a detectable label compound or composition which is conjugated directly or indirectly to an antibody, oligopeptide or other organic molecule so as to generate a "labeled" antibody, oligopeptide or other organic molecule. The label may be detectable by itself (e.g. radioisotope labels or fluorescent labels) or, in the case of an enzymatic label, may catalyze chemical alteration of a substrate compound or composition which is detectable.
As used herein, a "direct labels" refers an entity, which in its natural state, is readily visible, either to the naked eye, or with the aid of an optical filter and/or applied stimulation, e.g. ultraviolet light to promote fluorescence. Examples include, but are not limited to, colored labels, metallic sol particles, dye sol particles dyed latex or dyes encapsulated liposomes (described by U.S. Pat. No. 4,313,734, U.S. Pat. No. 4,373,932, WO 88/08534), EP-A 0 280 559, 0 281 327, U.S. Pat. No. 4,703,017). Other direct labels include a radionucleotide, radiopaque substances, a fluorescent moiety or a luminescent moiety.
As used herein, "indirect labels" refers to enzymes can also be used according to the present invention. Various types of enzyme linked immunoassays are well known in the art, for example, alkaline phosphatase and horseradish peroxidase, lysozyme, glucose-6-phosphate dehydrogenase, lactate dehydrogenase, urease, these and others have been discussed in detail by Eva Engvall in Enzyme Immunoassay ELISA and EMIT in Methods in Enzymology, 70:419-439 (1980) and in U.S. Pat. No. 4,857,453.
As used herein, the term "kit" refers to an article of manufacture comprising one or more containers and a label or package insert on or associated with the containers. In a preferred embodiment, the containers may contain a detectable-labeled antibody, detectable-labeled antibody fragments or detectable-labeled oligonucleotides. In another embodiment, the containers provide the means to obtain total RNA from a bodily sample, reverse transcribe the total RNA to obtain cDNA and subject the cDNA to a polymerase chain reaction using a set of primers wherein one or both primers are detectable-labeled. Additional containers of the kit may be included that contain, e.g., diluents and buffers, control antibodies, oligopeptides or small organic molecules. The label or package insert may provide a description of the composition as well as instructions for storage and for the intended in vitro or diagnostic use.

Furthermore, in accordance with the present invention there may be employed conventional molecular biology, microbiology, and recombinant DNA techniques within the skill of the art. Such techniques are explained fully in the literature. See, e.g., Sambrook, Fritsch & Maniatis, Molecular Cloning: A Laboratory Manual, Second Edition (1989) Cold Spring Harbor Laboratory Press, Cold Spring Harbor, New York (herein "Sambrook et al., 1989"); DNA Cloning: A Practical Approach, Volumes I and II (D.N. Glover ed. 1985); Oligonucleotide Synthesis (M.J. Gait ed. 1984); Nucleic Acid Hybridization [B.D. Hames & S.J. Higgins eds. (1985)]; Transcription And Translation [B.D. Hames & S.J. Higgins, eds. (1984)]; Animal Cell Culture [R.I. Freshney, ed. (1986)]; Immobilized Cells And Enzymes [IRL Press, (1986)]; B. Perbal, A Practical Guide To Molecular Cloning (1984); F.M. Ausubel et al. (eds.), Current Protocols in Molecular Biology, John Wiley & Sons, Inc. (1994).

In general, RNAi technology uses synthetic or vector-generated double stranded RNA to induce the degradation of mRNA containing homologous sequences (McManus and Sharp, Nat Rev Genet 3:737, 2002). RNAi screening has been used to elucidate gene function in many organisms such as Caenorhabditis elegans (Simmer et al., PloS Biol 1:E12, 2003), Drosophila (Lum et al., Science 299:2039, 2003) and mammalian cells (Aza-Blanc et al., Mol Cell 12:627, 2003).

In the present invention, HCT116 colon cancer cells were used to screen siRNAs directed against 101 cancer-related genes, mostly comprising the kinase family members, and the docetaxel killing effects were quantified by high-resolution dose-response curves. Using this approach, applicants have shown that under-expression of 9 genes, including BubR1, Bub1, Mad2, Mps1 and GEFT Rac/CDC, can inhibit killing by docetaxel, whereas under-expressing 15 others, including Pim1, p21, TACC3 and Aurora-A, can potentiate docetaxel-induced cell death.

Thus, broadly the present invention extends to diagnostic methods and kits that may be used to identify whether an individual who is either taking or considering taking docetaxel will be resistant or sensitive to the drug.

Applicants have observed drug resistance to docetaxel, whereby drug resistance is associated with the loss of an expanded set of checkpoint control genes including BubR1, Bub1, Mad2, Msp1 and GEFT Rac/CDC.

Increased docetaxel sensitivity corresponds with the loss of any one of several genes, including GBP-1, STK11, RXRA, Hec1, SPF45, Raf1, RELB, MKK3, PRKCD, HSPA1A, BRAF35, Aurora-A, Pim-1, TACC3 and p21waf1/Cip1, and inhibitors against these genes may serve as a valuable adjunct to docetaxel therapy.

The mitotic checkpoint genes serve as a failsafe mechanism to delay anaphase by the inhibition of Cdc20-APC (anaphase-promoting complex), until the kinetochores of sister chromatids are properly attached to the mitotic spindle and aligned at the equator (Zhou et al., J Cell Sci 115:3547, 2002), thus allowing cells to reliably exit mitosis and enter additional cell cycles with a precise complement of DNA. In the presence of microtubule inhibitors, cells exiting from mitosis can no longer properly segregate their chromosomes and typically undergo apoptosis immediately or after several cell cycles (Taylor and McKeon, Cell 89:727, 1997). The combination of microtubule inhibitors with loss of mitotic checkpoint genes such as BubR1 allows cells to escape mitotic arrest without undergoing apoptosis and lead to chromosome instability (CIN) and aneuploidy (Shin et al., Cancer Cell 4:483, 2003). Recently, drug resistance data were generated when microtubule inhibitor paclitaxel was used to treat BubR1 and Mad2 down regulated MCF-7 cells (Sudo et al., Cancer Res 64:2502, 2004).

The Pim-1 gene encodes a serine/threonine kinase that belongs to a small family of related kinases. Pim-1 function has been linked to proliferation, differentiation, apoptosis, tumorigenesis, hypoxia, angiogenesis and mitosis (Wang et al., Biochim Biophys Acta 1593:45, 2002). The microscopic imaging data, in conjunction with AKT hypo-phosphorylation data and elevated caspase-3 activity measurements, demonstrate that Pim-1 down regulation enhanced docetaxel-induced apoptosis by inactivating AKT signaling, which mediates cell survival. Pim-1 and p21 have very similar characteristics in sensitizing HCT116 cells to docetaxel; consistent with reports showing that p21 is a phosphorylation substrate of Pim-1 (Wang et al., Biochim Biophys Acta 1593:45, 2002). Pim-1 down regulation induced some apoptosis in the absence of docetaxel but was more effective in the presence of drug, whereas down regulation of transforming acidic coiled-coil protein TACC3 did not induced cell death in the absence of docetaxel, indicating the RNAi alone had no effect on cell proliferation. These results suggest that depletion of different proteins can evoke different mechanisms for sensitizing cells to docetaxel.

Accordingly, in one aspect of the invention, activation of one or more of the genes described in Table I and II and protein levels of these genes may be utilized to select a therapeutic treatment strategy and to monitor effectiveness of the selected treatment strategy.

In a particular embodiment, a method is provided that monitors the activation levels of one or more genes shown in Table I and II during the course of chemotherapy to assess and predict effectiveness of chemotherapy for the patient. Biopsies from tumors (e.g., breast, colon, non-small cell lung, and gastric tumors) or short-term culture of tumor biopsies from undiagnosed cancer patients or patients currently undergoing treatment is processed in order to isolate DNA (Hafner et al., Arch Pathol Lab Med 127:1221, 2003; Rodriguez et al., Clin Cancer Res 10:5785, 2004), messenger RNA (Chang et al., Lancet. 362:340, 2003) and/or proteins (Espina et al., J Immunol Methods 290:121, 2004) to assess the levels of gene activation or protein levels of the genes described in Table I and II or a subset thereof. Gene transcription and protein expression profiling has utility in diagnosis, prediction of therapeutic responses to potential treatment strategies and may be a useful tool to monitor a patient's response to therapy.

According to the present invention, RNA may be isolated from tumor samples using any of the available methods commonly used in the art (Ullmann et al., JBiomol Screen 9:95, 2004; Badiee et al., BMC Biotechnol 3:23, 2003). One or more cells from test subjects are obtained and RNA is isolated from these cells. As examples, peripheral blood leukocytes (PBLs) cells may be obtained from the subject or it is also possible to obtain a cell sample and enrich the sample for a desired cell type. Cells may be isolated from a cell mixture using a variety of techniques, such as cell isolation using antibodies that bind to an particular epitope on the desired cell type. Where the desired cells are in a solid tissue, particular cells may be dissected, for example, by microdissection or by laser capture microdissection (LCM) (Bonner et al., Science 278:1481, 1997; Fend et al., Am J Path 154:61, 1999). RNA may be extracted from tissue or cell samples by a variety of methods, for example, guanidium thiocyanate lysis followed by CsCl centrifugation (Chirgwin et al., Biochemistry 18:5294, 1979). RNA from single cells may be obtained as described in methods for preparing cDNA libraries from single cells (Dulac, Curr Top Dev Biol. 36:245, 1998). The RNA sample can be further enriched for a particular species. In one embodiment, for example, poly(A)+RNA may be isolated from an RNA sample. In particular, poly-T oligonucleotides may be immobilized on a solid support to serve as affinity ligands for mRNA. Kits for this purpose are commercially available, for example, the MessageMaker kit (Life Technologies, Grand Island, N.Y.). Enrichment may be accomplished, for example, by primer-specific cDNA synthesis, or multiple rounds of linear amplification based on cDNA synthesis and template-directed in vitro transcription (Wang et al., Proc Natl Acad. Sci USA 86:9717, 1989; Dulac *et al.*, supra).

A variety of amplification methods are suitable for use in the methods of the present invention, including, for example, PCR; ligase chain reaction (LCR) (Wu and Wallace, Genomics 4:560, 1989); self-sustained sequence replication (SSR) (Guatelli et al., Proc Natl Acad Sci USA 87:1874, 1990); nucleic acid based sequence amplification (NASBA) and transcription amplification (Kwoh et al., Proc Natl Acad Sci USA 86:1173, 1989). Methods for PCR technology are well known in the art (see, e.g., PCR Technology: Principles and Applications for DNA Amplification (ed. H. A. Erlich, Freeman Press, N.Y., N.Y., 1992); PCR Protocols: A Guide to Methods and Applications (eds. Innis, et al., Academic Press, San Diego, Calif.,1990).

The RNA may be labeled for detection by using any of the methods known to those persons skilled in the art. For example, using microarrays, such as those manufactured by Affymetrix. RNA is labeled and detected by hybridization using any of the methods commonly used, such as, direct RNA labeling with dyes such as Cyanine-3 or Cyanine-5 (Perkin Elmer MPS544001KT). Additional methods include direct cDNA labeling that is done by reverse transcription using nucleotides labeled with Cyanine-3 or Cyanine-5 (Badiee A 2003), fluorescein or Alexa dyes (Molecular Probes), or other fluorophores. Furthermore, indirect cDNA labeling such as FairPlay™/aminoallyl labeling (Stratagene Catalog number: 252002), 3DNA dendrimer labeling (Genisphere Inc) or enzymatic signal amplification (MICROMAX TSA, Perkin Elmer) may be utilized. Alternatively, RNA may be quantified by using RNA probes that are detected by enzymatic, fluorescence, radioactive or light emitting methods.

In one aspect of the present invention, RNA is obtained from the tumor sample and Taqman technology is employed in the detection of genetic markers. Primers are generated to detect the specific RNAs designated in Table I and II or subset thereof and amplified using reverse transcriptase. Detection of the specific fragment amplified by reverse transcription is accomplished by gel electrophoresis, polymer electrophoresis, direct DNA sequencing, light detection, loss of a fluorescence signal, enzymatic reaction, or detection by hybridization to a complementary RNA.

In another aspect of the present invention, Real Time PCR is utilized to detect oligonucleotides. The mRNA obtained from a tumor sample is reverse translated into cDNA. Primers are generated to amplify the specific RNAs designated in Table I and II or subset thereof using polymerase chain reaction technology. Detection of mRNA level is achieved by gel electrophoresis and ethidium bromide or CYBR green staining, or by measuring the fluorescence intensity from fluorophore-labeled sequence specific probes released by polymerase.

In a further aspect of the present invention, Northern blotting technology is utilized. RNA is obtained from the tumor sample and then separated by gel electrophoresis and transferred onto a membrane. RNA abundance is determined by hybridization using probes labeled with radioactive isotope such as P³² or by enzymatic based chromogenic or luminescent assays.

In yet another aspect of the present invention, gene dosage may be used as a surrogate indicator of transcript levels. The gene dosage of the transcripts in Table I and II or a subset thereof may be quantitatively determined and assessed for whether a tumor will be responsive or not to docetaxel therapy. (Rodriguez et al., Clin Cancer Res 10:5785, 2004). Since this assessment may be drawn prior to neoadjuvant (treatment prior to surgery) therapy, the patient may be treated with docetaxel if they are classified as a responder or another chemotherapeutic agent may be administered if they are classified as a non-responder.

DNA is extracted from patient tumor biopsies and purified to remove contaminants using methods as performed by those skilled in the art. Methods utilized to determine the gene dosage in the tumor DNA include, but are not limited to, quantitative PCR, genomic DNA-chips, in situ hybridization or Southern (Hafner et al., Arch Pathol Lab Med 127:1221, 2003; Rodriguez et al., Clin Cancer Res 10:5785, 2004). Accordingly, these methods may be utilized to determine copy number of one or more genes in Table I and II and compared to a control sample or reference markers.

In yet another aspect of the present invention, protein levels are used as a surrogate measure of transcript levels. Protein levels have been shown to correlate with suppression or elevation of the transcript levels. Thus, the present invention encompasses techniques to measure levels of proteins that correspond to the polypeptide products encoded by the transcripts in Table I and II. Proteins are detected and used as markers of a predictive response to docetaxel. Methods of protein detection are well known in the art. For example, immunoassays are methodologies that employ antibodies to detect the expression of a target protein.

In a particular embodiment, an immunoassay is used to detect the protein products of one or more genes listed in Table I and II. Additionally, antibodies against any one of the proteins listed in Table I and II may be used in a number of other detection methods. These detection methods include, but are not limited to, the following: Western blots, ELISA assays, sandwich ELISA. Alternatively, other assays not employing antibodies are contemplated and include approaches that utilize DNA oligonucleotides or polypeptides that recognize proteins encoded by transcripts in Table I and II (e.g., aptamers). Mass spectrometric analysis of biological samples may also be used that determine the composition of polypeptides by determining the precise molecular weight of peptide fragments after proteolytic cleavage.

The present invention further encompasses suitable labels including enzymes, fluorophores (e.g., fluorescein isothiocyanate (FITC), phycoerythrin (PE), Texas red (TR), rhodamine, free or chelated lanthanide series salts, chromophores, radioisotopes, chelating agents, dyes, colloidal gold, latex particles, ligands (e.g., biotin), and chemiluminescent agents.

In an aspect of the present invention, the radioactive label used may be isotopes such as 3H, 14C,32P, 35S, 36 Cl, 51 Cr, 57Co, 58Co,59Fe, 90Y, 125 I, 131I, and 186Re. Quantification of radioactivity levels may be performed through currently known and available counting procedures. Conversely, the use of radiopaque substances may be used. In the embodiment where the label is an enzyme, detection may be accomplished by any of the presently utilized colorimetric, spectrophotometric, fluorospectrophotometric, amperometric or gasometric techniques known in the art.

A further aspect of the invention is the use of peptide binding agents such as antibodies or fragments of antibodies. Antibodies include polyclonal and monoclonal antibodies, prepared according to conventional methodology. Only a small portion of an antibody molecule, the paratope, is involved in the binding of the antibody to its epitope (see, in general, Clark, W. R (1986) The Experimental Foundations of Modem Immunology Wiley & Sons, Inc., New York; Roitt, I. (1991) Essential Immunology, 7th Ed., Blackwell Scientific Publications, Oxford). The pFc' and Fc regions, for example, are effectors of the complement cascade but are not involved in antigen binding. An antibody from which the pFc' region has been enzymatically cleaved, or which has been produced without the pFc' region, designated an F(ab')2 fragment, retains both of the antigen binding sites of an intact antibody. Thus, an embodiment of the present invention utilizes a detectable-lableled antibody fragment such as the F(ab')2 fragment. Similarly, an antibody from which the Fc region has been enzymatically cleaved, or which has been produced without the Fc region, designated an Fab fragment, retains one of the antigen binding sites of an intact antibody molecule. Fab fragments consist of a covalently bound antibody light chain and a portion of the antibody heavy chain denoted Fd. The Fd fragments are the major determinant of antibody specificity (a single Fd fragment may be associated with up to ten different light chains without altering antibody specificity) and Fd fragments retain epitope-binding ability in isolation.

As is well-known in the art, within the antigen-binding portion of an antibody, there are complementarity determining regions (CDRs), which directly interact with the epitope of the antigen, and framework regions (FRs), which maintain the tertiary structure of the paratope (see, in general, Clark, 1986; Roitt, 1991). In both the heavy chain Fd fragment and the light chain of IgG immunoglobulins, there are four framework regions (FR1 through FR4) separated respectively by three complementarity determining regions (CDR1 through CDR3). The CDRs, and in particular the CDR3 regions, and more particularly the heavy chain CDR3, are largely responsible for antibody specificity. It is now well-established in the art that the non-CDR regions of a mammalian antibody may be replaced with similar regions of conspecific or heterospecific antibodies while retaining the epitopic specificity of the original antibody. This is most clearly manifested in the development and use of "humanized" antibodies in which non-human CDRs are covalently joined to human FR and/or Fc/pFc' regions to produce a functional antibody (refer to U.S. Pat. Nos. 4,816,567, 5,225,539, 5,585,089, 5,693,762 and 5,859,205).

Fully human monoclonal antibodies also can be prepared by immunizing mice transgenic for large portions of human immunoglobulin heavy and light chain loci. Following immunization of these mice (e.g., XenoMouse (Abgenix), HuMAb mice (Medarex/GenPharm)), monoclonal antibodies can be prepared according to standard hybridoma technology. These monoclonal antibodies will have human immunoglobulin amino acid sequences and therefore will not provoke human anti-mouse antibody (HAMA) responses when administered to humans.

Therefore, it is readily apparent to one of ordinary skill in the art, the present invention also encompasses F(ab')2, Fab, Fv and Fd fragments; chimeric antibodies in which the Fc and/or FR and/or CDR1 and/or CDR2 and/or light chain CDR3 regions have been replaced by homologous human or non-human sequences; chimeric F(ab')2 fragment antibodies in which the FR and/or CDR1 and/or CDR2 and/or light chain CDR3 regions have been replaced by homologous human or non-human sequences; chimeric Fab fragment antibodies in which the FR and/or CDR1 and/or CDR2 and/or light chain CDR3 regions have been replaced by homologous human or non-human sequences; and chimeric Fd fragment antibodies in which the FR and/or CDR1 and/or CDR2 regions have been replaced by homologous human or non-human sequences. The present invention also includes so-called single chain antibodies.

The present invention may be better understood by reference to the following nonlimiting Examples, which are provided as exemplary of the invention. The following Examples are presented in order to more fully illustrate the preferred embodiments of the invention. They should in no way be construed, however, as limiting the broad scope of the invention.

### EXAMPLES

### EXAMPLE 1: siRNA Screening

In order to identify genes that render cells more resistant or sensitive to docetaxel treatment, siRNAs against 101 genes were screened in HCT116 cells using a range of docetaxel concentrations for the generation of dose-response curves. Human colon cancer cell line HCT116 was obtained from ATCC and cultured in McCoy 5A supplemented with 100 units/ml penicillin, 100 µg/ml streptomycin, 4 mM L-glutamine and 10% fetal bovine serum at 37°C with 95% CO2 and 5% 02. The siRNA list was comprised of cancer-related genes, including those that were over expressed in docetaxel-resistant breast tumors as detected by gene expression profiling experiments (Chang et al., Lancet 362:362, 2003). Additional criteria were that the targets be amenable to drug development. The overall scheme is summarized as follows. On the first day, HCT116 cells were plated at 5,000 cells per well in a 96-well format and on the following day transfected with a pool of 3-4 siRNAs per gene. A representative sample of the siRNAs used is shown in Table I and II. Lipofectamine 2000 (Invitrogen) was used to transfect siRNAs (Dharmacon) into HCT 116 cells in 96 well plates. On the third day siRNA was removed and docetaxel was added at concentrations ranging from 0 to 40nM. On the sixth day cell viability was quantified using WST-1 assay. This is an assay that monitors the activity of mitochondrial dehydrogenases present in viable cells that results in the cleavage of tetrazolium salt WST-1 to form formazan. On the day of assay, media were taken off from 96 well plates. WST-1 reagent (Roche) was diluted 10 times in McCoy 5A medium and 100ul was added to each well. Plates were then incubated for 40 - 80 minutes at 37°C before reading on SpectroMax (Molecular Devices) at 450nm. The WST-1 values were clustered hierarchically by calculating the ratio of the experimental to the control siRNA and a total of 15 genes were characterized as conferring sensitivity, and 9 genes conferring resistance shown in Table III below. The sensitive and resistant populations could be further divided into additional groups: 1) where the siRNA effect was observed at lower concentrations of docetaxel (1-6 nM) to shift the IC50 or 2) where the major effect was observed at higher concentrations (> 6nM) with a smaller shift in IC50.

As compared to Mad2, BubR1 and Mps1 siRNAs, the siRNAs targeting Grb2, CDK6, sepharase, and calcium/calmodulin-dependent protein kinase II delta (CamKIID) showed more pronounced effects at lower docetaxel concentrations (1-6 nM), with a characteristic shift in the IC50. Overall, this subgroup showed weaker resistance than the mitotic checkpoint genes and the CamKIID siRNA generated the greatest level of protection, which was two-fold over control cells. These data demonstrate that loss of four mitotic checkpoint genes along with several other genes can increase docetaxel resistance to varying degrees. The data in Figure 1 show several characteristic dose-response curves for cells transfected with siRNAs followed by exposure to various concentrations of docetaxel. The siRNAs against four mitotic checkpoint genes, including BubR1, Bub1, Mad2, and Mps1 (data not shown for Bub1) as well as guanine nucleotide exchange factor (GEFT) for Rac/Cdc42 demonstrated significant docetaxel resistance with the effect being more pronounced at higher docetaxel concentrations (>6 nM). The greatest level of resistance was shown for Mad2 where cell viability increased five-fold over the control siRNA transfected cells. The majority of the siRNAs did not score in the screen and here we show human retinoblastoma 1 (RB 1) as a representative example.

Taken together, these results show that siRNA screening can provide insights about how down-regulating gene expression sensitizes or protects cells from docetaxel treatment and, depending on the concentrations, evokes different mechanisms of action to modulate docetaxel-mediated cell death.

| **Table I: Sequences of siRNAs Conferring Increased Sensitivity to Docetaxel:** | | | | |
|---|---|---|---|---|
| **Gene Name** | **siRNA sequence** | **SEQ ID NO** | **siRNA sequence** | **SEQ ID NO** |
| P21(Waf1) | GCGAUGGAACUUCGACUUU | 1 | AGACCAUGUGGACCUGUCA | 3 |
| | UGGAACUUCGACUUUGUCA | 2 | | |
| Pim-1 | CCUUCGAAGAAAUCCAGAA* | 4 | UAUUCCUUUCGAGCAUGAC | 6 |
| | UGGUGUGUGGAGAUAUUCC | 5 | | |
| GBP-1 | CGAAAGGCAUGUACCAUAA | 7 | GAACAGGAGCAACUACUAA | 9 |
| | GAUACAGGCUGAAGAGAUU | 8 | | |
| RXRA | GCAAGGACCUGACCUACAC | 10 | GACCCUGUCACCAACAUUU | 12 |
| | GCAAGGACCGGAACGAGAA | 11 | | |
| SPF45 | CAAAUCCGCUGACUGAAAU | 13 | GACCCUAUGUUUCCUAAUG | 15 |
| | GAACAAGACAGACCGAGAU | 14 | | |
| Hec1 | GUGUAUUCGACAACUCUGU | 16 | GUACUCAGUUGCAGACAUU | 18 |
| | GAUUGCAAGAUUGGAACAA | 17 | GUAUCACAAAUUGGCUAGA | 19 |
| Raf1 | GCACGGAGAUGUUGCAGUA | 20 | GACAUGAAAUCCAACAAUA | 22 |
| | GCAAAGAACAUCAUCCAUA | 21 | GGAAUGAGCUUGCAUGACU | 23 |
| Aurora A | GAGAACUGCUACUUAUAUA | 24 | GAAGGUCGGAUGCAUGAUG | 26 |
| | GAAUAUGCACCACUUGGAA | 25 | GUAAAGGAAAGUUUGGUAA | 27 |
| TACC3 | GCACCUCGCUUCCCACAAG | 28 | GAACGAAGAGUCACUGAAG | 30 |
| | GAGCGGACCUGUAAAACUA | 29 | | |
| RelB | GCAGAAAGAGGACAUAUCA | 31 | GCCCGUCUAUGACAAGAAA | 33 |
| | GCAGCGAGCCAUUGCCUUU | 32 | | |
| PRKCD | GGACGUGGAUUGCAAACAA | 34 | GAAAGAACGCUUCAACAUC | 36 |
| | GCAUGAAUGUGCACCAUAA | 35 | | |
| BRAF35 | GGGCGUACCAGCAGUCUGA | 37 | GUCUGAAGCCUAUAAGAUG | 39 |
| | GCUCUGGGCUCAUGAACAC | 38 | | |
| HSPA1L | GAGAUCGACUCCCUGUUUG | 40 | GAUCAACGACGGAGACAAG | 42 |
| | UGGAGGAGUUCAAGAGAAA | 41 | | |
| STK11 | GAAGAAGGAAAUUCAACUA | 43 | GAAACAUCCUCCGGCUGAA | 45 |
| | GAGAAGCGUUUCCCAGUGU | 44 | | |
| MKK3 | GGUGGAGGCUGAUGACUUG | 46 | GGAGGGCCAUGUGAAGAUG | 48 |
| | GGAGAUUGCUGUGUCUAUC | 47 | GAUGUGAAGCCCUCCAAUG | 49 |
| *Individual sequences having demonstrated good knockdown | | | | |

| **Table II: Sequences of siRNAs Conferring Decreased Sensitivity to Docetaxel:** | | | | |
|---|---|---|---|---|
| **Gene Name** | **siRNA sequence** | **SEQ ID NO** | **siRNA sequence** | **SEQ ID NO** |
| BubR1 | GCAAUGAGCCUUUGGAUAU* | 50 | GGAAGAAGAUCUAGAUGUA | 52 |
| | GGAACAACCUCAUUCUAAA | 51 | | |
| Mad2 | GAAAUCGUGGCCGAGUUCU | 53 | GCCGAGUUCUUCUCAUUCG | 55 |
| | GUGGCAUAUAUCCAUCUGA | 54 | GGAACAACUGAAAGAUUGG | 56 |
| Mps1 | GUCGUUACAGUCAAGCAAU* | 57 | GAUGAACUAAGCUUGAAUA | 59 |
| | GCACGUGACUACUUUCAAA | 58 | GAGCAGUACCACUAGAAAU | 60 |
| GEFT | GAACACAGCCUGGAUAUGU | 61 | GGAUGAAGAUGAGCUGUAA | 63 |
| | GCACCGAGACUAUUUCUUG | 62 | GCAUGUGGCUCAGAUCUUG | 64 |
| Bub1 | GUACAACAGUGACCUCCA | 65 | GCUUGUGAUAAAGAGUCAA | 67 |
| | GAUGCUGGAUGUGUGAAUA | 66 | GAUCCACCAGAUGCUAUUG | 68 |
| hSeparase | GCUGUCAGAUAGUUGAUUU | 69 | GUGGACAGUUGUAAAUCUA | 71 |
| | GCCUACAGCUUCUAUAGUC | 70 | GAAGAUCGUUUCCUAUACA | 72 |
| CamKIID | GCACGAAAGCAAGAGAUUA | 73 | GCUAGAAUCUGCCGUCUUU | 75 |
| | GAAGAAACCAGAUGGAGUA | 74 | GAUCAAGGCUGGAGCUUAU | 76 |
| CDK6 | GAACAUGUCGAUCAAGACU | 77 | GAGUAGUGCAUCGCGAUCU | 79 |
| | GUUUGUAACAGAUAUCGAU | 78 | GUAACAGAUAUCGAUGAAC | 80 |
| GRB2 | GAACGAAGAAUGUGAUCAG | 81 | GGUACAAGGCAGAGCUUAA | 83 |
| | GUGGAUUAUCACAGAUCUA | 82 | GUGCCACAGCAGCCGACAU | 84 |
| | | | | |

| **Control Sequences:** | | | | |
|---|---|---|---|---|
| | GUCGACCAUUAUGUACCGGAU | 85 | GGAUUCGUAACGUGAUUAGCG | 86 |
| | | | | |

| **Sequences of siRNAs Not Affecting Docetaxel Response:** | | | | |
|---|---|---|---|---|
| RB1 | GAAACAGAAGAACCUGAUU | 87 | GUACAUCUCAGAAUCUUGA | 89 |
| | GAAAGGACAUGUGAACUUA | 88 | | |
| | | | | |
| * Individual sequences having demonstrated good knockdown | | | | |

| **siRNAs Conferring Increased Sensitivity to Docetaxel:** | | | | | | |
|---|---|---|---|---|---|---|
| **Gene Name** | **SEQ ID NO** | **Accession number** | **IC50 ratio** | **p(IC50 ratio)** | **Min ratio** | **p(Min ratio)** |
| P21(Waf1) | 90 | NM_000389 | 0.70 | 0.0004 | 0.25 | 0.0000 |
| Pim-1 | 91 | NM_002648 | 0.80 | 0.0011 | 0.30 | 0.0000 |
| GBP-1 | 92 | NM_002053 | 0.59 | 0.0000 | 0.25 | 0.0000 |
| RXRA | 93 | NM_002957 | 0.64 | 0.0011 | 0.36 | 0.3098 |
| SPF45 | 94 | NM_032905 | 0.72 | 0.0000 | 0.47 | 0.0000 |
| Hec1 | 95 | NM_006101 | 0.64 | 0.0000 | 0.31 | 0.0121 |
| Raf1 | 96 | X03484 | 0.90 | 0.0059 | 0.30 | 0.0000 |
| Aurora A | 97 | AF008551 | 0.73 | 0.0000 | 1.37 | 0.0018 |
| TACC3 | 98 | NM_006342 | 0.29 | 0.0000 | 0.85 | 0.0077 |
| RelB | 99 | NM_006509 | 0.56 | 0.0000 | 0.77 | 0.0052 |
| PRKCD | 100 | NM_006254 | 0.46 | 0.0683 | 0.59 | 1.0000 |
| BRAF35 | 101 | NM_006339 | 0.44 | 0.0000 | 1.29 | 0.1361 |
| HSPA1L | 102 | NM_005345 | 0.60 | 0.0404 | 1.08 | 1.0000 |
| STK11 | 103 | NM_000455 | 0.94 | 0.4544 | 0.27 | 0.0000 |
| MKK3 | 104 | L36719 | 0.51 | 0.0000 | 0.82 | 0.0553 |

| **siRNAs Conferring Increased Resistance to Docetaxel:** | | | | | | |
|---|---|---|---|---|---|---|
| **Gene Name** | **SEQ ID NO** | **Accession number** | **IC50 ratio** | **p(IC50 ratio)** | **Min ratio** | **p(Min ratio)** |
| BubR1 | 105 | AF046079 | 1.88 | 0.2207 | 3.01 | 0.5663^ |
| Mad2 | 106 | AJ000186 | 3.22 | 0.0076 | 5.06 | 1.0000^ |
| Mps1 | 107 | NM_003318 | 3.55 | 0.0008 | 2.92 | 1.0000^ |
| GEFT for Rac1/CDC42 | 108 | NM_133483 | 3.50 | 1.0000 | 2.42 | 1.0000^ |
| Bub1 | 109 | NM_004336 | 1.38 | 0.0007 | 2.45 | 0.0000 |
| hSeparase | 110 | NM_012291 | 1.99 | 0.0000 | 0.88 | 1.0000 |
| CamKIID | 111 | NM_001221 | 2.03 | 0.0016 | 1.29 | 1.0000 |
| CDK6 | 112 | NM_001259 | 1.99 | 0.0000 | 1.14 | 1.0000 |
| GRB2 | 113 | NM_002086 | 1.97 | 0.0000 | 1.16 | 1.0000 |

| **siRNAs Having No Effect on the Response to Docetaxel:** | | | | | | |
|---|---|---|---|---|---|---|
| **Gene Name** | **SEQ ID NO** | **Accession number** | **IC50 ratio** | **p(IC50 ratio)** | **Min ratio** | **p(Min ratio)** |
| RB1 | 114 | NM_000321 | 0.87 | 0.0061 | 1.03 | 0.4863 |
| GAPDH | 115 | NM_002046 | nd | nd | nd | nd |
| RPS9 | 116 | BC071941 | nd | nd | nd | nd |
| **Notes:** | | | | | | |
| Min ratio: The ratio of a siRNA over the control at the highest docetaxel dose | | | | | | |
| The smaller the p value is, the more significant the difference between sample and control | | | | | | |
| The p values marked with ^ are high because of poor curve fitting on the dose response curves, even though the differences are significant and reproducible | | | | | | |
| Nd=not determined | | | | | | |

### EXAMPLE 2: Cross Validation on a Subset of siRNAs that Confer Increased Docetaxel Resistance

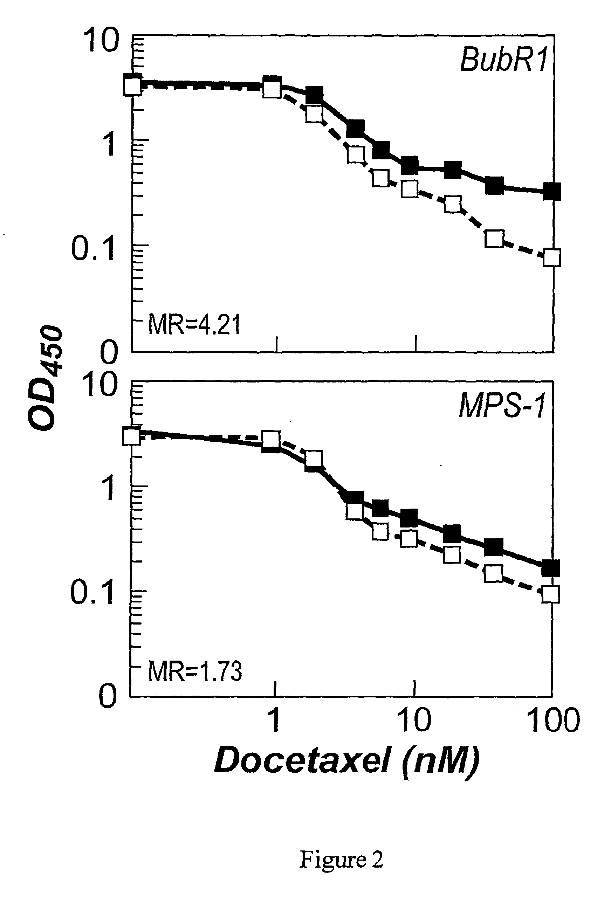

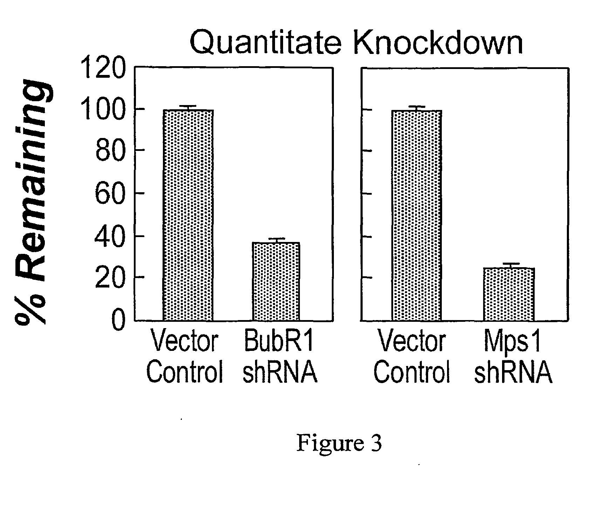

A potential drawback to siRNA screening approaches rests on the observation that siRNAs can anneal to transcripts with partial identity or initiate micro-RNA (miRNA) translational blocks, making it difficult to distinguish between target-specific effects and nonspecific (off-site) effects (Jackson et al., Nat Biotechnol 21:635, 2003). To confirm resistance using an independent approach with a second sequence, stable knock-down cells lines were generated for BubR1 and Mps1 by infecting HCT116 cells with a retroviral vector carrying DNA oligonucleotides encoding a short hairpin (sh)RNA. The shRNA contained a different sequence than the siRNA, targeting a distinct region of the open reading frame. The retroviral packaging cell line GP2-293 was obtained from Clontech. The cells were maintained in DMEM supplemented with 100 units/ml penicillin, 100 µg/ml streptomycin, 4 mM L-glutamine and 10% fetal bovine serum. Virus was generated by transiently transfecting GP2-293 cells. A total of 3.6 x 10⁶ cells were seeded in a 10 cm dish 24 hours prior to transfection. The medium was replaced 4 hours prior to transfection with DMEM containing 10% FBS minus antibiotics. The cells were transfected with 6 µg vector DNA, 6 µg of envelope plasmid VSV-G (Clontech) and 72 µl Lipfectamine-2000. The medium was replaced after 14-16 hours; the viral supernatant was harvested 24 hours later, filtered through a 0.45 µM filter and used to infect HCT116 cells at an M.O.I of 5 in the presence of 8 µg/ml polybrene. Forty-eight hours post-infection the cells were selected in 0.5 µg/ml puromycin for 7 days or until the background cells died off. The dose response curves generated with the BubR1 and Mps1 knock-down cell lines again showed enhanced resistance that was more pronounced at higher docetaxel concentrations (Figure 2), similar to the results obtained by siRNA transient transfections. To examine whether the shRNAs reduced mRNA levels we employed real-time PCR to detect endogenous transcripts and confirmed that the RNA levels were diminished in the BubR1 and Mps1 stable knock-down cell lines as compared to the vector control stable line (Figure 3).

Cells were lysed and RNA was extracted using RNAqueous -96 (Ambion). Taqman™ probes and forward and reverse primers were designed with Primer Express™ software (PE Applied Biosystems, UK). BLAST searches of the probe and primer sequences revealed no significant identity to other sequences other than the specific gene under test. For real time PCR, 20 µl of master TaqMan™ mix was made for each well: 4.825 µl Rnase-free water, 12.5 µl 2x Universal PCR master mix and 0.625 µl 40x MultiscribeTM and Rnase Inhibitor Mix (Applied BioSystems), 0.9 µl forward and reverse primers (100 µM), 0.25ul probe (100 µM). To each well, 5ul sample RNA (about 1ng/µl) was added. Plates were analyzed on a TaqMan™ ABI Prism 7700 Sequence Detector™ (Perkin-Elmer, UK). Cycling parameters were 48°C for 30 minutes, 95°C for 10 minutes, 40 cycles of 95°C for 15 seconds, and 60°C for 1 minute. Test gene mRNA values were extrapolated from the standard curve and presented as percent remaining.

### EXAMPLE 3: Downregulation of Mad2, BubR1 and Mps1 Protects Cells from Docetaxel-Induced Death by Escaping Mitotic Arrest, Coupled with the Generation of Aneuploidy

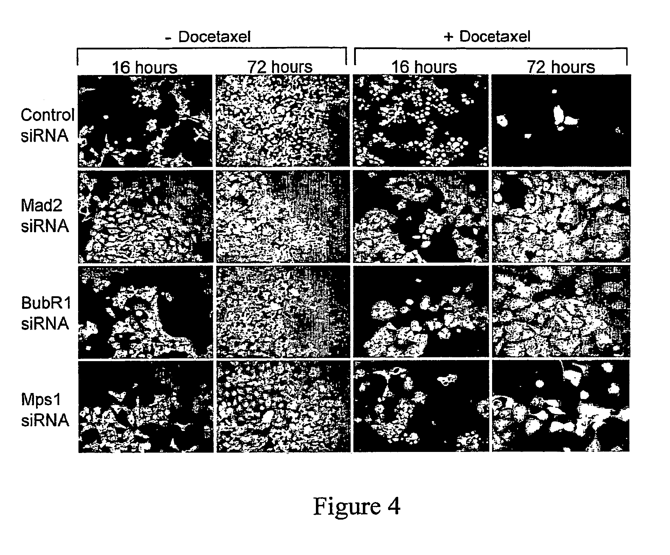

As determined by the WST-1 assay, loss of mitotic checkpoint genes in the presence of docetaxel correlated with increased cell viability to signify resistance (Figure 1). To further test the validity of WST-1 measurements, cytological experiments were performed to look at cell morphology and viability (Figure 4). The cells were tracked using confocal microscopy and Calcein-AM, a vital dye converted to green fluorescence by intracellular esterase serving as an indicator of metabolically active cells. Figure 4 demonstrates that 16 hours post docetaxel addition, the Mad2, BubR1 and, to a lesser extent, Mps1 siRNA transfected cells prematurely exited mitosis into an apparent interphase state coincident with a flattened cell morphology, whereas the majority of the control siRNA transfected cells were arrested in mitosis with a rounded up cell morphology. The flat cell phenotype has been previously described for other microtubule inhibitors and refers to the ability of cells to exit from mitotic arrest into an apparent interphase state without actually completing mitosis (Kung et al., Proc Natl Acad Sci 87:9553, 1990; Lanni and Jacks, Mol Cell Biol 18:1055, 1998). At 72 hours post treatment, the most striking observations for Mad2, BubR1 or Mps1 siRNA down-regulated cells were the presence of many more cells and their very large size, as compared to the siRNA control transfected cells that were virtually eliminated by docetaxel treatment. In the absence of docetaxel, mitotic checkpoint down-regulated cells looked similar to control cells at the two different time points and the increased number of cells at 72 hours suggested the cells were actively growing in the presence of the siRNA.

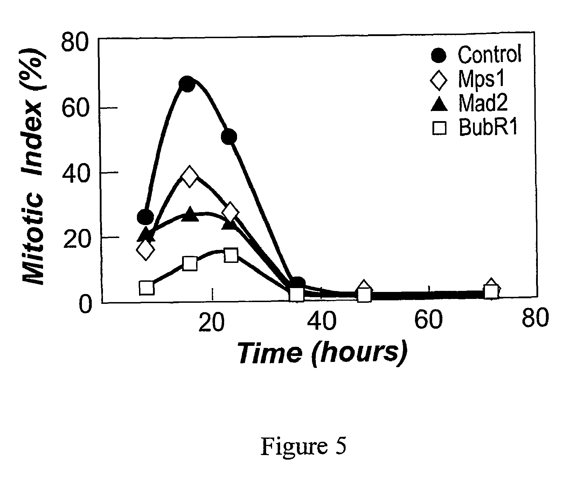

Mitotic checkpoint-impaired cells can bypass mitotic arrest and prematurely exit mitosis following treatment with microtubule inhibitors such as, colcemid, nocodazole and paclitaxel (Taylor and McKeon, Cell 89:727, 1997; Shin et al., Cancer Cell 4:483, 2003; Masuda et al., Am J Pathol 163:1109, 2003; Sudo et al., Cancer Res 64:2502, 2004). To determine if docetaxel treated HCT116 cells prematurely exit from mitosis following suppression of three specific checkpoint genes Mad2, BubR1 or Mps1, we measured mitotic indices of transfected cells. Accordingly, siRNA transfected Mad2, BubR1 or Mps1 cells were harvested without (0) treatment or at 8, 16, 24, 36, 48 and 72 hours after docetaxel treatment, then fixed and incubated with a polyclonal antibody that detects phosphorylation of histone H3 as an indication of mitosis (Mitotic Index Kit, Cellomics). A graphic representation for all time points are shown in Figure 5. The mitotic index peaked between 16 and 24 hours post treatment indicating that in all cases there was mitotic arrest. The mitotic index reached the highest value for cells transfected with the control siRNA, whereas cells transfected with Mad2 and BubR1 siRNA showed a significant decrease in mitotic index. The Mps1 siRNA effects were not as severe as Mad2 and BubR1. These results clearly show that mitotic checkpoint impaired cells can bypass mitotic arrest induced by docetaxel and that BubR1 siRNA was the most effective followed by Mad2 and Mps1.

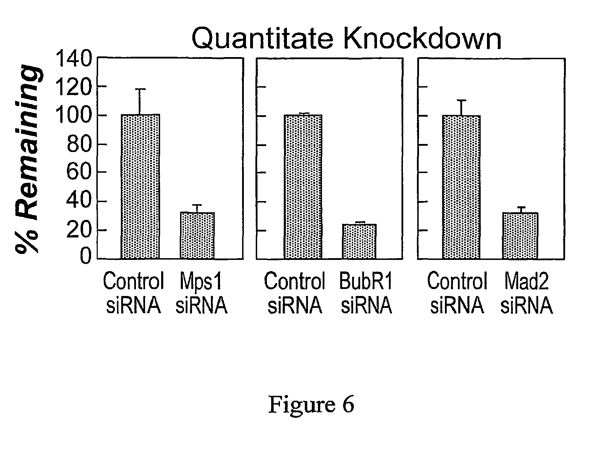

To rule out the possibility that changes in mitotic index were coincident with large differences in knockdown efficiencies, we employed real-time PCR to measure mitotic checkpoint transcript levels. The experiment shown in Figure 6 demonstrates that mRNA levels were diminished to comparable levels in Mad2, BubR1 and Mps1 siRNA transfected cells, suggesting that the mitotic index differences were more reflective of gene function.

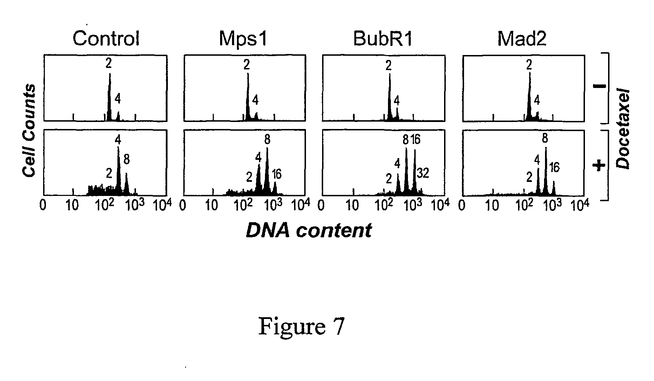

From the microscopy data, it appeared that the very large cells may harbor abnormal DNA content. In order to further investigate whether these mitotic checkpoint impaired cells were capable of overriding docetaxel-induced cell cycle arrest, and re-enter S-phase to generate aneuploid cells, we performed FACS analysis to measure DNA content. The, Mps1, BubR1, Mad2 and control siRNA transfected cells were grown in the absence or presence of docetaxel, fixed and stained with propidium iodide and analyzed by FACS. The data were collected at several different time points post docetaxel addition (data not shown) and histograms for the 72 hour time point are shown in Figure 7. At 72 hours post docetaxel addition, transfections with Mps1, BubR1, Mad2 and siRNAs caused an accumulation of cells with 8N, 16N and in the case of BubR1 32N DNA content. By just using the control siRNA, an 8N population of cells was detected in the presence of docetaxel, but the 8N numbers was far greater for the mitotic checkpoint down-regulated cells. At an earlier time point following docetaxel addition (data not shown), the majority of control cells were arrested at 4N, whereas cells transfected with Mps1, BubR1 and Mad2 siRNAs were advancing from 4N to 8N. These results indicate that knockdown of mitotic checkpoint genes allow cells to more rapidly accumulate abnormal DNA levels and cross a greater threshold of ploidy as compared to wild-type cells. Our results demonstrate roles for Mad2, BubR1 and Mps1 in regulating mitotic index and cell cycle progression with a previously uncharacterized microtubule inhibitor docetaxel.

### EXAMPLE 4: Formation of Docetaxel Resistant Colonies in BubR1 Downregulated Cells

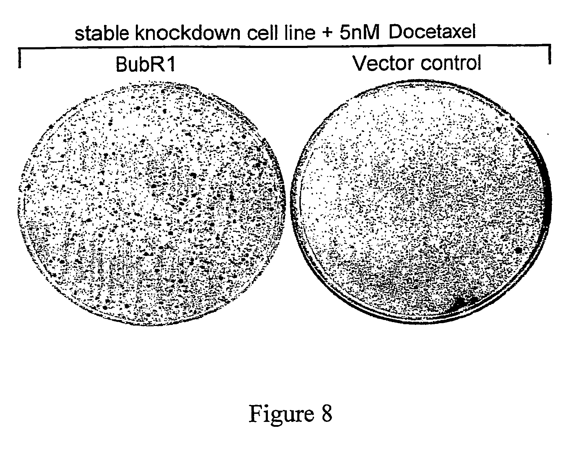

The apoptotic function of BubR1 appears to be important for the regulation of chromosome fidelity given that underexpression of BubR1 in combination with anti-microtubule agents was associated with aneuploidy, and restoration of BubR1 associated with an activated checkpoint and apoptosis of aneuploid cells (Shin et al., Cancer Cell 4:483, 2003). To determine if permanently downregulating mitotic checkpoint genes enables cells to grow with a greater proliferative capacity, the BubR1 and Mps1 stable knockdown lines (Figure 4) were tested for their ability to proliferate and establish colonies in the continuous presence of docetaxel. The Mad2 stable knockdown cell line could not be generated since impairment of Mad2 was lethal to cells (Michel et al., Proc Natl Acad Sci 101:4459, 2004). The BubR1, Mps1 and control shRNA stable knockdown cells lines were subjected to 5 nM docetaxel for 10 days and after that stained with crystal violet. Figure 8 shows that the BubR1 knockdown cell line produced many more large colonies as compared to the control cell line. The Mps1 knockdown cell line was not able to grow out colonies in the presence of 5 nM docetaxel. When the plates were incubated for as long as 17 days, colonies were observed on the control plates but were still fewer in number as compared to the BubR1 knockdown line. These results link low levels of BubR1, but not Mps1, with advanced cell growth in the presence of the chemotherapeutic drug docetaxel.

### EXAMPLE 5: Cross Validation of siRNA that Confers Increased Docetaxel Sensitivity

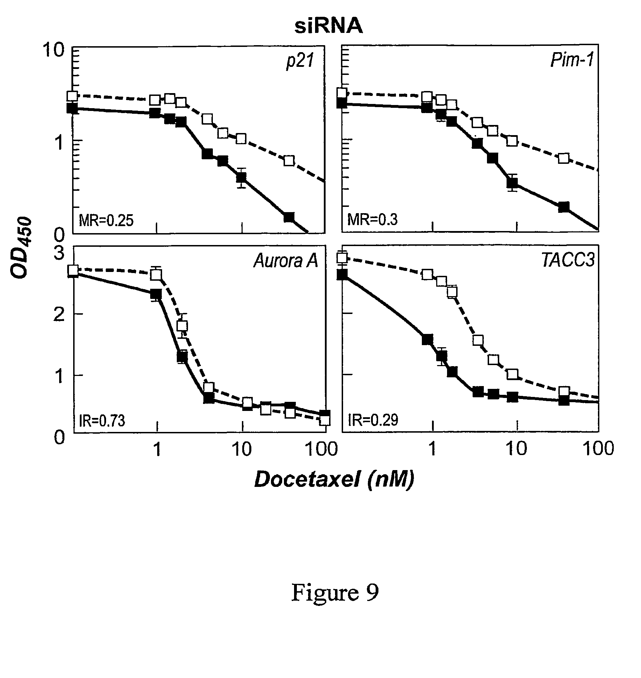

In addition to identifying siRNAs that protect cells from docetaxel-induced killing, the screen identified siRNAs that sensitize cells to killing. In Table III, Pim-1, p21^{waf1/Cip1}, GBP-1, RXRA, Hec1, SPF45, Raf1 represent a group of siRNAs that show pronounced killing at higher docetaxel concentrations (>6 nM) with just a small shift in IC₅₀. The siRNA dose-response curves for oncogenic serine/threonine kinase Pim-1 and cell cycle inhibitor p21^{waf1/Cip1} were very similar (Figure 9), which may reflect overlapping or cooperative roles within a signaling pathway; and previous reports have shown that p21^{waf1/Cip1} is a substrate for Pim-1 (Wang et al., Biochim Biophys Acta 1593:45, 2002). Of further interest were data on Pim-1 overexpression in prostate epithelial cells, which lead to mitotic checkpoint interference and genetic instability (Roh et al., Cancer Res 63:8079, 2003). These results are in alignment with Mad2, BubR1 and Mps1 underexpression data showing a comparable phenotype with opposing expression levels (see Figure 1). Another siRNA that conferred sensitivity to docetaxel was Hec1.

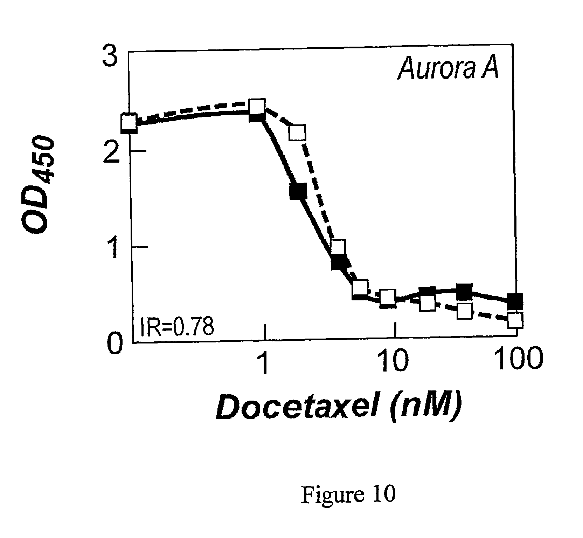

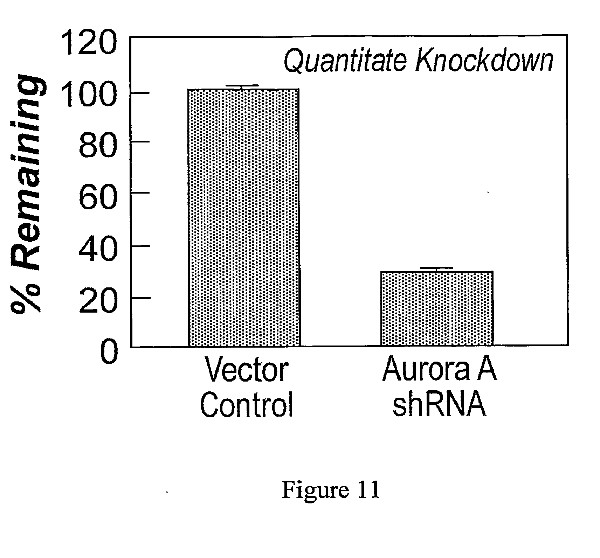

In Table III, TACC3, RELB, Aurora-A, PRKCD (PKC), HSPA1A, and BRAF35 represent siRNAs that increase sensitivity at lower docetaxel concentrations (1-6 nM) with a characteristic shift in IC50. Aurora-A is a serine/threonine kinase that functions at the onset of mitosis and has been implicated in centrosome maturation and spindle assembly (reviewed in Meraldi et al., Curr Opin Genet Dev 14:29, 2004). Of great interest were published data showing that Aurora-A over expression in HeLa cells protected cells from paclitaxel, in alignment with our results where under expression of Aurora-A enhanced docetaxel killing (Anand et al., Cancer Cell 3:51, 2003). Admittedly this was a small decrease in IC50 for Aurora-A representing a 25% decrease in survival; however, the results were precisely duplicated by using retroviral delivery of shRNA generating a stable knock-down of Aurora-A in HCT116 cells (compare Figures 9 to Figures 10 and 11).

### EXAMPLE 6: Pim1 Inhibits Proliferation of HCT116 cells in the Absence of Docetaxel

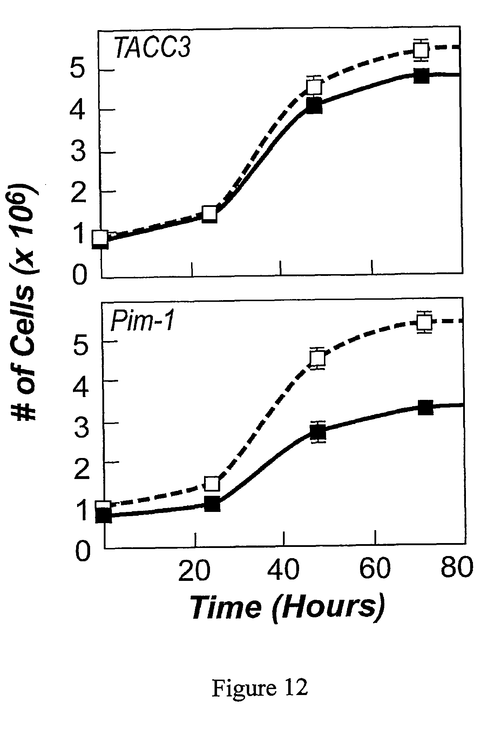

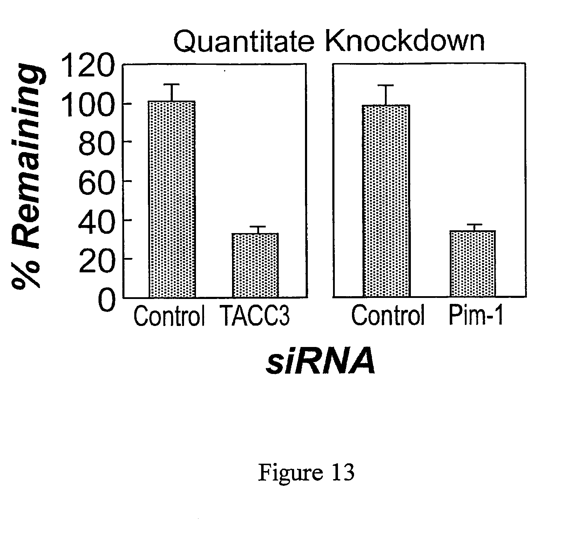

To further evaluate the positives from the screen, it was important to distinguish between siRNAs that affect cell viability in the absence of docetaxel, and those that act cooperatively with docetaxel to enhance killing. To distinguish between the possibilities we studied effects of siRNA transfection on proliferation using a trypan blue exclusion assay with cell counting to determine the number of viable cells in 6-well plates at 24, 48, 72 hours post-transfection. Our results show that Pim-1 siRNAs inhibit growth in HCT116 cells whereas TACC3 siRNAs have no effect on growth (Figure 12; knockdown quantitated in Figure 13), although both siRNAs enhance docetaxel-induced cell death (Figure 9).

### EXAMPLE 7: Validation of Subsets of Resistant and Sensitive Genes Using Live/Dead Assays

To visually compare siRNAs that confer docetaxel sensitivity and resistance, microscopic imaging is used to determine the ratio of live to dead cells where Calcein-AM is used to stain live cells green, and propidium iodide (PI) to stain the nucleus of dead cells red. Post docetaxel (72 hours) addition, siRNA transfected BubR1 cells had the greatest number of viable cells, whereas siRNA transfected Pim-1 and TACC3 had the greatest number of dead cells and the control cells were in between (data not shown). Although the BubR1 siRNA alone showed increased cell death by 72 hours, the cells ultimately become more resistant to docetaxel. Pim-1 siRNA alone induced cell death by 24 hours whereas the TACC3 siRNA did not cause death. These results demonstrate that BubR1 down regulation was associated with increased cell viability and drug resistance, whereas Pim-1 and TACC3 down regulation was associated with increased cell death and drug sensitivity. Both Pim-1 and TACC3 showed more killing in the presence of docetaxel but this assay could not distinguish between an additive effect and cooperative killing.

### EXAMPLE 8: Docetaxel-Induced Caspase-3 Activity was Diminished by BubR1 Down Regulation and Enhanced by Pim-1 Down Regulation

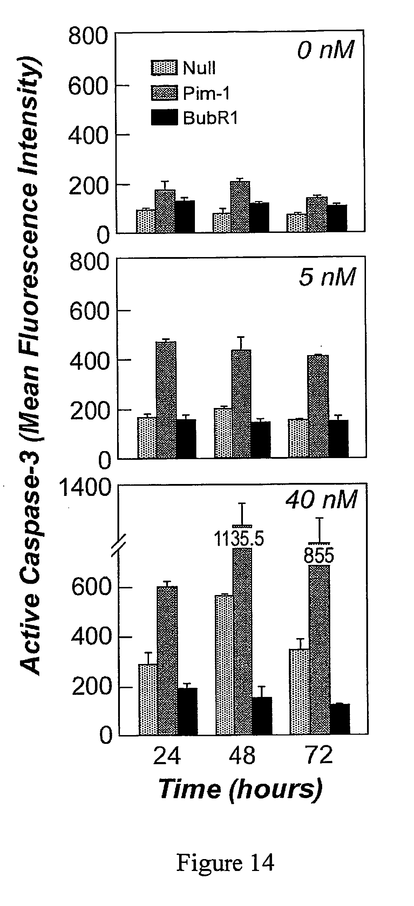

Docetaxel can cause cell death through induction of apoptosis (Kim et al., Int J Mol Med 11:799, 2003). To determine whether Pim-1 and BubR1 siRNAs could modulate a canonical apoptotic pathway in the absence and presence docetaxel, we studied activated caspase-3 levels in HCT116 cells (using a bioplex assay) (Figure 14). After 48 hours of 40 nM docetaxel, caspase-3 activity was induced in control cells and induction was further enhanced by down regulating Pim-1 and diminished by down regulating BubR1 (middle and lower panels). In the absence of docetaxel, down regulation of Pim-1 caused a slight increase in caspase-3 activity at 24 hours, but returned to control level by 48 and 72 hours (upper panel), indicating that other apoptotic machinery were activated to maintain the enhanced killing observed at 72 hours by WST-1 and LIVE/DEAD assays. Down regulation of BubR1 in the absence of docetaxel caused a very slight increase in caspase-3 activity, which is consistent with the observation that BubR1 knockdown initially caused cell death at 72 hours in the LIVE/DEAD assay. These results further implicate Pim-1 and BubR1 as effectors of docetaxel killing through the modulation of caspase-3 activity.

### EXAMPLE 9: Signaling Pathways Involved in Docetaxel-Induced Pim-1-Associated Sensitivity and BubR1-Associated Resistance

The activities attributed to Pim-1 strongly suggest that it plays a central role in blocking signaling events that lead to cell death while promoting those that foster cell survival. In an attempt to define the molecular basis for enhanced cell death associated with a Pim-1 knockdown, we examined the activation state of a key cellular pathway involved in cell survival, AKT. HCT116 were seeded at 4 X 10⁵ cells/well on 6-well plates and the next day transfected with siRNAs at 16 nM using Lipofectamine 2000 (Invitrogen). After 24 hours, cells were untreated or treated with 5 and 40 nM docetaxel. Cells were lysed 24, 48 and 72 hours after docetaxel addition with ice-cold lysis buffer (50 mM HEPES better (PH7.4), 1% NP40, 2.5 mM EDTA, 100 mM sodium fluoride, 10 mM sodium PP_{I}, protease inhibitor cocktail tablet (Roche), 2 mM sodium orthovanadate) and centrifuged at 12,000 g for 10 minutes. Lysates were then quantified for total protein levels using DC protein assay (BioRad). Active caspase-3 levels were determined using an active caspase-3 beadmates kit (Upstate) and the Luminex 100^{™} system with protocols suggested by the manufacture. Total and phosphorylated AKT levels were determined using a total AKT antibody bead kit and a phosphospecific AKT ^{S473} antibody bead kit (Biosource) and the Luminex 100^{™} system with protocols suggested by manufacture.

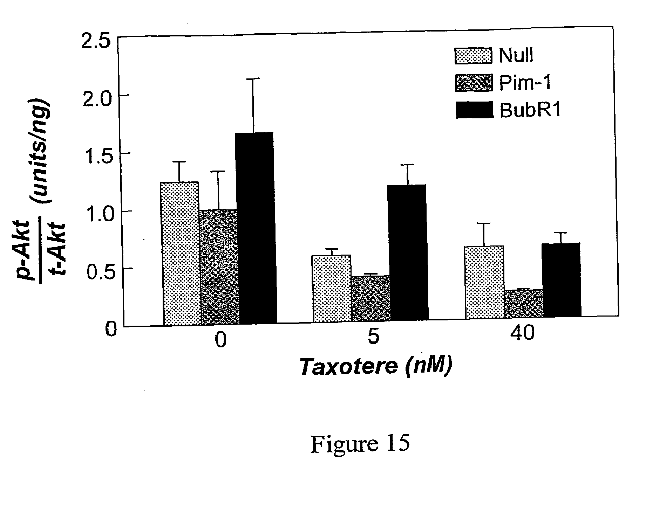

The experiment shown in Figure 15 demonstrates that Pim-1 downregulation decreased the baseline phosphorylation of AKT, as compared to the control and the effect was significantly accentuated with increasing concentrations of docetaxel. By comparison, BubR1 downregulation increased the baseline phosphorylation of AKT and the effect was accentuated at the lower but not higher concentration of docetaxel, indicating alternative-signaling pathways may be activated at higher doses.

Applicants performed Western Blot analysis using antibodies against Pim-1 and BubR1 to measure protein levels at the 48 hour time point. Equal amounts of proteins were loaded into NuPAGE Novex Bis-Tris gels (Invitrogen). Antibodies against Pim-1 (Santa Cruz biotechnology), BubR1 (BD bioscience) and α-tubulin (Sigma) were used as suggested by the manufacture. Secondary antibodies were conjugated to HRP (BioRad) and detected by ECL Western Blot Detection System (Amhersham Biosciences) followed by exposure to Hyperfilm ECL (Amhersham Biosciences). The experiment shown in Figure 16 demonstrated that Pim-1 and BubR1 protein levels were diminished as compared to the control, showing that reduced protein levels corresponded with a functional change (Figure 15). These data suggest that downregulating Pim-1 protein levels can block the phosphorylation and activation of AKT in order to compel the cells towards apoptosis. Pim-1 appears to mediate cell survival by activating pro-survival pathways.

The present invention is not limited in scope by the specific embodiments described herein. Indeed, various modifications of the invention in addition to those described herein will become apparent to those skilled in the art from the foregoing description and the accompanying figures. Such modifications are intended to fall within the scope of the appended claims.

All references cited herein are incorporated by reference in their entireties.
Anand S, Penrhyn-Lowe S, Venkitaraman AR. (2003) AURORA-A amplification overrides the mitotic spindle assembly checkpoint, inducing resistance to Taxol. Cancer Cell. 3:51-62.
Aza-Blanc P, Cooper CL, Wagner K, Batalov S, Deveraux QL, Cooke MP. (2003) Identification of modulators of TRAIL-induced apoptosis via RNAi-based phenotypic screening. Mol Cell. 12:627-37.
Chang JC, Wooten EC, Tsimelzon A, Hilsenbeck SG, Gutierrez MC, Elledge R, Mohsin S, Osborne CK, Chamness GC, Allred DC, O'Connell P. (2003) Gene expression profiling for the prediction of therapeutic response to docetaxel in patients with breast cancer. Lancet. 362:362-9.
Hong, WK (2002) The current status of docetaxel in solid tumors. Oncology 16:9-15.
Jackson AL, Bartz SR, Schelter J, Kobayashi SV, Burchard J, Mao M, Li B, Cavet G, Linsley PS. (2003) Expression profiling reveals off-target gene regulation by RNAi. Nat Biotechnol. 21:635-7.
Katsumata N. (2003) Docetaxel: an alternative taxane in ovarian cancer. Br J Cancer. 89 Suppl 3:S9-S15.
Kim R, Tanabe K, Uchida Y, Emi M, Toge T. (2003) Effect of Bcl-2 antisense oligonucleotide on drug-sensitivity in association with apoptosis in undifferentiated thyroid carcinoma. Int J Mol Med. 11:799-804.
Kolfschoten GM, Hulscher TM, Duyndam MC, Pinedo HM, Boven E. (2002) Variation in the kinetics of caspase-3 activation, Bcl-2 phosphorylation and apoptotic morphology in unselected human ovarian cancer cell lines as a response to docetaxel. Biochem Pharmacol. 63:733-43.
Kung AL, Sherwood SW, Schimke RT. (1990) Cell line-specific differences in the control of cell cycle progression in the absence of mitosis. Proc Natl Acad Sci USA. 87:9553-7.
Lanni JS, Jacks T. (1998) Characterization of the p53-dependent postmitotic checkpoint following spindle disruption. Mol Cell Biol. 18:1055-64.
Lee LF, Li G, Templeton DJ, Ting JP. (1998) Paclitaxel (Taxol)-induced gene expression and cell death are both mediated by the activation of c-Jun NH2-terminal kinase (JNK/SAPK). J Biol Chem. 273:28253-60.
Li Y, Dowbenko D, Lasky LA. (2002) AKT/PKB phosphorylation of p21Cip/WAF1 enhances protein stability of p21Cip/WAF1 and promotes cell survival. J Biol Chem. 277:11352-61.
Lum L, Yao S, Mozer B, Rovescalli A, Von Kessler D, Nirenberg M, Beachy PA. (2003) Identification of Hedgehog pathway components by RNAi in Drosophila cultured cells. Science. 299:2039-45.
Masuda A, Maeno K, Nakagawa T, Saito H, Takahashi T. (2003) Association between mitotic spindle checkpoint impairment and susceptibility to the induction of apoptosis by anti-microtubule agents in human lung cancers. Am J Pathol. 163:1109-16.
McManus MT, Sharp PA. (2002) Gene silencing in mammals by small interfering RNAs. Nat Rev Genet. 3:737-47.
Meraldi P, Honda R, Nigg EA. (2004) Aurora kinases link chromosome segregation and cell division to cancer susceptibility. Curr Opin Genet Dev. 14:29-36.
Michel L, Diaz-Rodriguez E, Narayan G, Hernando E, Murty VV, Benezra R. (2004) Complete loss of the tumor suppressor MAD2 causes premature cyclin B degradation and mitotic failure in human somatic cells. Proc Natl Acad Sci USA. 101:4459-64.
Ojima, I. and Geney, R. 109881 Aventis (2004) Curr Opin Investig Drugs 4:737-40.
Ringel I, Horwitz SB. (1991) Studies with RP 56976 (taxotere): a semisynthetic analogue of taxol. J Natl Cancer Inst. 83:288-91.
Roh M, Gary B, Song C, Said-Al-Naief N, Tousson A, Kraft A, Eltoum IE, Abdulkadir SA. (2003) Overexpression of the oncogenic kinase Pim-1 leads to genomic instability. Cancer Res. 63:8079-84.
Shin HJ, Baek KH, Jeon AH, Park MT, Lee SJ, Kang CM, Lee HS, Yoo SH, Chung DH, Sung YC, McKeon F, Lee CW. (2003) Dual roles of human BubR1, a mitotic checkpoint kinase, in the monitoring of chromosomal instability. Cancer Cell. 4:483-97.
Simmer F, Moorman C, Van Der Linden AM, Kuijk E, Van Den Berghe PV, Kamath R, Fraser AG, Ahringer J, Plasterk RH. (2003) Genome-Wide RNAi of C. elegans Using the Hypersensitive rrf-3 Strain Reveals Novel Gene Functions. PLoS Biol. 1:E12.
Sudo T, Nitta M, Saya H, Ueno NT. (2004) Dependence of paclitaxel sensitivity on a functional spindle assembly checkpoint. Cancer Res. 64:2502-8.
Tanabe K, Kim R, Inoue H, Emi M, Uchida Y, Toge T. (2003) Antisense Bcl-2 and HER-2 oligonucleotide treatment of breast cancer cells enhances their sensitivity to anticancer drugs. Int J Oncol. 22:875-81.
Taylor SS, McKeon F. (1997) Kinetochore localization of murine Bub1 is required for normal mitotic timing and checkpoint response to spindle damage.Cell. 89:727-35.
Wang TH, Wang HS, Soong YK (2000) Paclitaxel-induced cell death: where the cell cycle and apoptosis come together. Cancer. 88:2619-28.
Wang Z, Bhattacharya N, Mixter PF, Wei W, Sedivy J, Magnuson NS. (2002) Phosphorylation of the cell cycle inhibitor p21Cip1/WAF1 by Pim-1 kinase. Biochim Biophys Acta. 1593:45-55.
Zhou J, Yao J, Joshi HC. (2002) Attachment and tension in the spindle assembly checkpoint. J Cell Sci. 115:3547-55.

## Claims

1. A method for predicting or monitoring a cancer patient's response to a molecule of the taxoid family, comprising the steps of:
a) obtaining a test sample from a cancerous area of said patient;
b) obtaining a control sample;
c) measuring the level of STK11; and
d) comparing the measured levels of STK 11 in the test sample and the control sample;
wherein a decrease in the level of STK11 measured in the test sample as compared to the control sample indicates an increased sensitivity to a molecule of the taxoid family.

2. A kit for predicting or monitoring a cancer patient's response to molecule of the taxoid family according to the method of Claim 1 wherein the kit comprises detectable-labeled antibodies, detectable-labeled antibody fragments or detectable-labeled oligonucleotides comprising nucleic acids capable of hybridizing under stringent conditions to STK 11.

3. A kit for predicting or monitoring a cancer patient's response to molecule of the taxoid family according to the method of Claim 1 wherein the kit comprises the means of:
a) obtaining total RNA from a bodily sample;
b) reverse transcribing the total RNA to obtain cDNA; and
c) subjecting the cDNA to a polymerase chain reaction using a set of primers;
wherein one or both primers are detectably-labeled and both primers are derived from a nucleotide sequence of STK11.

4. A method for predicting or monitoring a cancer patient's response to a molecule of the taxoid family, comprising the steps of:
a) obtaining a test sample from a cancerous area of said patient;
b) measuring the level of STK11;
c) measuring the level of one or more reference genetic markers;
d) comparing the measured levels of STK 11 and said one or more reference genetic markers in the test sample;
wherein a decrease in the level of STK 11 as compared to the level of said one or more reference genetic markers indicates an increased susceptibility to a molecule of the taxoid family.

5. The method of Claim 1 or 4 wherein said molecule of the taxoid family is selected from the group consisting of paclitaxel, docetaxel XRP9881 and XRP6258.

6. The method of Claim 1 or 4 wherein said level of markers is measured by mRNA, DNA or protein.

7. The method of Claim 6 wherein said mRNA is measured by using a technique selected from the group consisting of in situ hybridization, reverse-transcriptase polymerase chain reaction, nucleic acid hybridization, electrophoresis, Northern blotting and mass spectrometry.

8. The method of Claim 6 wherein said DNA is measured by using a technique selected from the group consisting of quantitative polymerase chain reaction, genomic DNA-chips, in situ hybridization, electrophoresis, Southern blotting and mass spectrometry.

9. The method of Claim 6 wherein said protein is measured by using a technique selected from the group consisting of immunoassay, Western blots, ELISA, and mass spectrometry.

10. A kit for predicting or monitoring a cancer patient's response to a molecule of the taxoid family according to the method of Claim 4 wherein the kit comprises detectable-labeled antibodies, detectable-labeled antibody fragments or detectable-labeled oligonucleotides comprising nucleic acids capable of hybridizing under stringent conditions STK11 and said one or more genetic markers.

11. A kit for predicting or monitoring a cancer patient's response to a molecule of the taxoid family according to the method of claim 4 wherein the kit comprises the means of:
a) obtaining total RNA from a bodily sample;
b) reverse transcribing the total RNA to obtain cDNA; and
c) subjecting the cDNA to a polymerase chain reaction using a set of primers wherein one or both primers are detectable-labeled and both primers are derived from a nucleotide sequence of STK11.

12. The kit of any one of claims 2, 3, 10 and 11, wherein the detectable label comprises an enzyme, a radioactive isotope, or a chemical which fluoresces, a chemiluminescent molecule, radiopaque substances, liposomes, and haptenic molecules.

13. The method of any one of claims 1 and 4 to 9, wherein the level of one or more additional genetic marker is measured, said additional genetic marker being selected from the group consisting of:
P21(Waf1), Homo sapiens cyclin-dependent kinase inhibitor 1A (p21, Cip1) (CDKN1A), transcript variant 1, mRNA (GenBank accession number: NM_000389);
Pim-1, Homo sapiens pim-1 oncogene (PIM1), mRNA (GenBank accession number: NM_002648);
GBP-1, Homo sapiens guanylate binding protein 1, interferon-inducible, 67kDa (GBP1), mRNA (GenBank accession number: NM_002053);
RXRA Homo sapiens retinoid X receptor, alpha (RXRA), mRNA (GenBank accession number: NM_002957);
SPF45, Homo sapiens RNA binding motif protein 17 (RBM17), mRNA (GenBank accession number: NM_032905);
Hec1, Homo sapiens kinetochore associated 2 (KNTC2), mRNA (GenBank accession number: NM_006101);
Raf1, Human mRNA for raf oncogene (GenBank accession number: X03484);
Aurora A, Homo sapiens aurora-related kinase 1 (ARK1) mRNA, complete cds (GenBank accession number: AF008551);
TACC3, Homo sapiens transforming, acidic coiled-coil containing protein 3 (TACC3), mRNA (GenBank accession number: NM_006342);
RelB, Homo sapiens v-rel reticuloendotheliosis viral oncogene homolog B5 nuclear factor of kappa light polypeptide gene enhancer in B-cells 3 (avian) (RELB), mRNA (GenBank accession number: NM_006509);
PRKCD5 Homo sapiens protein kinase C, delta (PRKCD), transcript variant I5 mRNA (GenBank accession number: NM_006254);
BRAF35, Homo sapiens high-mobility group 20B (HMG20B), mRNA (GenBank accession number: NM_006339);
HSPA1L, Homo sapiens heat shock 70 kDa protein 1A (HSPA1A)5 mRNA (GenBank accession number: NM_005345);
MKK3, Homo sapiens MAP kinase kinase 3 (MKK3) mRNA, complete cds (GenBank accession number: L36719).

14. The kit of one of claims 2, 3, and 10 to 12, wherein the kit comprises in addition detectable-labeled antibodies, detectable-labeled antibody fragments or detectable-labeled oligonucleotides comprising nucleic acids capable of hybridizing under stringent conditions to one or more genetic markers selected from the group consisting of:
P21(Waf1), Homo sapiens cyclin-dependent kinase inhibitor 1A (p21, Cip1) (CDKN1A), transcript variant 1, mRNA (GenBank accession number: NM_000389);
Pim-1, Homo sapiens pim-1 oncogene (PIM1), mRNA (GenBank accession number: NM_002648);
GBP-1, Homo sapiens guanylate binding protein 1, interferon-inducible, 67kDa (GBP1), mRNA (GenBank accession number: NM_002053);
RXRA Homo sapiens retinoid X receptor, alpha (RXRA), mRNA (GenBank accession number: NM_002957);
SPF45, Homo sapiens RNA binding motif protein 17 (RBM17), mRNA (GenBank accession number: NM_032905);
Hec1, Homo sapiens kinetochore associated 2 (KNTC2), mRNA (GenBank accession number: NM_006101);
Raf1, Human mRNA for raf oncogene (GenBank accession number: X03484);
Aurora A, Homo sapiens aurora-related kinase 1 (ARK1) mRNA, complete cds (GenBank accession number: AF008551);
TACC3, Homo sapiens transforming, acidic coiled-coil containing protein 3 (TACC3), mRNA (GenBank accession number: NM_006342);
RelB, Homo sapiens v-rel reticuloendotheliosis viral oncogene homolog B5 nuclear factor of kappa light polypeptide gene enhancer in B-cells 3 (avian) (RELB), mRNA (GenBank accession number: NM_006509);
PRKCD5 Homo sapiens protein kinase C, delta (PRKCD), transcript variant I5 mRNA (GenBank accession number: NM_006254);
BRAF35, Homo sapiens high-mobility group 20B (HMG20B), mRNA (GenBank accession number: NM_006339);
HSPA1L, Homo sapiens heat shock 70 kDa protein 1A (HSPA1A)5 mRNA (GenBank accession number: NM_005345);
MKK3, Homo sapiens MAP kinase kinase 3 (MKK3) mRNA, complete cds (GenBank accession number: L36719).


<110>  GRUENBERG, Dorre
       HUANG, Xi
       NATESAN, Sridaran
       AUGUST, Paul

<120>  METHOD FOR MEASURING RESISTANCE OR SENSITIVITY TO DOCETAXEL

<130>  USAV2004/0088 US PCT

<160>  116

<170>  PatentIn version 3.3

<210>  1
<211>  19
<212>  RNA
<213>  Artificial

<220>
<223>  siRNA

<400>  1
gcgauggaac uucgacuuu                                                    19

<210>  2
<211>  19
<212>  RNA
<213>  Artificial

<220>
<223>  siRNA

<400>  2
uggaacuucg acuuuguca                                                    19

<210>  3
<211>  19
<212>  RNA
<213>  Artificial

<220>
<223>  siRNA

<400>  3
agaccaugug gaccuguca                                                    19

<210>  4
<211>  19
<212>  RNA
<213>  Artificial

<220>
<223>  siRNA

<400>  4
ccuucgaaga aauccagaa                                                    19

<210>  5
<211>  19
<212>  RNA

<213>  Artificial

<220>
<223>  siRNA

<400>  5
uggugugugg agauauucc                                                    19


<210>  6
<211>  19
<212>  RNA
<213>  Artificial

<220>
<223>  siRNA

<400>  6
uauuccuuuc gagcaugac                                                    19


<210>  7
<211>  19
<212>  RNA
<213>  Artificial

<220>
<223>  siRNA

<400>  7
cgaaaggcau guaccauaa                                                    19


<210>  8
<211>  19
<212>  RNA
<213>  Artificial

<220>
<223>  siRNA

<400>  8
gauacaggcu gaagagauu                                                    19


<210>  9
<211>  19
<212>  RNA
<213>  Artificial

<220>
<223>  siRNA

<400>  9
gaacaggagc aacuacuaa                                                    19


<210>  10
<211>  19
<212>  RNA
<213>  Artificial

<220>
<223>  siRNA

<400>  10
gcaaggaccu gaccuacac                                                    19

<210> 11
<211> 19
<212> RNA
<213> Artificial

<220>
<223> siRNA

<400> 11
gcaaggaccg gaacgagaa 19


<210> 12
<211> 19
<212> RNA
<213> Artificial

<220>
<223> siRNA

<400> 12
gacccuguca ccaacauuu 19


<210> 13
<211> 19
<212> RNA
<213> Artificial

<220>
<223> siRNA

<400> 13
caaauccgcu gacugaaau 19


<210> 14
<211> 19
<212> RNA
<213> Artificial

<220>
<223> siRNA

<400> 14
gaacaagaca gaccgagau 19


<210> 15
<211> 19
<212> RNA
<213> Artificial

<220>
<223> siRNA

<400> 15
gacccuaugu uuccuaaug 19


<210> 16
<211> 19
<212> RNA
<213> Artificial

<220>
<223> siRNA

<400>  16
guguauucga caacucugu                                                    19


<210>  17
<211>  19
<212>  RNA
<213>  Artificial

<220>
<223>  siRNA

<400>  17
gauugcaaga uuggaacaa                                                    19


<210>  18
<211>  19
<212>  RNA
<213>  Artificial

<220>
<223>  siRNA

<400>  18
guacucaguu gcagacauu                                                    19


<210>  19
<211>  19
<212>  RNA
<213>  Artificial

<220>
<223>  siRNA

<400>  19
guaucacaaa uuggcuaga                                                    19


<210>  20
<211>  19
<212>  RNA
<213>  Artificial

<220>
<223>  siRNA

<400>  20
gcacggagau guugcagua                                                    19


<210>  21
<211>  19
<212>  RNA
<213>  Artificial

<220>
<223>  siRNA

<400>  21
gcaaagaaca ucauccaua                                                    19


<210>  22
<211>  19
<212>  RNA

<213>  Artificial

<220>
<223>  siRNA

<400>  22
gacaugaaau ccaacaaua                                                    19


<210>  23
<211>  19
<212>  RNA
<213>  Artificial

<220>
<223>  siRNA

<400>  23
ggaaugagcu ugcaugacu                                                    19


<210>  24
<211>  19
<212>  RNA
<213>  Artificial

<220>
<223>  siRNA

<400>  24
gagaacugcu acuuauaua                                                    19


<210>  25
<211>  19
<212>  RNA
<213>  Artificial

<220>
<223>  siRNA

<400>  25
gaauaugcac cacuuggaa                                                    19


<210>  26
<211>  19
<212>  RNA
<213>  Artificial

<220>
<223>  siRNA

<400>  26
gaaggucgga ugcaugaug                                                    19


<210>  27
<211>  19
<212>  RNA
<213>  Artificial

<220>
<223>  siRNA

<400>  27
guaaaggaaa guuugguaa                                                    19

<210>  28
<211>  19
<212>  RNA
<213>  Artificial

<220>
<223>  siRNA

<400>  28
gcaccucgcu ucccacaag                                                    19

<210>  29
<211>  19
<212>  RNA
<213>  Artificial

<220>
<223>  siRNA

<400>  29
gagcggaccu guaaaacua                                                    19

<210>  30
<211>  19
<212>  RNA
<213>  Artificial

<220>
<223>  siRNA

<400>  30
gaacgaagag ucacugaag                                                    19

<210>  31
<211>  19
<212>  RNA
<213>  Artificial

<220>
<223>  siRNA

<400>  31
gcagaaagag gacauauca                                                    19

<210>  32
<211>  19
<212>  RNA
<213>  Artificial

<220>
<223>  siRNA

<400>  32
gcagcgagcc auugccuuu                                                    19

<210>  33
<211>  19
<212>  RNA
<213>  Artificial

<220>
<223>  siRNA

<400> 33
gcccgucuau gacaagaaa 19

<210> 34
<211> 19
<212> RNA
<213> Artificial

<220>
<223> siRNA

<400> 34
ggacguggau ugcaaacaa 19

<210> 35
<211> 19
<212> RNA
<213> Artificial

<220>
<223> siRNA

<400> 35
gcaugaaugu gcaccauaa 19

<210> 36
<211> 19
<212> RNA
<213> Artificial

<220>
<223> siRNA

<400> 36
gaaagaacgc uucaacauc 19

<210> 37
<211> 19
<212> RNA
<213> Artificial

<220>
<223> siRNA

<400> 37
gggcguacca gcagucuga 19

<210> 38
<211> 19
<212> RNA
<213> Artificial

<220>
<223> siRNA

<400> 38
gcucugggcu caugaacac 19

<210> 39
<211> 19
<212> RNA

<213>  Artificial

<220>
<223>  siRNA

<400>  39
gucugaagcc uauaagaug                                                    19


<210>  40
<211>  19
<212>  RNA
<213>  Artificial

<220>
<223>  siRNA

<400>  40
gagaucgacu cccuguuug                                                    19


<210>  41
<211>  19
<212>  RNA
<213>  Artificial

<220>
<223>  siRNA

<400>  41
uggaggaguu caagagaaa                                                    19


<210>  42
<211>  19
<212>  RNA
<213>  Artificial

<220>
<223>  siRNA

<400>  42
gaucaacgac ggagacaag                                                    19


<210>  43
<211>  19
<212>  RNA
<213>  Artificial

<220>
<223>  siRNA

<400>  43
gaagaaggaa auucaacua                                                    19


<210>  44
<211>  19
<212>  RNA
<213>  Artificial

<220>
<223>  siRNA

<400>  44
gagaagcguu ucccagugu                                                    19

<210> 45
<211> 19
<212> RNA
<213> Artificial

<220>
<223> siRNA

<400> 45
gaaacauccu ccggcugaa 19

<210> 46
<211> 19
<212> RNA
<213> Artificial

<220>
<223> siRNA

<400> 46
gguggaggcu gaugacuug 19

<210> 47
<211> 19
<212> RNA
<213> Artificial

<220>
<223> siRNA

<400> 47
ggagauugcu gugucuauc 19

<210> 48
<211> 19
<212> RNA
<213> Artificial

<220>
<223> siRNA

<400> 48
ggagggccau gugaagaug 19

<210> 49
<211> 19
<212> RNA
<213> Artificial

<220>
<223> siRNA

<400> 49
gaugugaagc ccuccaaug 19

<210> 50
<211> 19
<212> RNA
<213> Artificial

<220>
<223> siRNA

<400>  50
gcaaugagcc uuuggauau                                                    19

<210>  51
<211>  19
<212>  RNA
<213>  Artificial

<220>
<223>  siRNA

<400>  51
ggaacaaccu cauucuaaa                                                    19

<210>  52
<211>  19
<212>  RNA
<213>  Artificial

<220>
<223>  siRNA

<400>  52
ggaagaagau cuagaugua                                                    19

<210>  53
<211>  19
<212>  RNA
<213>  Artificial

<220>
<223>  siRNA

<400>  53
gaaaucgugg ccgaguucu                                                    19

<210>  54
<211>  19
<212>  RNA
<213>  Artificial

<220>
<223>  siRNA

<400>  54
guggcauaua uccaucuga                                                    19

<210>  55
<211>  19
<212>  RNA
<213>  Artificial

<220>
<223>  siRNA

<400>  55
gccgaguucu ucucauucg                                                    19

<210>  56
<211>  19
<212>  RNA

<213>  Artificial

<220>
<223>  siRNA

<400>  56
ggaacaacug aaagauugg                                                    19


<210>  57
<211>  19
<212>  RNA
<213>  Artificial

<220>
<223>  siRNA

<400>  57
gucguuacag ucaagcaau                                                    19


<210>  58
<211>  19
<212>  RNA
<213>  Artificial

<220>
<223>  siRNA

<400>  58
gcacgugacu acuuucaaa                                                    19


<210>  59
<211>  19
<212>  RNA
<213>  Artificial

<220>
<223>  siRNA

<400>  59
gaugaacuaa gcuugaaua                                                    19


<210>  60
<211>  19
<212>  RNA
<213>  Artificial

<220>
<223>  siRNA

<400>  60
gagcaguacc acuagaaau                                                    19


<210>  61
<211>  19
<212>  RNA
<213>  Artificial

<220>
<223>  siRNA

<400>  61
gagcaguacc acuagaaau                                                    19

<210>  62
<211>  19
<212>  RNA
<213>  Artificial

<220>
<223>  siRNA

<400>  62
gcaccgagac uauuucuug                                                    19


<210>  63
<211>  19
<212>  RNA
<213>  Artificial

<220>
<223>  siRNA

<400>  63
ggaugaagau gagcuguaa                                                    19


<210>  64
<211>  19
<212>  RNA
<213>  Artificial

<220>
<223>  siRNA

<400>  64
gcauguggcu cagaucuug                                                    19


<210>  65
<211>  18
<212>  RNA
<213>  Artificial

<220>
<223>  siRNA

<400>  65
guacaacagu gaccucca                                                     18


<210>  66
<211>  19
<212>  RNA
<213>  Artificial

<220>
<223>  siRNA

<400>  66
gaugcuggau gugugaaua                                                    19


<210>  67
<211>  19
<212>  RNA
<213>  Artificial

<220>
<223>  siRNA

<400> 67
gcuugugaua aagagucaa 19

<210> 68
<211> 19
<212> RNA
<213> Artificial

<220>
<223> siRNA

<400> 68
gauccaccag augcuauug 19

<210> 69
<211> 19
<212> RNA
<213> Artificial

<220>
<223> siRNA

<400> 69
gcugucagau aguugauuu 19

<210> 70
<211> 19
<212> RNA
<213> Artificial

<220>
<223> siRNA

<400> 70
gccuacagcu ucuauaguc 19

<210> 71
<211> 19
<212> RNA
<213> Artificial

<220>
<223> siRNA

<400> 71
guggacaguu guaaaucua 19

<210> 72
<211> 19
<212> RNA
<213> Artificial

<220>
<223> siRNA

<400> 72
gaagaucguu uccuauaca 19

<210> 73
<211> 19
<212> RNA

<213>  Artificial

<220>
<223>  siRNA

<400>  73
gcacgaaagc aagagauua                                                    19


<210>  74
<211>  19
<212>  RNA
<213>  Artificial

<220>
<223>  siRNA

<400>  74
gaagaaacca gauggagua                                                    19


<210>  75
<211>  19
<212>  RNA
<213>  Artificial

<220>
<223>  siRNA

<400>  75
gcuagaaucu gccgucuuu                                                    19


<210>  76
<211>  19
<212>  RNA
<213>  Artificial

<220>
<223>  siRNA

<400>  76
gaucaaggcu ggagcuuau                                                    19


<210>  77
<211>  19
<212>  RNA
<213>  Artificial

<220>
<223>  siRNA

<400>  77
gaacaugucg aucaagacu                                                    19


<210>  78
<211>  19
<212>  RNA
<213>  Artificial

<220>
<223>  siRNA

<400>  78
guuuguaaca gauaucgau                                                    19

<210> 79
<211> 19
<212> RNA
<213> Artificial

<220>
<223> siRNA

<400> 79
gaguagugca ucgcgaucu 19


<210> 80
<211> 19
<212> RNA
<213> Artificial

<220>
<223> siRNA

<400> 80
guaacagaua ucgaugaac 19


<210> 81
<211> 19
<212> RNA
<213> Artificial

<220>
<223> siRNA

<400> 81
gaacgaagaa ugugaucag 19


<210> 82
<211> 19
<212> RNA
<213> Artificial

<220>
<223> siRNA

<400> 82
guggauuauc acagaucua 19


<210> 83
<211> 19
<212> RNA
<213> Artificial

<220>
<223> siRNA

<400> 83
gguacaaggc agagcuuaa 19


<210> 84
<211> 19
<212> RNA
<213> Artificial

<220>
<223> siRNA

<400>  84
gugccacagc agccgacau                                                    19


<210>  85
<211>  21
<212>  RNA
<213>  Artificial

<220>
<223>  siRNA

<400>  85
gucgaccauu auguaccgga u                                                 21


<210>  86
<211>  21
<212>  RNA
<213>  Artificial

<220>
<223>  siRNA

<400>  86
ggauucguaa cgugauuagc g                                                 21


<210>  87
<211>  19
<212>  RNA
<213>  Artificial

<220>
<223>  siRNA

<400>  87
gaaacagaag aaccugauu                                                    19


<210>  88
<211>  19
<212>  RNA
<213>  Artificial

<220>
<223>  siRNA

<400>  88
gaaaggacau gugaacuua                                                    19


<210>  89
<211>  19
<212>  RNA
<213>  Artificial

<220>
<223>  siRNA

<400>  89
guacaucuca gaaucuuga                                                    19
<210>  90
<211>  2140
<212>  DNA
<213>  human

<400> 90
agctgaggtg tgagcagctg ccgaagtcag ttccttgtgg agccggagct gggcgcggat 60
tcgccgaggc accgaggcac tcagaggagg cgccatgtca gaaccggctg gggatgtccg 120
tcagaaccca tgcggcagca aggcctgccg ccgcctcttc ggcccagtgg acagcgagca 180
gctgagccgc gactgtgatg cgctaatggc gggctgcatc caggaggccc gtgagcgatg 240
gaacttcgac tttgtcaccg agacaccact ggagggtgac ttcgcctggg agcgtgtgcg 300
gggccttggc ctgcccaagc tctaccttcc cacggggccc cggcgaggcc gggatgagtt 360
gggaggaggc aggcggcctg gcacctcacc tgctctgctg caggggacag cagaggaaga 420
ccatgtggac ctgtcactgt cttgtaccct tgtgcctcgc tcaggggagc aggctgaagg 480
gtccccaggt ggacctggag actctcaggg tcgaaaacgg cggcagacca gcatgacaga 540
tttctaccac tccaaacgcc ggctgatctt ctccaagagg aagccctaat ccgcccacag 600
gaagcctgca gtcctggaag cgcgagggcc tcaaaggccc gctctacatc ttctgcctta 660
gtctcagttt gtgtgtctta attattattt gtgttttaat ttaaacacct cctcatgtac 720
ataccctggc cgccccctgc cccccagcct ctggcattag aattatttaa acaaaaacta 780
ggcggttgaa tgagaggttc ctaagagtgc tgggcatttt tattttatga aatactattt 840
aaagcctcct catcccgtgt tctccttttc ctctctcccg gaggttgggt gggccggctt 900
catgccagct acttcctcct ccccacttgt ccgctgggtg gtaccctctg gaggggtgtg 960
gctccttccc atcgctgtca caggcggtta tgaaattcac cccctttcct ggacactcag 1020
acctgaattc tttttcattt gagaagtaaa cagatggcac tttgaagggg cctcaccgag 1080
tgggggcatc atcaaaaact ttggagtccc ctcacctcct ctaaggttgg gcagggtgac 1140
cctgaagtga gcacagccta gggctgagct ggggacctgg taccctcctg gctcttgata 1200
cccccctctg tcttgtgaag gcagggggaa ggtggggtac tggagcagac caccccgcct 1260
gccctcatgg cccctctgac ctgcactggg gagcccgtct cagtgttgag ccttttccct 1320
ctttggctcc cctgtacctt ttgaggagcc ccagcttacc cttcttctcc agctgggctc 1380
tgcaattccc ctctgctgct gtccctcccc cttgtctttc ccttcagtac cctctcatgc 1440
tccaggtggc tctgaggtgc ctgtcccacc cccaccccca gctcaatgga ctggaagggg 1500
aagggacaca caagaagaag ggcaccctag ttctacctca ggcagctcaa gcagcgaccg 1560
ccccctcctc tagctgtggg ggtgagggtc ccatgtggtg gcacaggccc ccttgagtgg 1620
ggttatctct gtgttagggg tatatgatgg gggagtagat ctttctagga gggagacact 1680
ggcccctcaa atcgtccagc gaccttcctc atccacccca tccctcccca gttcattgca 1740
ctttgattag cagcggaaca aggagtcaga cattttaaga tggtggcagt agaggctatg 1800
gacagggcat gccacgtggg ctcatatggg gctgggagta gttgtctttc ctggcactaa 1860
cgttgagccc ctggaggcac tgaagtgctt agtgtacttg gagtattggg gtctgacccc 1920
aaacaccttc cagctcctgt aacatactgg cctggactgt ttctctcggg ctccccatgt 1980
gtcctggttc ccgtttctcc acctagactg taaacctctc gagggcaggg accacaccct 2040

gtactgttct gtgtctttca cagctcctcc cacaatgctg aatatacagc aggtgctcaa 2100
taaatgattc ttagtgactt taaaaaaaaa aaaaaaaaaa 2140

<210> 91
<211> 5449
<212> DNA
<213> human

<400> 91
gcgccggggg ccgccgcgcc cgccgcccgc tgcctgcgcc gccggccggg catgagttag 60
tcgcagacat ggacaccaaa catttcctgc cgctcgattt ctccacccag gtgaactcct 120
ccctcacctc cccgacgggg cgaggctcca tggctgcccc ctcgctgcac ccgtccctgg 180
ggcctggcat cggctccccg ggacagctgc attctcccat cagcaccctg agctccccca 240
tcaacggcat gggcccgcct ttctcggtca tcagctcccc catgggcccc cactccatgt 300
cggtgcccac cacacccacc ctgggcttca gcactggcag cccccagctc agctcaccta 360
tgaaccccgt cagcagcagc gaggacatca agccccccct gggcctcaat ggcgtcctca 420
aggtccccgc ccacccctca ggaaacatgg cttccttcac caagcacatc tgcgccatct 480
gcggggaccg ctcctcaggc aagcactatg gagtgtacag ctgcgagggg tgcaagggct 540
tcttcaagcg gacggtgcgc aaggacctga cctacacctg ccgcgacaac aaggactgcc 600
tgattgacaa gcggcagcgg aaccggtgcc agtactgccg ctaccagaag tgcctggcca 660
tgggcatgaa gcgggaagcc gtgcaggagg agcggcagcg tggcaaggac cggaacgaga 720
atgaggtgga gtcgaccagc agcgccaacg aggacatgcc ggtggagagg atcctggagg 780
ctgagctggc cgtggagccc aagaccgaga cctacgtgga ggcaaacatg gggctgaacc 840
ccagctcgcc gaacgaccct gtcaccaaca tttgccaagc agccgacaaa cagcttttca 900
ccctggtgga gtgggccaag cggatcccac acttctcaga gctgcccctg gacgaccagg 960
tcatcctgct gcgggcaggc tggaatgagc tgctcatcgc ctccttctcc caccgctcca 1020
tcgccgtgaa ggacgggatc ctcctggcca ccgggctgca cgtccaccgg aacagcgccc 1080
acagcgcagg ggtgggcgcc atctttgaca gggtgctgac ggagcttgtg tccaagatgc 1140
gggacatgca gatggacaag acggagctgg gctgcctgcg cgccatcgtc ctctttaacc 1200
ctgactccaa ggggctctcg aacccggccg aggtggaggc gctgagggag aaggtctatg 1260
cgtccttgga ggcctactgc aagcacaagt acccagagca gccgggaagg ttcgctaagc 1320
tcttgctccg cctgccggct ctgcgctcca tcgggctcaa atgcctggaa catctcttct 1380
tcttcaagct catcggggac acacccattg acaccttcct tatggagatg ctggaggcgc 1440
cgcaccaaat gacttaggcc tgcgggccca tcctttgtgc ccacccgttc tggccaccct 1500
gcctggacgc cagctgttct tctcagcctg agccctgtcc ctgcccttct ctgcctggcc 1560
tgtttggact ttggggcaca gcctgtcact gctctgccta agagatgtgt tgtcaccctc 1620
cttatttctg ttactacttg tctgtggccc agggcagtgg ctttcctgag gcagcagcct 1680

tcgtggcaag aactagcgtg agcccagcca ggcgcctccc caccgggctc tcaggacacc 1740
ctgccacacc ccacggggct tgggcgacta cagggtcttc gggccccagc cctggagctg 1800
caggagttgg gaacggggct tttgtttccg ttgctgttta tcgatgctgg ttttcagaat 1860
tcctgtgtgg ccctcctgtc tggagtgaca tcttcatctg ctctgaatac tggtgcccag 1920
ccagcccgtg acagcttccc cctaatcagg aggggacagc tgggggcgca agctggtgtg 1980
tcatcagcaa agacctcagc cgcctcgggg atgagagggg actcgtgggg caagcaagct 2040
gccctgtgct ctgagtgagg gggaaggtag cccctttttc caaagataac tcacagtttt 2100
gccctcgagc caatgagaac atgagctgcc ctctgtgcaa ggtttcgggg ccacctccag 2160
gctgcagggg cgggtcactc acccccctgt tttctctctg ccttggtgtt ctggtttcag 2220
actcccgact ccccgttcag accagagtgc cccggcccct ccccagcctg agtcttctcc 2280
ttgctctgcg gggtgggctg aggcttgtcc ttgtttcctg cagggctggc cctggctcgg 2340
gcagggtggg gcatcaccac ctcactggcc ttgctggagg cacagggctc tgcggacctg 2400
cagccatctg tgaggcccgc ggggatggga ggggaggagg gtggcctgtt ggtttccctc 2460
agagggggca ggtggcctgg agagagaggg gctcaggaac tgggagcctc gtgggtgggg 2520
cagatgctcc gcggcctgga gtggctctgc cggggcattg gtgggacccc tgctcaggcc 2580
ttctctctgg ctgccagttg tgtctaaaag actcttggaa tctgagaacc cggagtcgca 2640
gcgccctcgg gcctgggcca cacgcaggcc ctggtgggac cacccagcct ggtattgtcc 2700
acggacagcg ttgttcaccc agagccttac ttgggagcct cactgaacgc ctgctctggt 2760
tgaaggtggg gtgggggcgg ggcttggggc ctccctggct cagcccagtg cggcctggcg 2820
ctcctcccgc aggctctgcc cccgggctcc ggtggtgcgg ggccctctca ggttgaactc 2880
gcctcttttg cactggaagg ccctcccttt ggcctgagta cttttcccgt tcacgcctca 2940
gtcccgtgga cccagccttt gtcagtggca ggtgcctgaa cagagggtgg atggggggga 3000
taccggaggg ggtcttgtct tcccagccgc agtctaggaa tgatgcgggg gggtggacgc 3060
cttctccata gtctttcccc acctggagca ggggcttcct cagtggtgag gggagctgcc 3120
tacaggttgg accgggaggc agtggcttgg agaggcagct ttccagcctt ggtggggaag 3180
aaagtgtcca ttctttgcct tcctggagct cccagccaga gctgagctta ggcacccgag 3240
tggagcctgc agctgagtct gtgcccgaga caggctgtca gagattccag aagcctctcc 3300
tccccgccgc cctccacccc tgcctttcag cgttgtggat ccctagaggt ggccccctgc 3360
ccgatccacc gtcctgaggc agagtgttga gcctcatacc tgtaccaggt ccccggccag 3420
ctgggcccct cccaggcact gccaggaagc cccagctgcc cctggcgggt gtggtggaaa 3480
tggcaggagg gtgcaggtac tcttggggcc ccagcggtgg gagtgcaaaa gacccaacgc 3540
caacacctgg tgccttttgc agccagcgcc cacccatccg tgcccggacc cttgggaatg 3600
cccgcggctc cagaggaaaa agcccaggga cggggcctcc gttgcggggg gtcggctgct 3660
tcttgggaac tttgtcgttt ccggcgctgg ctggctggct ggctgtaaag cactgaagcc 3720

ccccggccgc caacccctga aagcagaacc tggcctccct ggccacagca gccttaccca 3780
ccgctctacg tgtcccgggc acttcccgca gccttcccgt ccctttctca tcggccttgt 3840
agttgtacag tgctgttggt ttgaaaaggt gatgtgtggg gagtgcggct catcactgag 3900
tagagaggta gaatttctat ttaaccagac ctgtagtagt attaccaatc cagttcaatt 3960
aaggtgattt tttgtaatta ttattatttt ggtgggacaa tctttaattt tctaaagata 4020
gcactaacat cagctcatta gccacctgtg cctgtccccg ccttggcccg gctggatgaa 4080
gcggcttccc cgcagggccc ccacttccca gtggctgctt cctggggacc cagggcaccc 4140
cggcaccttc aggcacgctc ctcagctggt cacctcccgg ctttgccgtt cagatggggc 4200
tcctgaggct caggagtgaa gatgccacag agccgggctc ccctaggctg cgtcgggcat 4260
gcttggaagc tggcctgcca ggaccttcca ccctggggcc tgtgtcagcc gccggccctc 4320
cgcaccctgg aagcacacgg cctctgggaa ggacagccct gaccttcggt tttccgagca 4380
cggtgtttcc caagaattct gggctggcgg cctggtggca gtgctggaga tgaccccgag 4440
cccctccccg tggggcaccc aggagggccc tgccggaatg tgcagcctgt gggtagtcgg 4500
ctggtgtccc tgtcgtggag ctggggtgcg tgatctggtg ctcgtccacg caggtgtgtg 4560
gtgtaaacat gtatgtgctg tacagagaga cgcgtgtgga gagagccgca caccagcgcc 4620
acccaggaaa ggcggagcgg ttaccagtgt tttgtgttta tttttaatca agacgtttcc 4680
cctgttttcc tataaatttg cttcgtgtaa gcaagtacat aaggaccctc ctttggtgaa 4740
atccgggttc gaatgaatat ctcaaggcag gagatgcatc tattttaaga tgctttggag 4800
cagacagctt tagccgttcc caatccttag caatgcctta gctgggacgc atagctaata 4860
ctttagagag gatgacagat ccataaagag agtaaagata agagaaaatg tctaaagcat 4920
ctggaaaggt aaaaaaaaaa aatctatttt tgtacaaatg taattttatc cctcatgtat 4980
acttggatat ggcgggggga gggctgggac tgtttcgttt ctgcttctag agattgaggt 5040
gaaagcttcg tccgagaaac gccaggacag acgatggcag aggagagggc tcctgtgacg 5100
gcggcgaggc ttgggaggaa accgccgcaa tgggggtgtc ttccctcggg gcaggagggt 5160
gggcctgagg ctttcaaggg ttttcttccc tttcgagtaa tttttaaagc cttgctctgt 5220
tgtgtcctgt tgccggctct ggccttcctg tgactgactg tgaagtggct tctccgtacg 5280
attgtctctg aaacatcgtg gcctcaggtg ccagggtttg atggacagta gcattagaat 5340
tgtggaaaag gaacacgcaa agggagaagt gtgagaggag aaacaaaata tgagcgttta 5400
aaatacatcg ccattcagtt cgttaaaaaa aaaaaaaaaa aaaaaaaaa 5449

<210> 92
<211> 2881
<212> DNA
<213> human

<400> 92
   acagaagtgc tagaagccag tgctcgtgaa ctaaggagaa aaagaacaga caagggaaca 60
   gcctggacat ggcatcagag atccacatga caggcccaat gtgcctcatt gagaacacta 120
   atgggcgact gatggcgaat ccagaagctc tgaagatcct ttctgccatt acacagccta 180
   tggtggtggt ggcaattgtg ggcctctacc gcacaggcaa atcctacctg atgaacaagc 240
   tggctggaaa gaaaaagggc ttctctctgg gctccacggt gcagtctcac actaaaggaa 300
   tctggatgtg gtgtgtgccc caccccaaga agccaggcca catcctagtt ctgctggaca 360
   ccgagggtct gggagatgta gagaagggtg acaaccagaa tgactcctgg atcttcgccc 420
   tggccgtcct cctgagcagc accttcgtgt acaatagcat aggaaccatc aaccagcagg 480
   ctatggacca actgtactat gtgacagagc tgacacatag aatccgatca aaatcctcac 540
   ctgatgagaa tgagaatgag gttgaggatt cagctgactt tgtgagcttc ttcccagact 600
   ttgtgtggac actgagagat ttctccctgg acttggaagc agatggacaa cccctcacac 660
   cagatgagta cctgacatac tccctgaagc tgaagaaagg taccagtcaa aaagatgaaa 720
   cttttaacct gcccagactc tgtatccgga aattcttccc aaagaaaaaa tgctttgtct 780
   ttgatcggcc cgttcaccgc aggaagcttg cccagctcga gaaactacaa gatgaagagc 840
   tggaccccga atttgtgcaa caagtagcag acttctgttc ctacatcttt agtaattcca 900
   aaactaaaac tctttcagga ggcatccagg tcaacgggcc tcgtctagag agcctggtgc 960
   tgacctacgt caatgccatc agcagtgggg atctgccgtg catggagaac gcagtcctgg 1020
   ccttggccca gatagagaac tcagctgcag tgcaaaaggc tattgcccac tatgaacagc 1080
   agatgggcca gaaggtgcag ctgcccacag aaagcctcca ggagctgctg gacctgcaca 1140
   gggacagtga gagagaggcc attgaagtct tcatcaggag ttccttcaaa gatgtggacc 1200
   atctatttca aaaggagtta gcggcccagc tagaaaaaaa gcgggatgac ttttgtaaac 1260
   agaatcagga agcatcatca gatcgttgct caggtttact tcaggtcatt ttcagtcctc 1320
   tagaagaaga agtgaaggcg ggaatttatt cgaaaccagg gggctatcgt ctctttgttc 1380
   agaagctaca agacctgaag aaaaagtact atgaggaacc gaggaagggg atacaggctg 1440
   aagagattct gcagacatac ttgaaatcca aggagtctat gactgatgca attctccaga 1500
   cagaccagac tctcacagaa aaagaaaagg agattgaagt ggaacgtgtg aaagctgagt 1560
   ctgcacaggc ttcagcaaaa atgttgcagg aaatgcaaag aaagaatgag cagatgatgg 1620
   aacagaagga gaggagttat caggaacact tgaaacaact gactgagaag atggagaacg 1680
   acagggtcca gttgctgaaa gagcaagaga ggaccctcgc tcttaaactt caggaacagg 1740
   agcaactact aaaagaggga tttcaaaaag aaagcagaat aatgaaaaat gagatacagg 1800
   atctccagac gaaaatgaga cgacgaaagg catgtaccat aagctaaaga ccagagcctt 1860
   cctgtcaccc ctaaccaagg cataattgaa acaattttag aatttggaac aagcgtcact 1920
   acatttgata ataattagat cttgcatcat aacaccaaaa gtttataaag gcatgtggta 1980
   caatgatcaa aatcatgttt tttcttaaaa aaaaaaaaaa gactgtaaat tgtgcaacaa 2040
   agatgcattt acctctgtat caactcagga aatctcataa gctggtacca ctcaggagaa 2100
   gtttattctt ccagatgacc agcagtagac aaatggatac tgagcagagt cttaggtaaa 2160
   agtcttggga aatatttggg cattggtctg gccaagtcta caatgtccca atatcaagga 2220
   caaccaccct agcttcttag tgaagacaat gtacagttat ccattagatc aagactacac 2280
   ggtctatgag caataatgtg atttctggac attgcccatg tataatcctc actgatgatt 2340
   tcaagctaaa gcaaaccacc ttatacagag atctagaatc tctttatgtt ctccagagga 2400
   aggtggaaga aaccatgggc aggagtagga attgagtgat aaacaattgg gctaatgaag 2460
   aaaacttctc ttattgttca gttcatccag attataactt caatgggaca ctttagacca 2520
   ttagacaatt gacactggat taaacaaatt cacataatgc caaatacaca atgtatttat 2580
   agcaacgtat aatttgcaaa gatggacttt aaaagatgct gtgtaactaa actgaaataa 2640
   ttcaattact tattatttag aatgttaaag cttatgatag tcttttctaa ttcttaacac 2700
   tcatacttga aatctttccg agtttcccca gaagagaata tgggattttt tttgacattt 2760
   ttgacccatt taataatgct cttgtgttta cctagtatat gtagactttg tcttatgtgt 2820
   caaaagtcct aggaaagtgg ttgatgtttc ttatagcaat taaaaattat ttttgaactg 2880
   a 2881

<210> 93
<211> 2684
<212> DNA
<213> human

<400> 93
   cccgagagga gtcggtggca gcggcggcgg cgggaccggc agcagcagca gcagcagcag 60
   cagcaaccac tagcctcctg ccccgcggcg ctgccgcacg agccccacga gccgctcacc 120
   ccgccgttct cagcgctgcc cgaccccgct ggcgcgccct cccgccgcca gtcccggcag 180
   cgccctcagt tgtcctccga ctcgccctcg gccttccgcg ccagccgcag ccacagccgc 240
   aacgccaccc gcagccacag ccacagccac agccccaggc atagccttcg gcacagcccc 300
   ggctccggct cctgcggcag ctcctctggg caccgtccct gcgccgacat cctggaggtt 360
   gggatgctct tgtccaaaat caactcgctt gcccacctgc gcgccgcgcc ctgcaacgac 420
   ctgcacgcca ccaagctggc gcccggcaag gagaaggagc ccctggagtc gcagtaccag 480
   gtgggcccgc tactgggcag cggcggcttc ggctcggtct actcaggcat ccgcgtctcc 540
   gacaacttgc cggtggccat caaacacgtg gagaaggacc ggatttccga ctggggagag 600
   ctgcctaatg gcactcgagt gcccatggaa gtggtcctgc tgaagaaggt gagctcgggt 660
   ttctccggcg tcattaggct cctggactgg ttcgagaggc ccgacagttt cgtcctgatc 720
   ctggagaggc ccgagccggt gcaagatctc ttcgacttca tcacggaaag gggagccctg 780
   caagaggagc tggcccgcag cttcttctgg caggtgctgg aggccgtgcg gcactgccac 840
   aactgcgggg tgctccaccg cgacatcaag gacgaaaaca tccttatcga cctcaatcgc 900
   ggcgagctca agctcatcga cttcgggtcg ggggcgctgc tcaaggacac cgtctacacg 960
   gacttcgatg ggacccgagt gtatagccct ccagagtgga tccgctacca tcgctaccat 1020
   ggcaggtcgg cggcagtctg gtccctgggg atcctgctgt atgatatggt gtgtggagat 1080
   attcctttcg agcatgacga agagatcatc aggggccagg ttttcttcag gcagagggtc 1140
   tcttcagaat gtcagcatct cattagatgg tgcttggccc tgagaccatc agataggcca 1200
   accttcgaag aaatccagaa ccatccatgg atgcaagatg ttctcctgcc ccaggaaact 1260
   gctgagatcc acctccacag cctgtcgccg gggcccagca aatagcagcc tttctggcag 1320
   gtcctcccct ctcttgtcag atgcccgagg gaggggaagc ttctgtctcc agcttcccga 1380
   gtaccagtga cacgtctcgc caagcaggac agtgcttgat acaggaacaa catttacaac 1440
   tcattccaga tcccaggccc ctggaggctg cctcccaaca gtggggaaga gtgactctcc 1500
   aggggtccta ggcctcaact cctcccatag atactctctt cttctcatag gtgtccagca 1560
   ttgctggact ctgaaatatc ccgggggtgg ggggtggggg tgggtcagaa ccctgccatg 1620
   gaactgtttt cttcatcatg agttctgctg aatgccgcga tgggtcaggt aggggggaaa 1680
   caggttggga tgggatagga ctagcaccat tttaagtccc tgtcacctct tccgactctt 1740
   tctgagtgcc ttctgtgggg actccggctg tgctgggaga aatacttgaa cttgcctctt 1800
   ttacctgctg cttctccaaa aatctgcctg ggttttgttc cctatttttc tctcctgtcc 1860
   tccctcaccc cctccttcat atgaaaggtg ccatggaaga ggctacaggg ccaaacgctg 1920
   agccacctgc ccttttttct gcctccttta gtaaaactcc gagtgaactg gtcttccttt 1980
   ttggttttta cttaactgtt tcaaagccaa gacctcacac acacaaaaaa tgcacaaaca 2040
   atgcaatcaa cagaaaagct gtaaatgtgt gtacagttgg catggtagta tacaaaaaga 2100
   ttgtagtgga tctaattttt aagaaatttt gcctttaagt tattttacct gtttttgttt 2160
   cttgttttga aagatgcgca ttctaacctg gaggtcaatg ttatgtattt atttatttat 2220
   ttatttggtt cccttcctat tccaagcttc catagctgct gccctagttt tctttcctcc 2280
   tttcctcctc tgacttgggg accttttggg ggagggctgc gacgcttgct ctgtttgtgg 2340
   ggtgacggga ctcaggcggg acagtgctgc agctccctgg cttctgtggg gcccctcacc 2400
   tacttaccca ggtgggtccc ggctctgtgg gtgatgggga ggggcattgc tgactgtgta 2460
   tataggataa ttatgaaaag cagttctgga tggtgtgcct tccagatcct ctctggggct 2520
   gtgttttgag cagcaggtag cctgctggtt ttatctgagt gaaatactgt acaggggaat 2580
   aaaagagatc ttattttttt ttttatactt ggcgtttttt gaataaaaac cttttgtctt 2640
   aaaaaaaaaa aaaaaaaaaa aaaaaaaaaa aaaaaaaaaa aaaa 2684

<210> 94
<211> 2709
<212> DNA
<213> human

<400> 94
ccctgacccc gcgccgccga gcgccctgag gactcagcga agggtgggcg ccgccgaggc 60
ctcctgccgc tggcgggttt ccgcggagtg ccgcccggct ccgctctgcc gccggcgcgg 120
ctcatgggca gagtcggccg ggcgggccgg cattaaactg aagaaaagat gtccctgtac 180

gatgacctag gagtggagac cagtgactca aaaacagaag gctggtccaa aaacttcaaa 240
cttctgcagt ctcagcttca ggtgaagaag gcagctctca ctcaggcaaa gagccaaagg 300
acgaaacaaa gtacagtcct cgccccagtc attgacctga agcgaggtgg ctcctcagat 360
gaccggcaaa ttgtggacac tccaccgcat gtagcagctg ggctgaagga tcctgttccc 420
agtgggtttt ctgcagggga agttctgatt cccttagctg acgaatatga ccctatgttt 480
cctaatgatt atgagaaagt agtgaagcgc caaagagagg aacgacagag acagcgggag 540
ctggaaagac aaaaggaaat agaagaaagg gaaaaaaggc gtaaagacag acatgaagca 600
agtgggtttg caaggagacc agatccagat tctgatgaag atgaagatta tgagcgagag 660
aggaggaaaa gaagtatggg cggagctgcc attgccccac ccacttctct ggtagagaaa 720
gacaaagagt taccccgaga ttttccttat gaagaggact caagacctcg atcacagtct 780
tccaaagcag ccattcctcc cccagtgtac gaggaacaag acagaccgag atctccaacc 840
ggacctagca actccttcct cgctaacatg gggggcacgg tggcgcacaa gatcatgcag 900
aagtacggct tccgggaggg ccagggtctg gggaagcatg agcagggcct gagcactgcc 960
ttgtcagtgg agaagaccag caagcgtggc ggcaagatca tcgtgggcga cgccacagag 1020
aaagatgcat ccaagaagtc agattcaaat ccgctgactg aaatacttaa gtgtcctact 1080
aaagtggtct tactaaggaa catggttggt gcgggagagg tggatgaaga cttggaagtt 1140
gaaaccaagg aagaatgtga aaaatatggc aaagttggaa aatgtgtgat atttgaaatt 1200
cctggtgccc ctgatgatga agcagtacgg atatttttag aatttgagag agttgaatca 1260
gcaattaaag cggttgttga cttgaatggg aggtattttg gtggacgggt ggtaaaagca 1320
tgtttctaca atttggacaa attcagggtc ttggatttgg cagaacaagt ttgattttaa 1380
gaactagagc acgagtcatc tccggtgatc cttaaatgaa ctgcaggctg agaaaagaag 1440
gaaaaaggtc acagcctcca tggctgttgc ataccaagac tcttggaagg acttctaaga 1500
tatatgttga ttgatccctt ttttattttg tggtttttta atatagtata aaaatccttt 1560
taaaaaaaca acaatctgtg tgcctctctg gttgtttctc ttttttatta ttactcctga 1620
gttgatgaca ttttttgtta gatttcatgg taattctcaa gtgcttcaat gatgcagcat 1680
ttcttgcact aaaaaaaaaa aaaaaaaaaa aactagaaag ttttgggaca tggggttata 1740
ttaaattatt ctttgttttt ctttttcttt taataaagcc tgcaagttac taaattgtag 1800
tttcataaat tctgtagtaa agtatcatct tggcagtgtg ccaaaggtga aaatgatgct 1860
ttctctaaca gagaaattct tagtgactcc agtcgtagaa aaacgtcttt acaacctgaa 1920
taagattgaa gaattgtgaa cataccatgg cctattggat gaatcatttg ccgtaggcta 1980
aatcagactg tagggtttgt gatggattta tggagtatgt gggtatagaa atcatgaatc 2040
tagcatttgt tttcagagat tcaagcatag tcttaagggt agatcagaaa tgacaaatga 2100
attcaaaacc tagcaggtgc attgtaaatg tgtgcccagt tatgttttgg aaatggcagt 2160
tccttggggt catgtttcta ctggcaaaat ttgcaatagt gttctattgt atgtaatttt 2220

aaaatttata agattatcca cgttggccaa gtaaactgta ctgccaatag aattctggaa 2280
ttgtgagaaa ttgtatcatt gaagttcagt aggatgtgtg gcttaaaaat ttatcaggac 2340
cacaaaaaag aaaacaaaaa tatttggtac tgaggttcat tgccagggca ggaggtattt 2400
ccagaaaata ctcatgcctg tgttctgttc cttgctttcc caaatactgc atgtgacttt 2460
cctaagcggc agctgaaaga ctcgagcccg tgctgtctcc tttggttatt atgacatgaa 2520
agtgtatcaa gaactcagca tttctttgca tccatggact tggtttggag acataaggaa 2580
tattctgacc ctttttaaaa aaggattttc tcatgttttt atttaacata aataaaagaa 2640
taacatttta tcttttgtgg tattatttta ttgaataaaa ttgagtttta tgataaaaaa 2700
aaaaaaaaa 2709

<210> 95
<211> 2150
<212> DNA
<213> human

<400> 95
ctcgagccac gaaggccccg ctgtcctgtc tagcagatac ttgcacggtt tacagaaatt 60
cggtccctgg gtcgtgtcag gaaactggaa aaaaggtcat aagcatgaag cgcagttcag 120
tttccagcgg tggtgctggc cgcctctcca tgcaggagtt aagatcccag gatgtaaata 180
aacaaggcct ctatacccct caaaccaaag agaaaccaac ctttggaaag ttgagtataa 240
acaaaccgac atctgaaaga aaagtctcgc tatttggcaa aagaactagt ggacatggat 300
cccggaatag tcaacttggt atattttcca gttctgagaa aatcaaggac ccgagaccac 360
ttaatgacaa agcattcatt cagcagtgta ttcgacaact ctgtgagttt cttacagaaa 420
atggttatgc acataatgtg tccatgaaat ctctacaagc tccctctgtt aaagacttcc 480
tgaagatctt cacatttctt tatggcttcc tgtgcccctc atacgaactt cctgacacaa 540
agtttgaaga agaggttcca agaatcttta aagaccttgg gtatcctttt gcactatcca 600
aaagctccat gtacacagtg ggggctcctc atacatggcc tcacattgtg gcagccttag 660
tttggctaat agactgcatc aagatacata ctgccatgaa agaaagctca cctttatttg 720
atgatgggca gccttgggga gaagaaactg aagatggaat tatgcataat aagttgtttt 780
tggactacac cataaaatgc tatgagagtt ttatgagtgg tgccgacagc tttgatgaga 840
tgaatgcaga gctgcagtca aaactgaagg atttatttaa tgtggatgct tttaagctgg 900
aatcattaga agcaaaaaac agagcattga atgaacagat tgcaagattg gaacaagaaa 960
gagaaaaaga accgaatcgt ctagagtcgt tgagaaaact gaaggcttcc ttacaaggag 1020
atgttcaaaa gtatcaggca tacatgagca atttggagtc tcattcagcc attcttgacc 1080
agaaattaaa tggtctcaat gaggaaattg ctagagtaga actagaatgt gaaacaataa 1140
aacaggagaa cactcgacta cagaatatca ttgacaacca gaagtactca gttgcagaca 1200
ttgagcgaat aaatcatgaa agaaatgaat tgcagcagac tattaataaa ttaaccaagg 1260

acctggaagc tgaacaacag aagttgtgga atgaggagtt aaaatatgcc agaggcaaag 1320
aagcgattga aacacaatta gcagagtatc acaaattggc tagaaaatta aaacttattc 1380
ctaaaggtgc tgagaattcc aaaggttatg actttgaaat taagtttaat cccgaggctg 1440
gtgccaactg ccttgtcaaa tacagggctc aagtttatgt acctcttaag gaactcctga 1500
atgaaactga agaagaaatt aataaagccc taaataaaaa aatgggtttg gaggatactt 1560
tagaacaatt gaatgcaatg ataacagaaa gcaagagaag tgtgagaact ctgaaagaag 1620
aagttcaaaa gctggatgat ctttaccaac aaaaaattaa ggaagcagag gaagaggatg 1680
aaaaatgtgc cagtgagctt gagtccttgg agaaacacaa gcacctgcta gaaagtactg 1740
ttaaccaggg gctcagtgaa gctatgaatg aattagatgc tgttcagcgg gaataccaac 1800
tagttgtgca aaccacgact gaagaaagac gaaaagtggg aaataacttg caacgtctgt 1860
tagagatggt tgctacacat gttgggtctg tagagaaaca tcttgaggag cagattgcta 1920
aagttgatag agaatatgaa gaatgcatgt cagaagatct ctcggaaaat attaaagaga 1980
ttagagataa gtatgagaag aaagctactc taattaagtc ttctgaagaa tgaagataaa 2040
atgttgatca tgtatatata tccatagtga ataaaattgt ctcagtaaaa aaaaaaaaaa 2100
aaaaaaaaaa aaaaaaaaaa aaaaaaaaaa aaaaaaaaaa aaaaaaaaaa 2150

<210> 96
<211> 2977
<212> DNA
<213> human

<400> 96
ccgaatgtga ccgcctcccg ctccctcacc cgccgcgggg aggaggagcg ggcgagaagc 60
tgccgccgaa cgacaggacg ttggggcggc ctggctccct caggtttaag aattgtttaa 120
gctgcatcaa tggagcacat acagggagct tggaagacga tcagcaatgg ttttggattc 180
aaagatgccg tgtttgatgg ctccagctgc atctctccta caatagttca gcagtttggc 240
tatcagcgcc gggcatcaga tgatggcaaa ctcacagatc cttctaagac aagcaacact 300
atccgtgttt tcttgccgaa caagcaaaga acagtggtca atgtgcgaaa tggaatgagc 360
ttgcatgact gccttatgaa agcactcaag gtgaggggcc tgcaaccaga gtgctgtgca 420
gtgttcagac ttctccacga acacaaaggt aaaaaagcac gcttagattg gaatactgat 480
gctgcgtctt tgattggaga agaacttcaa gtagatttcc tggatcatgt tcccctcaca 540
acacacaact ttgctcggaa gacgttcctg aagcttgcct tctgtgacat ctgtcagaaa 600
ttcctgctca atggatttcg atgtcagact tgtggctaca aatttcatga gcactgtagc 660
accaaagtac ctactatgtg tgtggactgg agtaacatca gacaactctt attgtttcca 720
aattccacta ttggtgatag tggagtccca gcactacctt ctttgactat gcgtcgtatg 780
cgagagtctg tttccaggat gcctgttagt tctcagcaca gatattctac acctcacgcc 840
ttcaccttta acacctccag tccctcatct gaaggttccc tctcccagag gcagaggtcg 900
acatccacac ctaatgtcca catggtcagc accacgctgc ctgtggacag caggatgatt 960

gaggatgcaa ttcgaagtca cagcgaatca gcctcacctt cagccctgtc cagtagcccc 1020
aacaatctga gcccaacagg ctggtcacag ccgaaaaccc ccgtgccagc acaaagagag 1080
cgggcaccag tatctgggac ccaggagaaa aacaaaatta ggcctcgtgg acagagagat 1140
tcaagctatt attgggaaat agaagccagt gaagtgatgc tgtccactcg gattgggtca 1200
ggctcttttg gaactgttta taagggtaaa tggcacggag atgttgcagt aaagatccta 1260
aaggttgtcg acccaacccc agagcaattc caggccttca ggaatgaggt ggctgttctg 1320
cgcaaaacac ggcatgtgaa cattctgctt ttcatggggt acatgacaaa ggacaacctg 1380
gcaattgtga cccagtggtg cgagggcagc agcctctaca aacacctgca tgtccaggag 1440
accaagtttc agatgttcca gctaattgac attgcccggc agacggctca gggaatggac 1500
tatttgcatg caaagaacat catccataga gacatgaaat ccaacaatat atttctccat 1560
gaaggcttaa cagtgaaaat tggagatttt ggtttggcaa cagtaaagtc acgctggagt 1620
ggttctcagc aggttgaaca acctactggc tctgtcctct ggatggcccc agaggtgatc 1680
cgaatgcagg ataacaaccc attcagtttc cagtcggatg tctactccta tggcatcgta 1740
ttgtatgaac tgatgacggg ggagcttcct tattctcaca tcaacaaccg agatcagatc 1800
atcttcatgg tgggccgagg atatgcctcc ccagatctta gtaagctata taagaactgc 1860
cccaaagcaa tgaagaggct ggtagctgac tgtgtgaaga aagtaaagga agagaggcct 1920
ctttttcccc agatcctgtc ttccattgag ctgctccaac actctctacc gaagatcaac 1980
cggagcgctt ccgagccatc cttgcatcgg gcagcccaca ctgaggatat caatgcttgc 2040
acgctgacca cgtccccgag gctgcctgtc ttctagttga ctttgcacct gtcttcaggc 2100
tgccagggga ggaggagaag ccagcaggca ccacttttct gctccctttc tccagaggca 2160
gaacacatgt tttcagagaa gctctgctaa ggaccttcta gactgctcac agggccttaa 2220
cttcatgttg ccttcttttc tatccctttg ggccctggga gaaggaagcc atttgcagtg 2280
ctggtgtgtc ctgctccctc cccacattcc ccatgctcaa ggcccagcct tctgtagatg 2340
cgcaagtgga tgttgatggt agtacaaaaa gcaggggccc agccccagct gttggctaca 2400
tgagtattta gaggaagtaa ggtagcaggc agtccagccc tgatgtggag acacatggga 2460
ttttggaaat cagcttctgg aggaatgcat gtcacaggcg ggactttctt cagagagtgg 2520
tgcagcgcca gacattttgc acataaggca ccaaacagcc caggactgcc gagactctgg 2580
ccgcccgaag gagcctgctt tggtactatg gaacttttct taggggacac gtcctccttt 2640
cacagcttct aaggtgtcca gtgcattggg atggttttcc aggcaaggca ctcggccaat 2700
ccgcatctca gccctctcag gagcagtctt ccatcatgct gaattttgtc ttccaggagc 2760
tgcccctatg gggcgggccg cagggccagc ctgtttctct aacaaacaaa caaacaaaca 2820
gccttgtttc tctagtcaca tcatgtgtat acaaggaagc caggaataca ggttttcttg 2880
atgatttggg ttttaatttt gtttttattg cacctgacaa aatacagtta tctgatggtc 2940
cctcaattat gttattttaa taaaataaat taaattt 2977

<210> 97
<211> 1212
<212> DNA
<213> human

<400> 97
atggaccgat ctaaagaaaa ctgcatttca ggacctgtta aggctacagc tccagttgga 60

ggtccaaaac gtgttctcgt gactcagcaa tttccttgtc agaatccatt acctgtaaat 120

agtggccagg ctcagcgggt cttgtgtcct tcaaattctt cccagcgcat tcctttgcaa 180

gcacaaaagc ttgtctccag tcacaagccg gttcagaatc agaagcagaa gcaattgcag 240

gcaaccagtg tacctcatcc tgtctccagg ccactgaata acacccaaaa gagcaagcag 300

cccctgccat cggcacctga aaataatcct gaggaggaac tggcatcaaa acagaaaaat 360

gaagaatcaa aaaagaggca gtgggctttg gaagactttg aaattggtcg ccctctgggt 420

aaaggaaagt ttggtaatgt ttatttggca agagaaaagc aaagcaagtt tattctggct 480

cttaaagtgt tatttaaagc tcagctggag aaagccggag tggagcatca gctcagaaga 540

gaagtagaaa tacagtccca ccttcggcat cctaatattc ttagactgta tggttatttc 600

catgatgcta ccagagtcta cctaattctg gaatatgcac cacttggaac agtttataga 660

gaacttcaga aactttcaaa gtttgatgag cagagaactg ctacttatat aacagaattg 720

gcaaatgccc tgtcttactg ccattcgaag agagttattc atagagacat taagccagag 780

aacttacttc ttggatcagc tggagagctt aaaattgcag attttgggtg gtcagtacat 840

gctccatctt ccaggaggac cactctctgt ggcaccctgg actacctgcc ccctgaaatg 900

attgaaggtc ggatgcatga tgagaaggtg gatctctgga gccttggagt tctttgctat 960

gaatttttag ttgggaagcc tccttttgag gcaaacacat accaagagac ctacaaaaga 1020

atatcacggg ttgaattcac attccctgac tttgtaacag agggagccag ggacctcatt 1080

tcaagactgt tgaagcataa tcccagccag aggccaatgc tcagagaagt acttgaacac 1140

ccctggatca cagcaaattc atcaaaacca tcaaattgcc aaaacaaaga atcagctagc 1200

aaacagtctt ag 1212

<210> 98
<211> 2788
<212> DNA
<213> human

<400> 98
   ggcggcggta gcagccaggc ttggcccccg gcgtggagca gacgcggacc cctccttcct 60
   ggcggcggcg gcgcgggctc agagcccggc aacgggcggg cgggcagaat gagtctgcag 120
   gtcttaaacg acaaaaatgt cagcaatgaa aaaaatacag aaaattgcga cttcctgttt 180
   tcgccaccag aagttaccgg aagatcgtct gttcttcgtg tgtcacagaa agaaaatgtg 240
   ccacccaaga acctggccaa agctatgaag gtgacttttc agacacctct gcgggatcca 300
   cagacgcaca ggattctaag tcctagcatg gccagcaaac ttgaggctcc tttcactcag 360
   gatgacaccc ttggactgga aaactcacac ccggtctgga cacagaaaga gaaccaacag 420
   ctcatcaagg aagtggatgc caaaactact catggaattc tacagaaacc agtggaggct 480
   gacaccgacc tcctggggga tgcaagccca gcctttggga gtggcagctc cagcgagtct 540
   ggcccaggtg ccctggctga cctggactgc tcaagctctt cccagagccc aggaagttct 600
   gagaaccaaa tggtgtctcc aggaaaagtg tctggcagcc ctgagcaagc cgtggaggaa 660
   aaccttagtt cctattcctt agacagaaga gtgacacccg cctctgagac cctagaagac 720
   ccttgcagga cagagtccca gcacaaagcg gagactccgc acggagccga ggaagaatgc 780
   aaagcggaga ctccgcacgg agccgaggag gaatgccggc acggtggggt ctgtgctccc 840
   gcagcagtgg ccacttcgcc tcctggtgca atccctaagg aagcctgcgg aggagcaccc 900
   ctgcagggtc tgcctggcga agccctgggc tgccctgcgg gtgtgggcac ccccgtgcca 960
   gcagatggca ctcagaccct tacctgtgca cacacctctg ctcctgagag cacagcccca 1020
   accaaccacc tggtggctgg cagggccatg accctgagtc ctcaggaaga agtggctgca 1080
   ggccaaatgg ccagctcctc gaggagcgga cctgtaaaac tagaatttga tgtatctgat 1140
   ggcgccacca gcaaaagggc acccccacca aggagactgg gagagaggtc cggcctcaag 1200
   cctcccttga ggaaagcagc agtgaggcag caaaaggccc cgcaggaggt ggaggaggac 1260
   gacggtagga gcggagcagg agaggacccc cccatgccag cttctcgggg ctcttaccac 1320
   ctcgactggg acaaaatgga tgacccaaac ttcatcccgt tcggaggtga caccaagtct 1380
   ggttgcagtg aggcccagcc cccagaaagc cctgagacca ggctgggcca gccagcggct 1440
   gaacagttgc atgctgggcc tgccacggag gagccaggtc cctgtctgag ccagcagctg 1500
   cattcagcct cagcggagga cacgcctgtg gtgcagttgg cagccgagac cccaacagca 1560
   gagagcaagg agagagcctt gaactctgcc agcacctcgc ttcccacaag ctgtccaggc 1620
   agtgagccag tgcccaccca tcagcagggg cagcctgcct tggagctgaa agaggagagc 1680
   ttcagagacc ccgctgaggt tctaggcacg ggcgcggagg tggattacct ggagcagttt 1740
   ggaacttcct cgtttaagga gtcggccttg aggaagcagt ccttatacct caagttcgac 1800
   cccctcctga gggacagtcc tggtagacca gtgcccgtgg ccaccgagac cagcagcatg 1860
   cacggtgcaa atgagactcc ctcaggacgt ccgcgggaag ccaagcttgt ggagttcgat 1920
   ttcttgggag cactggacat tcctgtgcca ggcccacccc caggtgttcc cgcgcctggg 1980
   ggcccacccc tgtccaccgg acctatagtg gacctgctcc agtacagcca gaaggacctg 2040
   gatgcagtgg taaaggcgac acaggaggag aaccgggagc tgaggagcag gtgtgaggag 2100
   ctccacggga agaacctgga actggggaag atcatggaca ggttcgaaga ggttgtgtac 2160
   caggccatgg aggaagttca gaagcagaag gaactttcca aagctgaaat ccagaaagtt 2220
   ctaaaagaaa aagaccaact taccacagat ctgaactcca tggagaagtc cttctccgac 2280
   ctcttcaagc gttttgagaa acagaaagag gtgatcgagg gctaccgcaa gaacgaagag 2340
   tcactgaaga agtgcgtgga ggattacctg gcaaggatca cccaggaggg ccagaggtac 2400
   caagccctga aggcccacgc ggaggagaag ctgcagctgg caaacgagga gatcgcccag 2460
   gtccggagca aggcccaggc ggaagcgttg gccctccagg ccagcctgag gaaggagcag 2520
   atgcgcatcc agtcgctgga gaagacagtg gagcagaaga ctaaagagaa cgaggagctg 2580
   accaggatct gcgacgacct catctccaag atggagaaga tctgacctcc acggagccgc 2640
   tgtccccgcc cccctgctcc cgtctgtctg tcctgtctga ttctcttagg tgtcatgttc 2700
   ttttttctgt cttgtcttca acttttttta aaactagatt gctttgaaaa catgactcaa 2760
   taaaagtttc ctttcaattt aaaaaaaa 2788

<210> 99
<211> 2287
<212> DNA
<213> human

<400> 99
cgcgccccgc gcagccccgg gcgccgcgcg tcctgcccgg cctgcggccc cagcccttgc 60
gccgctcgtc cgacccgcga tcgtccacca gaccgtgcct cccggccgcc cggccggccc 120
gcgtgcatgc ttcggtctgg gccagcctct gggccgtccg tccccactgg ccgggccatg 180
ccgagtcgcc gcgtcgccag accgccggct gcgccggagc tgggggcctt agggtccccc 240
gacctctcct cactctcgct cgccgtttcc aggagcacag atgaattgga gatcatcgac 300
gagtacatca aggagaacgg cttcggcctg gacgggggac agccgggccc gggcgagggg 360
ctgccacgcc tggtgtctcg cggggctgcg tccctgagca cggtcaccct gggccctgtg 420
gcgcccccag ccacgccgcc gccttggggc tgccccctgg gccgactagt gtccccagcg 480
ccgggcccgg gcccgcagcc gcacctggtc atcacggagc agcccaagca gcgcggcatg 540
cgcttccgct acgagtgcga gggccgctcg gccggcagca tccttgggga gagcagcacc 600
gaggccagca agacgctgcc cgccatcgag ctccgggatt gtggagggct gcgggaggtg 660
gaggtgactg cctgcctggt gtggaaggac tggcctcacc gagtccaccc ccacagcctc 720
gtggggaaag actgcaccga cggcatctgc agggtgcggc tccggcctca cgtcagcccc 780
cggcacagtt ttaacaacct gggcatccag tgtgtgagga agaaggagat tgaggctgcc 840
attgagcgga agattcaact gggcattgac ccctacaacg ctgggtccct gaagaaccat 900
caggaagtag acatgaatgt ggtgaggatc tgcttccagg cctcatatcg ggaccagcag 960
ggacagatgc gccggatgga tcctgtgctt tccgagcccg tctatgacaa gaaatccaca 1020
aacacatcag agctgcggat ttgccgaatt aacaaggaaa gcgggccgtg caccggtggc 1080
gaggagctct acttgctctg cgacaaggtg cagaaagagg acatatcagt ggtgttcagc 1140
agggcctcct gggaaggtcg ggctgacttc tcccaggccg acgtgcaccg ccagattgcc 1200
attgtgttca agacgccgcc ctacgaggac ctggagattg tcgagcccgt gacagtcaac 1260
gtcttcctgc agcggctcac cgatggggtc tgcagcgagc cattgccttt cacgtacctg 1320
cctcgcgacc atgacagcta cggcgtggac aagaagcgga aacgggggat gcccgacgtc 1380

cttggggagc tgaacagctc tgacccccat ggcatcgaga gcaaacggcg gaagaaaaag 1440
ccggccatcc tggaccactt cctgcccaac cacggctcag gcccgttcct cccgccgtca 1500
gccctgctgc cagaccctga cttcttctct ggcaccgtgt ccctgcccgg cctggagccc 1560
cctggcgggc ctgacctcct ggacgatggc tttgcctacg accctacggc ccccacactc 1620
ttcaccatgc tggacctgct gcccccggca ccgccacacg ctagcgctgt tgtgtgcagc 1680
ggaggtgccg gggccgtggt tggggagacc cccggccctg aaccactgac actggactcg 1740
taccaggccc cgggccccgg ggatggaggc accgccagcc ttgtgggcag caacatgttc 1800
cccaatcatt accgcgaggc ggcctttggg ggcggcctcc tatccccggg gcctgaagcc 1860
acgtagcccc gcgatgccag aggaggggca ctgggtgggg agggaggtgg aggagccgtg 1920
caatcccaac caggatgtct agcaccccca tccccttggc ccttcctcat gcttctgaag 1980
tggacatatt cagccttggc gagaagctcc gttgcacggg tttccccttg agcccatttt 2040
acagatgagg aaactgagtc cggagaggaa aagggacatg gctcccgtgc actagcttgt 2100
tacagctgcc tctgtcccca catgtggggg caccttctcc agtaggattc ggaaaagatt 2160
gtacatatgg gaggaggggg cagattcctg gccctccctc cccagacttg aaggtggggg 2220
gtaggttggt tgttcagagt cttcccaata aagatgagtt tttgagcctc aaaaaaaaaa 2280
aaaaaaa 2287

<210> 100
<211> 2850
<212> DNA
<213> human

<400> 100
tcgcacttcc gtgtgccgcg gcgccggagc ccgaggcggc tgtagcccac atctcccgag 60
cgaccccggg cgcccgcccg ccgcgcggag gcccgggcca cacctcactg gccgcttggc 120
ccatcccagt cagcgccgcg ccgaaccccg tccgcgcgcg ccggggagcg gcgcccccgc 180
cgctgccgcc gcgacccttg gcgcctgccc ctgcaacggg aggtctgcag ggaactggcc 240
aggcaagggg gcaggcccgt ttctcctggt ggttggtgcg ttgtagcagc agcgggagcc 300
aggactaagg acaagcagga gctgggagcc ccagccccac tgcaggcccc accatggcgc 360
cgttcctgcg catcgccttc aactcctatg agctgggctc cctgcaggcc gaggacgagg 420
cgaaccagcc cttctgtgcc gtgaagatga aggaggcgct cagcacagag cgtgggaaaa 480
cactggtgca gaagaagccg accatgtatc ctgagtggaa gtcgacgttc gatgcccaca 540
tctatgaggg gcgcgtcatc cagattgtgc taatgcgggc agcagaggag ccagtgtctg 600
aggtgaccgt gggtgtgtcg gtgctggccg agcgctgcaa gaagaacaat ggcaaggctg 660
agttctggct ggacctgcag cctcaggcca aggtgttgat gtctgttcag tatttcctgg 720
aggacgtgga ttgcaaacag tctatgcgca gtgaggacga ggccaagttc ccaacgatga 780
accgccgcgg agccatcaaa caggccaaaa tccactacat caagaaccat gagtttatcg 840
ccaccttctt tgggcaaccc accttctgtt ctgtgtgcaa agactttgtc tggggcctca 900

acaagcaagg ctacaaatgc aggcaatgta acgctgccat ccacaagaaa tgcatcgaca 960
agatcatcgg cagatgcact ggcaccgcgg ccaacagccg ggacactata ttccagaaag 1020
aacgcttcaa catcgacatg ccgcaccgct tcaaggttca caactacatg agccccacct 1080
tctgtgacca ctgcggcagc ctgctctggg gactggtgaa gcagggatta aagtgtgaag 1140
actgcggcat gaatgtgcac cataaatgcc gggagaaggt ggccaacctc tgcggcatca 1200
accagaagct tttggctgag gccttgaacc aagtcaccca gagagcctcc cggagatcag 1260
actcagcctc ctcagagcct gttgggatat atcagggttt cgagaagaag accggagttg 1320
ctggggagga catgcaagac aacagtggga cctacggcaa gatctgggag ggcagcagca 1380
agtgcaacat caacaacttc atcttccaca aggtcctggg caaaggcagc ttcgggaagg 1440
tgctgcttgg agagctgaag ggcagaggag agtactttgc catcaaggcc ctcaagaagg 1500
atgtggtcct gatcgacgac gacgtggagt gcaccatggt tgagaagcgg gtgctgacac 1560
ttgccgcaga gaatcccttt ctcacccacc tcatctgcac cttccagacc aaggaccacc 1620
tgttctttgt gatggagttc ctcaacgggg gggacctgat gtaccacatc caggacaaag 1680
gccgctttga actctaccgt gccacgtttt atgccgctga gataatgtgt ggactgcagt 1740
ttctacacag caagggcatc atttacaggg acctcaaact ggacaatgtg ctgctggacc 1800
gggatggcca catcaagatt gccgactttg ggatgtgcaa agagaacata ttcggggaga 1860
gccgggccag caccttctgc ggcacccctg actatatcgc ccctgagatc ctacagggcc 1920
tgaagtacac attctctgtg gactggtggt ctttcggggt ccttctgtac gagatgctca 1980
ttggccagtc ccccttccat ggtgatgatg aggatgaact cttcgagtcc atccgtgtgg 2040
acacgccaca ttatccccgc tggatcacca aggagtccaa ggacatcctg gagaagctct 2100
ttgaaaggga accaaccaag aggctgggag tgaccggaaa catcaaaatc caccccttct 2160
tcaagaccat aaactggact ctgctggaaa agcggaggtt ggagccacct ttcaggccca 2220
aagtgaagtc acccagagac tacagtaact ttgaccagga gttcctgaac gagaaggcgc 2280
gcctctccta cagcgacaag aacctcatcg actccatgga ccagtctgca ttcgctggct 2340
tctcctttgt gaaccccaaa ttcgagcacc tcctggaaga ttgaggttcc tggacagatc 2400
aggctagccc tgccctccac ccacacctgc ccgctcccca cgataagcac cagtgggact 2460
gtggtgactt ctgctgctgg ccccgcccct gcccccagag cgtccttggc tgccgtctgg 2520
ccgggctctc atggtacttc ctctgtgaac tgtgtgtgaa tctgcttttc ctctgccttc 2580
ggagggaaat tgtaaatcct gtgtttcatt acttgaatgt agttatctat tgaaaatata 2640
tattatatac atagacatat atatatatat aataggctgt atatattgct cagtagagaa 2700
aaaccatggg ggactggtga tatgttgatc tttttcaaaa aaatatatat atgacaaaaa 2760
aaaaaaaaaa ggagcacaag ctgtttgaac caccaggttt atttgtgtgt ctaaataaac 2820
accaaatagt accaaaaaaa aaaaaaaaaa 2850

<210> 101
<211> 1584
<212> DNA
<213> human

<400> 101
gttggttgga gcgtcgcgca gtcgggaggt ccgggaaagt ttctttggag gtccggcccg 60
gagcggccat gtcccacggc cccaagcagc ccggcgcggc cgccgcgccg gcgggcggca 120
aggctccggg ccagcatggg ggcttcgtgg tgactgtcaa gcaagagcgc ggcgagggtc 180
cacgcgcggg cgagaagggg tcccacgagg aggagccggt gaagaaacgc ggctggccca 240
agggcaagaa gcggaagaag attctgccga atgggcccaa ggcaccggtc acgggctacg 300
tgcgcttcct gaacgagcgg cgcgagcaga tccgcacgcg ccacccggat ctgccctttc 360
ccgagatcac caagatgctg ggcgccgagt ggagcaagct gcagccaacg gaaaagcagc 420
ggtacctgga tgaggccgag agagagaagc agcagtacat gaaggagctg cgggcgtacc 480
agcagtctga agcctataag atgtgcacgg agaagatcca ggagaagaag atcaagaaag 540
aagactcgag ctctgggctc atgaacactc tcctgaatgg acacaagggt ggggactgcg 600
atggcttctc caccttcgat gttcccatct tcactgaaga gttcttggac caaaacaaag 660
cgcgtgaggc ggagcttcgg cgcttgcgga agatgaatgt ggccttcgag gagcagaacg 720
cggtactgca gaggcacacg cagagcatga gcagcgcgcg cgagcgtctg gagcaggagc 780
tggcgctgga ggagcggagg acgctggcgc tgcagcagca gctccaggcc gtgcgccagg 840
cgctcaccgc cagcttcgcc tcactgccgg tgccgggcac gggcgaaacg cccacgctgg 900
gcactctgga cttctacatg gcccggcttc acggagccat cgagcgcgac cccgcccagc 960
acgagaagct catcgtccgc atcaaggaaa tcctggccca ggtcgccagc gagcacctgt 1020
gaggagtggg cgggcccacg atgcagagga gaagctgtgg gcgcggccct gccacacccc 1080
accccgtgga cgagaggctg ggggtccacc ctttggggcc tggtcccatc ctgcaccttg 1140
ggggctccag cccccctaaa attaaatttc tgcagcatcc ctttagcttt caatctcccc 1200
agccccctga acccggaaaa agcactcgct gcgcgataca cccagaagaa cctcacagcc 1260
gagggtgccc ctcctcggag gacagccacg cgctacactg gctctccggg ccacccccag 1320
gacacagggc agacgaaacc cacccccagc acacggcagg accccccaaa ttactcacta 1380
cggggggctg tgccataggc cacacaggaa gctgccttgt ggggacttac ctggggtgtc 1440
ccccgcatgc ctgtacccca gatgggtggg ggccggcttt gcccatcctg ctctcctcca 1500
gccgagggac cctggtgggg gtggctcctt ctcactgctg gatccggact ttttaaataa 1560
aaacaagtaa aatttgtgtt ttaa 1584

<210> 102
<211> 2383
<212> DNA
<213> human

<400> 102
gctgctgcga cagtccacta cctttttcga gagtgactcc cgttgtccca aggcttccca 60

gagcgaacct gtgcggctgc aggcaccggc gcgtcgagtt tccggcgtcc ggaaggaccg 120
agctcttctc gcggatccag tgttccgttt ccagccccca atctcagagc cgagccgaca 180
gagagcaggg aaccggcatg gccaaagccg cggcgatcgg catcgacctg ggcaccacct 240
actcctgcgt gggggtgttc caacacggca aggtggagat catcgccaac gaccagggca 300
accgcaccac ccccagctac gtggccttca cggacaccga gcggctcatc ggggatgcgg 360
ccaagaacca ggtggcgctg aacccgcaga acaccgtgtt tgacgcgaag cggctgatcg 420
gccgcaagtt cggcgacccg gtggtgcagt cggacatgaa gcactggcct ttccaggtga 480
tcaacgacgg agacaagccc aaggtgcagg tgagctacaa gggggacacc aaggcattct 540
accccgagga gatctcgtcc atggtgctga ccaagatgaa ggagatcgcc gaggcgtacc 600
tgggctaccc ggtgaccaac gcggtgatca ccgtgccggc ctacttcaac gactcgcagc 660
gccaggccac caaggatgcg ggtgtgatcg cggggctcaa cgtgctgcgg atcatcaacg 720
agcccacggc cgccgccatc gcctacggcc tggacagaac gggcaagggg gagcgcaacg 780
tgctcatctt tgacctgggc gggggcacct tcgacgtgtc catcctgacg atcgacgacg 840
gcatcttcga ggtgaaggcc acggccgggg acacccacct gggtggggag gactttgaca 900
acaggctggt gaaccacttc gtggaggagt tcaagagaaa acacaagaag gacatcagcc 960
agaacaagcg agccgtgagg cggctgcgca ccgcctgcga gagggccaag aggaccctgt 1020
cgtccagcac ccaggccagc ctggagatcg actccctgtt tgagggcatc gacttctaca 1080
cgtccatcac cagggcgagg ttcgaggagc tgtgctccga cctgttccga agcaccctgg 1140
agcccgtgga gaaggctctg cgcgacgcca agctggacaa ggcccagatt cacgacctgg 1200
tcctggtcgg gggctccacc cgcatcccca aggtgcagaa gctgctgcag gacttcttca 1260
acgggcgcga cctgaacaag agcatcaacc ccgacgaggc tgtggcctac ggggcggcgg 1320
tgcaggcggc catcctgatg ggggacaagt ccgagaacgt gcaggacctg ctgctgctgg 1380
acgtggctcc cctgtcgctg gggctggaga cggccggagg cgtgatgact gccctgatca 1440
agcgcaactc caccatcccc accaagcaga cgcagatctt caccacctac tccgacaacc 1500
aacccggggt gctgatccag gtgtacgagg gcgagagggc catgacgaaa gacaacaatc 1560
tgttggggcg cttcgagctg agcggcatcc ctccggcccc caggggcgtg ccccagatcg 1620
aggtgacctt cgacatcgat gccaacggca tcctgaacgt cacggccacg gacaagagca 1680
ccggcaaggc caacaagatc accatcacca acgacaaggg ccgcctgagc aaggaggaga 1740
tcgagcgcat ggtgcaggag gcggagaagt acaaagcgga ggacgaggtg cagcgcgaga 1800
gggtgtcagc caagaacgcc ctggagtcct acgccttcaa catgaagagc gccgtggagg 1860
atgaggggct caagggcaag atcagcgagg ccgacaagaa gaaggtgctg gacaagtgtc 1920
aagaggtcat ctcgtggctg gacgccaaca ccttggccga gaaggacgag tttgagcaca 1980
agaggaagga gctggagcag gtgtgtaacc ccatcatcag cggactgtac cagggtgccg 2040
gtggtcccgg gcctgggggc ttcggggctc agggtcccaa gggagggtct gggtcaggcc 2100

ccaccattga ggaggtagat taggggcctt tccaagattg ctgtttttgt tttggagctt 2160
caagactttg catttcctag tatttctgtt tgtcagttct caatttcctg tgtttgcaat 2220
gttgaaattt tttggtgaag tactgaactt gctttttttc cggtttctac atgcagagat 2280
gaatttatac tgccatctta cgactatttc ttctttttaa tacacttaac tcaggccatt 2340
ttttaagttg gttacttcaa agtaaataaa ctttaaaatt caa 2383

<210> 103
<211> 3283
<212> DNA
<213> human

<400> 103
gcgtgtcggg cgcggaaggg ggaggcggcc cggggcgccc gcgagtgagg cgcggggcgg 60
cgaagggagc gcgggtggcg gcacttgctg ccgcggcctt ggatgggctg ggcccccctc 120
gccgctccgc ctcctccaca cgcgcggcgg ccgcggcgag ggggacgcgc cgcccggggc 180
ccggcacctt cgggaacccc ccggcccgga gcctgcggcc tgcgccgcct cggccgccgg 240
gagccccgtg gagcccccgc cgccgcgccg ccccgcggac cggacgctga gggcactcgg 300
ggcggggcgc gcgctcgggc agacgtttgc ggggaggggg gcgcctgccg ggccccggcg 360
accaccttgg gggtcgcggg ccggctcggg gggcgcccag tgcgggccct cgcgggcgcc 420
gggcagcgac cagccctgag cggagctgtt ggccgcggcg ggaggcctcc cggacgcccc 480
cagccccccg aacgctcgcc cgggccggcg ggagtcggcg ccccccggga ggtccgctcg 540
gtcgtccgcg gcggagcgtt tgctcctggg acaggcggtg ggaccggggc gtcgccggag 600
acgcccccag cgaagttggg ctctccaggt gtgggggtcc cggggggtag cgacgtcgcg 660
gacccggcct gtgggatggg cggcccggag aagactgcgc tcggccgtgt tcatacttgt 720
ccgtgggcct gaggtccccg gaggatgacc tagcactgaa aagccccggc cggcctcccc 780
agggtccccg aggacgaagt tgaccctgac cgggccgtct cccagttctg aggcccgggt 840
cccactggaa ctcgcgtctg agccgccgtc ccggaccccc ggtgcccgcc ggtccgcaga 900
ccctgcaccg ggcttggact cgcagccggg actgacgtgt agaacaatcg tttctgttgg 960
aagaagggtt tttcccttcc ttttggggtt tttgttgcct tttttttttc tttttctttt 1020
gtaaaatttt ggagaaggga agtcggaaca caaggaagga ccgctcaccc gcggactcag 1080
ggctggcggc gggactccag gaccctgggt ccagcatgga ggtggtggac ccgcagcagc 1140
tgggcatgtt cacggagggc gagctgatgt cggtgggtat ggacacgttc atccaccgca 1200
tcgactccac cgaggtcatc taccagccgc gccgcaagcg ggccaagctc atcggcaagt 1260
acctgatggg ggacctgctg ggggaaggct cttacggcaa ggtgaaggag gtgctggact 1320
cggagacgct gtgcaggagg gccgtcaaga tcctcaagaa gaagaagttg cgaaggatcc 1380
ccaacgggga ggccaacgtg aagaaggaaa ttcaactact gaggaggtta cggcacaaaa 1440
atgtcatcca gctggtggat gtgttataca acgaagagaa gcagaaaatg tatatggtga 1500

tggagtactg cgtgtgtggc atgcaggaaa tgctggacag cgtgccggag aagcgtttcc 1560
cagtgtgcca ggcccacggg tacttctgtc agctgattga cggcctggag tacctgcata 1620
gccagggcat tgtgcacaag gacatcaagc cggggaacct gctgctcacc accggtggca 1680
ccctcaaaat ctccgacctg ggcgtggccg aggcactgca cccgttcgcg gcggacgaca 1740
cctgccggac cagccagggc tccccggctt tccagccgcc cgagattgcc aacggcctgg 1800
acaccttctc cggcttcaag gtggacatct ggtcggctgg ggtcaccctc tacaacatca 1860
ccacgggtct gtaccccttc gaaggggaca acatctacaa gttgtttgag aacatcggga 1920
aggggagcta cgccatcccg ggcgactgtg gccccccgct ctctgacctg ctgaaaggga 1980
tgcttgagta cgaaccggcc aagaggttct ccatccggca gatccggcag cacagctggt 2040
tccggaagaa acatcctccg gctgaagcac cagtgcccat cccaccgagc ccagacacca 2100
aggaccggtg gcgcagcatg actgtggtgc cgtacttgga ggacctgcac ggcgcggacg 2160
aggacgagga cctcttcgac atcgaggatg acatcatcta cactcaggac ttcacggtgc 2220
ccggacaggt cccagaagag gaggccagtc acaatggaca gcgccggggc ctccccaagg 2280
ccgtgtgtat gaacggcaca gaggcggcgc agctgagcac caaatccagg gcggagggcc 2340
gggcccccaa ccctgcccgc aaggcctgct ccgccagcag caagatccgc cggctgtcgg 2400
cctgcaagca gcagtgaggc tggccgcctg cagcccgtgt ccaggagccc cgccaggtgc 2460
ccgcgccagg ccctcagtct tcctgccggt tccgcccgcc ctcccggaga ggtggccgcc 2520
atgcttctgt gccgaccacg ccccaggacc tccggagcgc cctgcagggc cgggcagggg 2580
gacagcaggg accgggcgca gccctccccc ctcggccgcc cggcagtgca cgcggcttgt 2640
tgacttcgca gccccgggcg gagccttccc gggcgggcgt gggaggaggg aggcggcctc 2700
catgcacttt atgtggagac tactggcccc gcccgtggcc tcgtgctccg cagggcgccc 2760
agcgccgtcc ggcggccccg ccgcagacca gctggcgggt gtggagacca ggctcctgac 2820
cccgccatgc atgcagcgcc acctggaagc cgcgcggccg ctttggtttt ttgtttggtt 2880
ggttccattt tctttttttc tttttttttt taagaaaaaa taaaaggtgg atttgagctg 2940
tggctgtgag gggtgtttgg gagctgctgg gtggcagggg ggctgtgggg tcgggctcac 3000
gtcgcggccg cctttgcgct ctcgggtcac cctgctttgg cggcccggcc ggagggcagg 3060
accctcacct ctcccccaag gccactgcgc tcttgggacc ccagagaaaa cccggagcaa 3120
gcaggagtgt gcggtcaata tttatatcat ccagaaaaga aaaacacgag aaacgccatc 3180
gcgggatggt gcagacgcgg cggggactcg gagggtgccg tgcgggcgag gccgcccaaa 3240
tttggcaata aataaagctt gggaagcttg gacctgaaaa aaa 3283

<210> 104
<211> 2030
<212> DNA
<213> human

<400> 104
tggctggcaa tggccttgct gacctcgagc cgggcccacg tggggacctt tggagcacag 60

cctacgatcc tggtgcaagg ccggtggatg cagaggccag tccatatacc acccaggcct 120
gcgaggagcg tggtccccac ccatccagcc catatgtgca agtgcccttg acagagaggc 180
tggtcatatc catggtgacc atttatgggc cacaacaggt ccccatctgc gcagtgaacc 240
ctgtgctgag caccttgcag acgtgatctt gcttcgtcct gcagcactgt gcggggcagg 300
aaaatccaag aggaagaagg atctacggat atcctgcatg tccaagccac ccgcacccaa 360
ccccacaccc ccccggaacc tggactcccg gaccttcatc accattggag acagaaactt 420
tgaggtggag gctgatgact tggtgaccat ctcagaactg ggccgtggag cctatggggt 480
ggtagagaag gtgcggcacg cccagagcgg caccatcatg gccgtgaagc ggatccgggc 540
caccgtgaac tcacaggagc agaagcggct gctcatggac ctggacatca acatgcgcac 600
ggtcgactgt ttctacactg tcaccttcta cggggcacta ttcagagagg gagacgtgtg 660
gatctgcatg gagctcatgg acacatcctt ggacaagttc taccggaagg tgctggataa 720
aaacatgaca attccagagg acatccttgg ggagattgct gtgtctatcg tgcgggccct 780
ggagcatctg cacagcaagc tgtcggtgat ccacagagat gtgaagccct ccaatgtcct 840
tatcaacaag gagggccatg tgaagatgtg tgactttggc atcagtggct acttggtgga 900
ctctgtggcc aagacgatgg atgccggctg caagccctac atggcccctg agaggatcaa 960
cccagagctg aaccagaagg gctacaatgt caagtccgac gtctggagcc tgggcatcac 1020
catgattgag atggccatcc tgcggttccc ttacgagtcc tgggggaccc cgttccagca 1080
gctgaagcag gtggtggagg agccgtcccc ccagctccca gccgaccgtt tctcccccga 1140
gtttgtggac ttcactgctc agtgcctgag gaagaacccc gcagagcgta tgagctacct 1200
ggagctgatg gagcacccct tcttcacctt gcacaaaacc aagaagacgg acattgctgc 1260
cttcgtgaag aagatcctgg gagaagactc ataggggctg ggcctcggac cccactccgg 1320
ccctccagag ccccacagcc ccatctgcgg gggcagtgct cacccacacc ataagctact 1380
gccatcctgg cccagggcat ctgggaggaa ccgagggggc tgctcccacc tggctctgtg 1440
gcgagccatt tgtcccaagt gccaaagaag cagaccattg gggctcccag ccaggccctt 1500
gtcggcccca ccagtgcctc tccctgctgc tcctaggacc cgtctccagc tgctgagatc 1560
ctggactgag gggcctggga tgccccctgt ggatgctgct gcccctgcac agcaggctgc 1620
cagtgcctgg gtggatgggc caccgccttg cccagcctgg atgccatcca agttgtatat 1680
ttttttaatc tctcgactga atggactttg cacactttgg cccagggtgg ccacacctct 1740
atcccggctt tggtgcgggg tacacaagag gggatgagtt gtgtgaatac cccaagactc 1800
ccatgaggga gatgccatga gccgcccaag gccttcccct ggcactggca aacagggcct 1860
ctgcggagca cactggctca cccagtcctg cccgccaccg ttatcggtgt cattcacctt 1920
tcgtgttttt tttaatttat cctctgttga tttttctttt tgctttatgg gtttggcttg 1980
tttttcttgc atggtttgga gctgatcgct tctcccccac cccctagggg 2030

<210> 105
<211> 3583
<212> DNA
<213> human

<400> 105
aaaggcctgc agcaggacga ggacctgagc caggaatgca ggatggcggc ggtgaagaag 60
gaagggggtg ctctgagtga agccatgtcc ctggagggag atgaatggga actgagtaaa 120
gaaaatgtac aacctttaag gcaagggcgg atcatgtcca cgcttcaggg agcactggca 180
caagaatctg cctgtaacaa tactcttcag cagcagaaac gggcatttga atatgaaatt 240
cgattttaca ctggaaatga ccctctggat gtttgggata ggtatatcag ctggacagag 300
cagaactatc ctcaaggtgg gaaagagagt aatatgtcaa cgttattaga aagagctgta 360
gaagcactac aaggagaaaa acgatattat agtgatcctc gatttctcaa tctctggctt 420
aaattagggc gtttatgcaa tgagcctttg gatatgtaca gttacttgca caaccaaggg 480
attggtgttt cacttgctca gttctatatc tcatgggcag aagaatatga agctagagaa 540
aactttagga aagcagatgc gatatttcag gaagggattc aacagaaggc tgaaccacta 600
gaaagactac agtcccagca ccgacaattc caagctcgag tgtctcggca aactctgttg 660
gcacttgaga aagaagaaga ggaggaagtt tttgagtctt ctgtaccaca acgaagcaca 720
ctagctgaac taaagagcaa agggaaaaag acagcaagag ctccaatcat ccgtgtagga 780
ggtgctctca aggctccaag ccagaacaga ggactccaaa atccatttcc tcaacagatg 840
caaaataata gtagaattac tgtttttgat gaaaatgctg atgaggcttc tacagcagag 900
ttgtctaagc ctacagtcca gccatggata gcacccccca tgcccagggc caaagagaat 960
gagctgcaag caggcccttg gaacacaggc aggtccttgg aacacaggcc tcgtggcaat 1020
acagcttcac tgatagctgt acccgctgtg cttcccagtt tcactccata tgtggaagag 1080
actgcacaac agccagttat gacaccatgt aaaattgaac ctagtataaa ccacatccta 1140
agcaccagaa agcctggaaa ggaagaagga gatcctctac aaagggttca gagccatcag 1200
caagcgtctg aggagaagaa agagaagatg atgtattgta aggagaagat ttatgcagga 1260
gtaggggaat tctcctttga agaaattcgg gctgaagttt tccggaagaa attaaaagag 1320
caaagggaag ccgagctatt gaccagtgca gagaagagag cagaaatgca gaaacagatt 1380
gaagagatgg agaagaagct aaaagaaatc caaactactc agcaagaaag aacaggtgat 1440
cagcaagaag agacgatgcc tacaaaggag acaactaaac tgcaaattgc ttccgagtct 1500
cagaaaatac caggaatgac tctatccagt tctgtttgtc aagtaaactg ttgtgccaga 1560
gaaacttcac ttgcggagaa catttggcag gaacaacctc attctaaagg tcccagtgta 1620
cctttctcca tttttgatga gtttcttctt tcagaaaaga agaataaaag tcctcctgca 1680
gatcccccac gagttttagc tcaacgaaga ccccttgcag ttctcaaaac ctcagaaagc 1740
atcacctcaa atgaagatgt gtctccagat gtttgtgatg aatttacagg aattgaaccc 1800
ttgagcgagg atgccattat cacaggcttc agaaatgtaa caatttgtcc taacccagaa 1860

gacacttgtg actttgccag agcagctcgt tttgtatcca ctccttttca tgagataatg 1920
tccttgaagg atctcccttc tgatcctgag agactgttac cggaagaaga tctagatgta 1980
aagacctctg aggaccagca gacagcttgt ggcactatct acagtcagac tctcagcatc 2040
aagaagctga gcccaattat tgaagacagt cgtgaagcca cacactcctc tggcttctct 2100
ggttcttctg cctcggttgc aagcacctcc tccatcaaat gtcttcaaat tcctgagaaa 2160
ctagaactta ctaatgagac ttcagaaaac cctactcagt caccatggtg ttcacagtat 2220
cgcagacagc tactgaagtc cctaccagag ttaagtgcct ctgcagagtt gtgtatagaa 2280
gacagaccaa tgcctaagtt ggaaattgag aaggaaattg aattaggtaa tgaggattac 2340
tgcattaaac gagaatacct aatatgtgaa gattacaagt tattctgggt ggcgccaaga 2400
aactctgcag aattaacagt aataaaggta tcttctcaac ctgtcccatg ggacttttat 2460
atcaacctca agttaaagga acgtttaaat gaagattttg atcatttttg cagctgttat 2520
caatatcaag atggctgtat tgtttggcac caatatataa actgcttcac ccttcaggat 2580
cttctccaac acagtgaata tattacccat gaaataacag tgttgattat ttataacctt 2640
ttgacaatag tggagatgct acacaaagca gaaatagtcc atggtgactt gagtccaagg 2700
tgtctgattc tcagaaacag aatccacgat ccctatgatt gtaacaagaa caatcaagct 2760
ttgaagatag tggacttttc ctacagtgtt gaccttaggg tgcagctgga tgtttttacc 2820
ctcagcggct ttcggactgt acagatcctg gaaggacaaa agatcctggc taactgttct 2880
tctccctacc aggtagacct gtttggtata gcagatttag cacatttact attgttcaag 2940
gaacacctac aggtcttctg ggatgggtcc ttctggaaac ttagccaaaa tatttctgag 3000
ctaaaagatg gtgaattgtg gaataaattc tttgtgcgga ttctgaatgc caatgatgag 3060
gccacagtgt ctgttcttgg ggagcttgca gcagaaatga atggggtttt tgacactaca 3120
ttccaaagtc acctgaacaa agccttatgg aaggtaggga agttaactag tcctggggct 3180
ttgctctttc agtgagctag gcaatcaagt ctcacagatt gctgcctcag agcaatggtt 3240
gtattgtgga acactgaaac tgtatgtgct gtaatttaat ttaggacaca tttagatgca 3300
ctaccattgc tgttctactt tttggtacag gtatattttg acgtcactga tattttttat 3360
acagtgatat acttactcat ggccttgtct aacttttgtg aagaactatt ttattctaaa 3420
cagactcatt acaaatggtt accttgttat ttaacccatt tgtctctact tttccctgta 3480
cttttcccat ttgtaatttg taaaatgttc tcttatgatc accatgtatt ttgtaaataa 3540
taaaatagta tctgttaaaa aaaaaaaaaa aaaaaaaaaa aaa 3583

<210> 106
<211> 1379
<212> DNA
<213> human

<400> 106
ctgttggagc cgctgtggtt gctgtccgcg gagtggaagc gcgtgctttt gtttgtgtcc 60
ctggccatgg cgctgcagct ctcccgggag cagggaatca ccctgcgcgg gagcgccgaa 120

atcgtggccg agttcttctc attcggcatc aacagcattt tatatcagcg tggcatatat 180
ccatctgaaa cctttactcg agtgcagaaa tacggactca ccttgcttgt aactactgat 240
cttgagctca taaaatacct aaataatgtg gtggaacaac tgaaagattg gttatacaag 300
tgttcagttc agaaactggt tgtagttatc tcaaatattg aaagtggtga ggtcctggaa 360
agatggcagt ttgatattga gtgtgacaag actgcaaaag atgacagtgc acccagagaa 420
aagtctcaga aagctatcca ggatgaaatc cgttcagtga tcagacagat cacagctacg 480
gtgacatttc tgccactgtt ggaagtttct tgttcatttg atctgctgat ttatacagac 540
aaagatttgg ttgtacctga aaaatgggaa gagtcgggac cacagtttat taccaattct 600
gaggaagtcc gccttcgttc atttactact acaatccaca aagtaaatag catggtggcc 660
tacaaaattc ctgtcaatga ctgaggatga catgaggaaa ataatgtaat tgtaattttg 720
aaatgtggtt ttcctgaaat caagtcatct atagttgata tgttttattt cattggttaa 780
tttttacatg gagaaaacca aaatgatact tactgaactg tgtgtaattg ttcctttatt 840
tttttggtac ctatttgact taccatggag ttaacatcat gaatttattg cacattgttc 900
aaaaggaacc aggaggtttt tttgtcaaca ttgtgatgta tattcctttg aagatagtaa 960
ctgtagatgg aaaaacttgt gctataaagc tagatgcttt cctaaatcag atgttttggt 1020
caagtagttt gactcagtat aggtagggag atatttaagt ataaaataca acaaaggaag 1080
tctaaatatt cagaatcttt gttaaggtcc tgaaagtaac tcataatcta taaacaatga 1140
aatattgctg tatagctcct tttgaccttc atttcatgta tagttttccc tattgaatca 1200
gtttccaatt atttgacttt aatttatgta acttgaacct atgaagcaat ggatatttgt 1260
actgtttaat gttctgtgat acagaactct taaaaatgtt ttttcatgtg ttttataaaa 1320
tcaagtttta agtgaaagtg aggaaataaa gttaagtttg ttttaaattt gtcttaaaa 1379

<210> 107
<211> 2984
<212> DNA
<213> human

<400> 107
ggaaattcaa acgtgtttgc ggaaaggagt ttgggttcca tcttttcatt tccccagcgc 60
agctttctgt agaaatggaa tccgaggatt taagtggcag agaattgaca attgattcca 120
taatgaacaa agtgagagac attaaaaata agtttaaaaa tgaagacctt actgatgaac 180
taagcttgaa taaaatttct gctgatacta cagataactc gggaactgtt aaccaaatta 240
tgatgatggc aaacaaccca gaggactggt tgagtttgtt gctcaaacta gagaaaaaca 300
gtgttccgct aagtgatgct cttttaaata aattgattgg tcgttacagt caagcaattg 360
aagcgcttcc cccagataaa tatggccaaa atgagagttt tgctagaatt caagtgagat 420
ttgctgaatt aaaagctatt caagagccag atgatgcacg tgactacttt caaatggcca 480
gagcaaactg caagaaattt gcttttgttc atatatcttt tgcacaattt gaactgtcac 540

aaggtaatgt caaaaaaagt aaacaacttc ttcaaaaagc tgtagaacgt ggagcagtac 600
cactagaaat gctggaaatt gccctgcgga atttaaacct ccaaaaaaag cagctgcttt 660
cagaggagga aaagaagaat ttatcagcat ctacggtatt aactgcccaa gaatcatttt 720
ccggttcact tgggcattta cagaatagga acaacagttg tgattccaga ggacagacta 780
ctaaagccag gtttttatat ggagagaaca tgccaccaca agatgcagaa ataggttacc 840
ggaattcatt gagacaaact aacaaaacta aacagtcatg cccatttgga agagtcccag 900
ttaaccttct aaatagccca gattgtgatg tgaagacaga tgattcagtt gtaccttgtt 960
ttatgaaaag acaaacctct agatcagaat gccgagattt ggttgtgcct ggatctaaac 1020
caagtggaaa tgattcctgt gaattaagaa atttaaagtc tgttcaaaat agtcatttca 1080
aggaacctct ggtgtcagat gaaaagagtt ctgaacttat tattactgat tcaataaccc 1140
tgaagaataa aacggaatca agtcttctag ctaaattaga agaaactaaa gagtatcaag 1200
aaccagaggt tccagagagt aaccagaaac agtggcaatc taagagaaag tcagagtgta 1260
ttaaccagaa tcctgctgca tcttcaaatc actggcagat tccggagtta gcccgaaaag 1320
ttaatacaga gcagaaacat accacttttg agcaacctgt cttttcagtt tcaaaacagt 1380
caccaccaat atcaacatct aaatggtttg acccaaaatc tatttgtaag acaccaagca 1440
gcaatacctt ggatgattac atgagctgtt ttagaactcc agttgtaaag aatgactttc 1500
cacctgcttg tcagttgtca acaccttatg gccaacctgc ctgtttccag cagcaacagc 1560
atcaaatact tgccactcca cttcaaaatt tacaggtttt agcatcttct tcagcaaatg 1620
aatgcatttc ggttaaagga agaatttatt ccattttaaa gcagatagga agtggaggtt 1680
caagcaaggt atttcaggtg ttaaatgaaa agaaacagat atatgctata aaatatgtga 1740
acttagaaga agcagataac caaactcttg atagttaccg gaacgaaata gcttatttga 1800
ataaactaca acaacacagt gataagatca tccgacttta tgattatgaa atcacggacc 1860
agtacatcta catggtaatg gagtgtggaa atattgatct taatagttgg cttaaaaaga 1920
aaaaatccat tgatccatgg gaacgcaaga gttactggaa aaatatgtta gaggcagttc 1980
acacaatcca tcaacatggc attgttcaca gtgatcttaa accagctaac tttctgatag 2040
ttgatggaat gctaaagcta attgattttg ggattgcaaa ccaaatgcaa ccagatacaa 2100
caagtgttgt taaagattct caggttggca cagttaatta tatgccacca gaagcaatca 2160
aagatatgtc ttcctccaga gagaatggga aatctaagtc aaagataagc cccaaaagtg 2220
atgtttggtc cttaggatgt attttgtact atatgactta cgggaaaaca ccatttcagc 2280
agataattaa tcagatttct aaattacatg ccataattga tcctaatcat gaaattgaat 2340
ttcccgatat tccagagaaa gatcttcaag atgtgttaaa gtgttgttta aaaagggacc 2400
caaaacagag gatatccatt cctgagctcc tggctcatcc ctatgttcaa attcaaactc 2460
atccagttaa ccaaatggcc aagggaacca ctgaagaaat gaaatatgtt ctgggccaac 2520
ttgttggtct gaattctcct aactccattt tgaaagctgc taaaacttta tatgaacact 2580

atagtggtgg tgaaagtcat aattcttcat cctccaagac ttttgaaaaa aaaaggggaa 2640
aaaaatgatt tgcagttatt cgtaatgtca aataccacct ataaaatata ttggactgtt 2700
atactcttga atccctgtgg aaatctacat ttgaagacaa catcactctg aagtgttatc 2760
agcaaaaaaa attcagtaga ttatctttaa aagaaaactg taaaaatagc aaccacttat 2820
ggtactgtat atattgtaga cttgttttct ctgttttatg ctcttgtgta atctacttga 2880
catcatttta ctcttggaat agtgggtgga tagcaagtat attctaaaaa actttgtaaa 2940
taaagttttg tggctaaaat gacactaaaa aaaaaaaaaa aaaa 2984

<210> 108
<211> 2166
<212> DNA
<213> human

<400> 108
   gggggtcgct gtgcctgtcc ccgtgtgatc cgaaaagtgc tggcaaaatg cggctgctgc 60
   ttcgcccggg ggggtaaggg ggtgccctgg tgaaattttc tatcctggcc aactcaaatg 120
   aggatggcaa gagagtgagc ccttggagag agaggctgag catcatgaag agaagaccct 180
   gggagttccc tggggaccct gagtctggag ctggggcaag gccccccagg gcccgtcagt 240
   ggcctgagga gatggttgga tcattccaaa cattgtctca gtgtggaaac tgaggcagac 300
   agtggtcagg caggaccata tgagaactgg atgttggagc cagctctagc cacaggagag 360
   gagctgccgg aactgacctt gctgaccaca ctgttggagg gccctggaga taagacgcag 420
   ccacctgaag aggagacttt gtcccaagcc cctgagagtg aggaggaaca gaagaagaag 480
   gctctggaaa ggagtatgta tgtcctgagt gaactggtag aaacagagaa aatgtacgtg 540
   gacgacttgg ggcagattgt ggagggttat atggccacca tggctgctca gggggtcccc 600
   gagagtcttc gaggccgtga caggattgtg tttgggaata tccagcaaat ctatgagtgg 660
   caccgagact atttcttgca agagctacaa cggtgtctga aagatcctga ttggctggct 720
   cagctattca tcaaacacga gcgccggctg catatgtatg tggtgtactg tcagaataag 780
   cccaagtcag agcatgtggt gtcagagttt ggggacagct actttgagga gctccggcag 840
   cagctggggc accgcctgca gctgaacgac ctcctcatca aacctgtgca gcggatcatg 900
   aaataccagc tgctgctcaa ggattttctc aagtattaca atagagctgg gatggatact 960
   gcagacctag agcaagctgt ggaggtcatg tgctttgtgc ccaagcgctg caacgatatg 1020
   atgacgctgg ggagattgcg gggatttgag ggcaaactga ctgctcaggg gaagctcttg 1080
   ggccaggaca ctttctgggt caccgagcct gaggctggag ggctgctatc ttcccgaggt 1140
   cgagagaggc gcgtcttcct ctttgagcaa atcatcatct tcagtgaagc cctgggagga 1200
   ggagtgagag gtggaacaca gcctggatat gtatacaaga acagcattaa ggtgagctgc 1260
   ctgggactgg aggggaacct ccaaggtgac ccttgccgct ttgcactgac ctccagaggg 1320
   ccagagggtg ggatccagcg ctatgtcctg caggctgcag accctgctat cagtcaggcc 1380
   tggatcaagc atgtggctca gatcttggag agccaacggg acttcctcaa cgcattgcag 1440
   tcacccattg agtaccagag acgggagagc cagaccaaca gcctggggcg gccaagaggg 1500
   cctggagtgg ggagccctgg aagaattcgg cttggagatc aggcccaggg cagcacacac 1560
   acacccatca atggctctct cccctctctg ctgctgtcac ccaaagggga ggtggccaga 1620
   gccctcttgc cactggataa acaggccctt ggtgacatcc cccaggctcc ccatgactct 1680
   cctccagtct ctccaactcc aaaaacccct ccctgccaag ccagacttgc caagctggat 1740
   gaagatgagc tgtaactggt gaaaaccatg ggggtggtgc tgactcagcc gcctattccc 1800
   caaggagctt cagggcagtc cttctggcac tgctccagaa ttcctccttc ttggtgtgtc 1860
   tggagggtgg gcaaggctgg gagggatatc aacttggagg agaacaccta gacccaagga 1920
   cttttttctg cccaaggaac acagtttcct tcagctccca tccctatgca tgcatcatgg 1980
   tccccccaaa aggaggatat gtgggtgggt gggagggctg gggcaggggc cagatagaaa 2040
   ttattggttt tgttttttaa ttttgttttt cctgttttct gagaataaag gttttgttat 2100
   atcaaaaaaa aaaaaaaaaa aaaaaaaaaa aaaaaaaaaa aaaaaaaaaa aaaaaaaaaa 2160
   aaaaaa 2166

<210> 109
<211> 3486
<212> DNA
<213> human

<400> 109
gagccgactg cggctgcgcg gggtattcga atcggcggcg gcttctagtt tgcggttcag 60
gtttggccgc tgccggccag cgtcctctgg ccatggacac cccggaaaat gtccttcaga 120
tgcttgaagc ccacatgcag agctacaagg gcaatgaccc tcttggtgaa tgggaaagat 180
acatacagtg ggtagaagag aattttcctg agaataaaga atacttgata actttactag 240
aacatttaat gaaggaattt ttagataaga agaaatacca caatgaccca agattcatca 300
gttattgttt aaaatttgct gagtacaaca gtgacctcca tcaatttttt gagtttctgt 360
acaaccatgg gattggaacc ctgtcatccc ctctgtacat tgcctgggcg gggcatctgg 420
aagcccaagg agagctgcag catgccagtg ctgtccttca gagaggaatt caaaaccagg 480
ctgaacccag agagttcctg caacaacaat acaggttatt tcagacacgc ctcactgaaa 540
cccatttgcc agctcaagct agaacctcag aacctctgca taatgttcag gttttaaatc 600
aaatgataac atcaaaatca aatccaggaa ataacatggc ctgcatttct aagaatcagg 660
gttcagagct ttctggagtg atatcttcag cttgtgataa agagtcaaat atggaacgaa 720
gagtgatcac gatttctaaa tcagaatatt ctgtgcactc atctttggca tccaaagttg 780
atgttgagca ggttgttatg tattgcaagg agaagcttat tcgtggggaa tcagaatttt 840
cctttgaaga attgagagcc cagaaataca atcaacggag aaagcatgag caatgggtaa 900
atgaagacag acattatatg aaaaggaaag aagcaaatgc ttttgaagaa cagctattaa 960
aacagaaaat ggatgaactt cataagaagt tgcatcaggt ggtggagaca tcccatgagg 1020

atctgcccgc ttcccaggaa aggtccgagg ttaatccagc acgtatgggg ccaagtgtag 1080
gctcccagca ggaactgaga gcgccatgtc ttccagtaac ctatcagcag acaccagtga 1140
acatggaaaa gaacccaaga gaggcacctc ctgttgttcc tcctttggca aatgctattt 1200
ctgcagcttt ggtgtcccca gccaccagcc agagcattgc tcctcctgtt cctttgaaag 1260
cccagacagt aacagactcc atgtttgcag tggccagcaa agatgctgga tgtgtgaata 1320
agagtactca tgaattcaag ccacagagtg gagcagagat caaagaaggg tgtgaaacac 1380
ataaggttgc caacacaagt tcttttcaca caactccaaa cacatcactg ggaatggttc 1440
aggcaacgcc atccaaagtg cagccatcac ccaccgtgca cacaaaagaa gcattaggtt 1500
tcatcatgaa tatgtttcag gctcctacac ttcctgatat ttctgatgac aaagatgaat 1560
ggcaatctct agatcaaaat gaagatgcat ttgaagccca gtttcaaaaa aatgtaaggt 1620
catctggggc ttggggagtc aataagatca tctcttcttt gtcatctgct tttcatgtgt 1680
ttgaagatgg aaacaaagaa aattatggat taccacagcc taaaaataaa cccacaggag 1740
ccaggacctt tggagaacgc tctgtcagca gacttccttc aaaaccaaag gaggaagtgc 1800
ctcatgctga agagtttttg gatgactcaa ctgtatgggg tattcgctgc aacaaaaccc 1860
tggcacccag tcctaagagc ccaggagact tcacatctgc tgcacaactt gcgtctacac 1920
cattccacaa gcttccagtg gagtcagtgc acattttaga agataaagaa aatgtggtag 1980
caaaacagtg tacccaggcg actttggatt cttgtgagga aaacatggtg gtgccttcaa 2040
gggatggaaa attcagtcca attcaagaga aaagcccaaa acaggccttg tcgtctcaca 2100
tgtattcagc atccttactt cgtctgagcc agcctgctgc aggtggggta cttacctgtg 2160
aggcagagtt gggcgttgag gcttgcagac tcacagacac tgacgctgcc attgcagaag 2220
atccaccaga tgctattgct gggctccaag cagaatggat gcagatgagt tcacttggga 2280
ctgttgatgc tccaaacttc attgttggga acccatggga tgataagctg attttcaaac 2340
ttttatctgg gctttctaaa ccagtgagtt cctatccaaa tacttttgaa tggcaatgta 2400
aacttccagc catcaagccc aagactgaat ttcaattggg ttctaagctg gtctatgtcc 2460
atcaccttct tggagaagga gcctttgccc aggtgtacga agctacccag ggagatctga 2520
atgatgctaa aaataaacag aaatttgttt taaaggtcca aaagcctgcc aacccctggg 2580
aattctacat tgggacccag ttgatggaaa gactaaagcc atctatgcag cacatgttta 2640
tgaagttcta ttctgcccac ttattccaga atggcagtgt attagtagga gagctctaca 2700
gctatggaac attattaaat gccattaacc tctataaaaa tacccctgaa aaagtgatgc 2760
ctcaaggtct tgtcatctct tttgctatga gaatgcttta catgattgag caagtgcatg 2820
actgtgaaat cattcatgga gacattaaac cagacaattt catacttgga aacggatttt 2880
tggaacagga tgatgaagat gatttatctg ctggcttggc actgattgac ctgggtcaga 2940
gtatagatat gaaacttttt ccaaaaggaa ctatattcac agcaaagtgt gaaacatctg 3000
gttttcagtg tgttgagatg ctcagcaaca aaccatggaa ctaccagatc gattactttg 3060

gggttgctgc aacagtatat tgcatgctct ttggcactta catgaaagtg aaaaatgaag 3120
gaggagagtg taagcctgaa ggtcttttta gaaggcttcc tcatttggat atgtggaatg 3180
aattttttca tgttatgttg aatattccag attgtcatca tcttccatct ttggatttgt 3240
taaggcaaaa gctgaagaaa gtatttcaac aacactatac taacaagatt agggccctac 3300
gtaataggct aattgtactg ctcttagaat gtaagcgttc acgaaaataa aatttggata 3360
tagacagtcc ttaaaaatca cactgtaaat atgaatctgc tcactttaaa cctgtttttt 3420
tttcatttat tgtttatgta aatgtttgtt aaaaataaat cccatggaat atttccatgt 3480
aaaaaa 3486

<210> 110
<211> 6641
<212> DNA
<213> human

<400> 110
ggttacattt tggatcctcg cggagtactg gtcaggcggt taagtcctgt acctaggaaa 60
gagggcgagc tctggggcgc tctccggtgt catgaggagc ttcaaaagag tcaactttgg 120
gactctgcta agcagccaga aggaggctga agagttgctg cccgccttga aggagttcct 180
gtccaaccct ccagctggtt ttcccagcag ccgatctgat gctgagagga gacaagcttg 240
tgatgccatc ctgagggctt gcaaccagca gctgactgct aagctagctt gccctaggca 300
tctggggagc ctgctggagc tggcagagct ggcctgtgat ggctacttag tgtctacccc 360
acagcgtcct cccctctacc tggaacgaat tctctttgtc ttactgcgga atgctgctgc 420
acaaggaagc ccagaggcca cactccgcct tgctcagccc ctccatgcct gcttggtgca 480
gtgctctcgc gaggctgctc cccaggacta tgaggccgtg gctcggggca gcttttctct 540
gctttggaag ggggcagaag ccctgttgga acggcgagct gcatttgcag ctcggctgaa 600
ggccttgagc ttcctagtac tcttggagga tgaaagtacc ccttgtgagg ttcctcactt 660
tgcttctcca acagcctgtc gagcggtagc tgcccatcag ctatttgatg ccagtggcca 720
tggtctaaat gaagcagatg ctgatttcct agatgacctg ctctccaggc acgtgatcag 780
agccttggtg ggtgagagag ggagctcttc tgggcttctt tctccccaga gggccctctg 840
cctcttggag ctcaccttgg aacactgccg tcgcttttgc tggagccgcc accatgacaa 900
agccatcagc gcagtggaga aggctcacag ttacctaagg aacaccaatc tagcccctag 960
ccttcagcta tgtcagctgg gggttaagct gctgcaggtt ggggaggaag gacctcaggc 1020
agtggccaag cttctgatca aggcatcagc tgtcctgagc aagagtatgg aggcaccatc 1080
acccccactt cgggcattgt atgagagctg ccagttcttc ctttcaggcc tggaacgagg 1140
caccaagagg cgctatagac ttgatgccat tctgagcctc tttgcttttc ttggagggta 1200
ctgctctctt ctgcagcagc tgcgggatga tggtgtgtat gggggctcct ccaagcaaca 1260
gcagtctttt cttcagatgt actttcaggg acttcacctc tacactgtgg tggtttatga 1320
ctttgcccaa ggctgtcaga tagttgattt ggctgacctg acccaactag tggacagttg 1380

taaatctacc gttgtctgga tgctggaggc cttagagggc ctgtcgggcc aagagctgac 1440
ggaccacatg gggatgaccg cttcttacac cagtaatttg gcctacagct tctatagtca 1500
caagctctat gccgaggcct gtgccatctc tgagccgctc tgtcagcacc tgggtttggt 1560
gaagccaggc acttatcccg aggtgcctcc tgagaagttg cacaggtgct tccggctaca 1620
agtagagagt ttgaagaaac tgggtaaaca ggcccagggc tgcaagatgg tgattttgtg 1680
gctggcagcc ctgcaaccct gtagccctga acacatggct gagccagtca ctttctgggt 1740
tcgggtcaag atggatgcgg ccagggctgg agacaaggag ctacagctaa agactctgcg 1800
agacagcctc agtggctggg acccggagac cctggccctc ctgctgaggg aggagctgca 1860
ggcctacaag gcggtgcggg ccgacactgg acaggaacgc ttcaacatca tctgtgacct 1920
cctggagctg agccccgagg agacaccagc cggggcctgg gcacgagcca cccacctggt 1980
agaactggct caggtgctct gctaccacga ctttacgcag cagaccaact gctctgctct 2040
ggatgctatc cgggaagccc tgcagcttct ggactctgtg aggcctgagg cccaggccag 2100
agatcagctt ctggacgata aagcacaggc cttgctgtgg ctttacatct gtactctgga 2160
agccaaaatg caggaaggta tcgagcggga tcggagagcc caggcccctg gtaacttgga 2220
ggaatttgaa gtcaatgacc tgaactatga agataaactc caggaagatc gtttcctata 2280
cagtaacatt gccttcaacc tggctgcaga tgctgctcag tccaaatgcc tggaccaagc 2340
cctggccctg tggaaggagc tgcttacaaa ggggcaggcc ccagctgtac ggtgtctcca 2400
gcagacagca gcctcactgc agatcctagc agccctctac cagctggtgg caaagcccat 2460
gcaggctctg gaggtcctcc tgctgctacg gattgtctct gagagactga aggaccactc 2520
gaaggcagct ggctcctcct gccacatcac ccagctcctc ctgaccctcg gctgtcccag 2580
ctatgcccag ttacacctgg aagaggcagc atcgagcctg aagcatctcg atcagactac 2640
tgacacatac ctgctccttt ccctgacctg tgatctgctt cgaagtcaac tctactggac 2700
tcaccagaag gtgaccaagg gtgtctctct gctgctgtct gtgcttcggg atcctgccct 2760
ccagaagtcc tccaaggctt ggtacttgct gcgtgtccag gtcctgcagc tggtggcagc 2820
ttaccttagc ctcccgtcaa acaacctctc acactccctg tgggagcagc tctgtgccca 2880
aggctggcag acacctgaga tagctctcat agactcccat aagctcctcc gaagcatcat 2940
cctcctgctg atgggcagtg acattctctc aactcagaaa gcagctgtgg agacatcgtt 3000
tttggactat ggtgaaaatc tggtacaaaa atggcaggtt ctttcagagg tgctgagctg 3060
ctcagagaag ctggtctgcc acctgggccg cctgggtagt gtgagtgaag ccaaggcctt 3120
ttgcttggag gccctaaaac ttacaacaaa gctgcagata ccacgccagt gtgccctgtt 3180
cctggtgctg aagggcgagc tggagctggc ccgcaatgac attgatctct gtcagtcgga 3240
cctgcagcag gttctgttct tgcttgagtc ttgcacagag tttggtgggg tgactcagca 3300
cctggactct gtgaagaagg tccacctgca gaaggggaag cagcaggccc aggtcccctg 3360
tcctccacag ctcccagagg aggagctctt cctaagaggc cctgctctag agctggtggc 3420

cactgtggcc aaggagcctg gccccatagc accttctaca aactcctccc cagtcttgaa 3480
aaccaagccc cagcccatac ccaacttcct gtcccattca cccacctgtg actgctcgct 3540
ctgcgccagc cctgtcctca cagcagtctg tctgcgctgg gtattggtca cggcaggggt 3600
gaggctggcc atgggccacc aagcccaggg tctggatctg ctgcaggtcg tgctgaaggg 3660
ctgtcctgaa gccgctgagc gcctcaccca agctctccaa gcttccctga atcataaaac 3720
acccccctcc ttggttccaa gcctcttgga tgagatcttg gctcaagcat acacactgtt 3780
ggcactggag ggcctgaacc agccatcaaa cgagagcctg cagaaggttc tacagtcagg 3840
gctgaagttt gtagcagcac ggatacccca cctagagccc tggcgagcca gcctgctctt 3900
gatttgggcc ctcacaaaac taggtggcct cagctgctgt actacccaac tttttgcaag 3960
ctcctggggc tggcagccac cattaataaa aagtgtccct ggctcagagc cctctaagac 4020
tcagggccaa aaacgttctg gacgagggcg ccaaaagtta gcctctgctc ccctgcgcct 4080
caataatacc tctcagaaag gtctggaagg tagaggactg ccctgcacac ctaaaccccc 4140
agaccggatc aggcaagctg gccctcatgt ccccttcacg gtgtttgagg aagtctgccc 4200
tacagagagc aagcctgaag taccccaggc ccccagggta caacagagag tccagacgcg 4260
cctcaaggtg aacttcagtg atgacagtga cttggaagac cctgtctcag ctgaggcctg 4320
gctggcagag gagcctaaga gacggggcac tgcttcccgg ggccgggggc gagcaaggaa 4380
gggcctgagc ctaaagacgg atgccgtggt tgccccaggt agtgcccctg ggaaccctgg 4440
cctgaatggc aggagccgga gggccaagaa ggtggcatca agacattgtg aggagcggcg 4500
tccccagagg gccagtgacc aggccaggcc tggccctgag atcatgagga ccatccctga 4560
ggaagaactg actgacaact ggagaaaaat gagctttgag atcctcaggg gctctgacgg 4620
ggaagactca gcctcaggtg ggaagactcc agctccgggc cctgaggcag cttctggaga 4680
atgggagctg ctgaggctgg attccagcaa gaagaagctg cccagcccat gcccagacaa 4740
ggagagtgac aaggaccttg gtcctcggct ccggctcccc tcagcccccg tagccactgg 4800
tctttctacc ctggactcca tctgtgactc cctgagtgtt gctttccggg gcattagtca 4860
ctgtcctcct agtgggctct atgcccacct ctgccgcttc ctggccttgt gcctgggcca 4920
ccgggatcct tatgccactg ctttccttgt caccgagtct gtctccatca cctgtcgcca 4980
ccagctgctc acccacctcc acagacagct cagcaaggcc cagaagcacc gaggatcact 5040
tgaaatagca gaccagctgc aggggctgag ccttcaggag atgcctggag atgtccccct 5100
ggcccgcatc cagcgcctct tttccttcag ggctttggaa tctggccact tcccccagcc 5160
tgaaaaggag agtttccagg agcgcctggc tctgatcccc agtggggtga ctgtgtgtgt 5220
gttggccctg gccaccctcc agcccggaac cgtgggcaac accctcctgc tgacccggct 5280
ggaaaaggac agtcccccag tcagtgtgca gattcccact ggccagaaca agcttcatct 5340
gcgttcagtc ctgaatgagt ttgatgccat ccagaaggca cagaaagaga acagcagctg 5400
tactgacaag cgagaatggt ggacagggcg gctggcactg gaccacagga tggaggttct 5460

catcgcttcc ctagagaagt ctgtgctggg ctgctggaag gggctgctgc tgccgtccag 5520
tgaggagccc ggccctgccc aggaggcctc ccgcctacag gagctgctac aggactgtgg 5580
ctggaaatat cctgaccgca ctctgctgaa aatcatgctc agtggtgccg gtgccctcac 5640
ccctcaggac attcaggccc tggcctacgg gctgtgccca acccagccag agcgagccca 5700
ggagctcctg aatgaggcag taggacgtct acagggcctg acagtaccaa gcaatagcca 5760
ccttgtcttg gtcctagaca aggacttgca gaagctgccg tgggaaagca tgcccagcct 5820
ccaagcactg cctgtcaccc ggctgccctc cttccgcttc ctactcagct actccatcat 5880
caaagagtat ggggcctcgc cagtgctgag tcaaggggtg gatccacgaa gtaccttcta 5940
tgtcctgaac cctcacaata acctgtcaag cacagaggag caatttcgag ccaatttcag 6000
cagtgaagct ggctggagag gagtggttgg ggaggtgcca agacctgaac aggtgcagga 6060
agccctgaca aagcatgatt tgtatatcta tgcagggcat ggggctggtg cccgcttcct 6120
tgatgggcag gctgtcctgc ggctgagctg tcgggcagtg gccctgctgt ttggctgtag 6180
cagtgcggcc ctggctgtgc gtggaaacct ggagggggct ggcatcgtgc tcaagtacat 6240
catggctggt tgccccttgt ttctgggtaa tctctgggat gtgactgacc gcgacattga 6300
ccgctacacg gaagctctgc tgcaaggctg gcttggagca ggcccagggg ccccccttct 6360
ctactatgta aaccaggccc gccaagctcc ccgactcaag tatcttattg gggctgcacc 6420
tatagcctat ggcttgcctg tctctctgcg gtaaccccat ggagctgtct tattgatgct 6480
agaagcctca taactgttct acctccaagg ttagatttaa tccttaggat aactctttta 6540
aagtgatttt ccccagtgtt ttatatgaaa catttccttt tgatttaacc tcagtataat 6600
aaagatacat catttaaacc ctgaaaaaaa aaaaaaaaaa a 6641

<210> 111
<211> 2080
<212> DNA
<213> human

<400> 111
gcggccgctg ggctcaggct gtccgctctg ggctccgcgg cctcggcccc gctgcactcc 60
acctccgccc cctcggactc cctcccctct gcttctactc ctcctgctcc agtgcggatc 120
gtttcgcaac tgcttgccac tcgtcccgtg cctggctgtt tttccatttc ccggccccct 180
cttcttgagt actttacccc ctgcatttgg ggacagggac tggaaaaggg gcgggtggag 240
cgtccagtgg agaagaagga agcgaggccc gcaggaggag gaggatcggc ggactgtggg 300
gaggagaccc cacgccaccc tttctggtca tctcccctcc cgccccgccc ctgcgcacac 360
tccctcgcgg gcgagctact ttcggaccag gaaagtaaga gcggccctgg gtgacagcgc 420
cgcggggcca gtcccggggt tagccgcgcg tctgctcgct tctggtccgt cgcgctccca 480
gccagggcac agcccggacc gaggatggct tcgaccacaa cctgcaccag gttcacggac 540
gagtatcagc ttttcgagga gcttggaaag ggggcattct cagtggtgag aagatgtatg 600

aaaattccta ctggacaaga atatgctgcc aaaattatca acaccaaaaa gctttctgct 660
agggatcatc agaaactaga aagagaagct agaatctgcc gtcttttgaa gcaccctaat 720
attgtgcgac ttcatgatag catatcagaa gagggctttc actacttggt gtttgattta 780
gttactggag gtgaactgtt tgaagacata gtggcaagag aatactacag tgaagctgat 840
gccagtcatt gtatacagca gattctagaa agtgttaatc attgtcacct aaatggcata 900
gttcacaggg acctgaagcc tgagaatttg cttttagcta gcaaatccaa gggagcagct 960
gtgaaattgg cagactttgg cttagccata gaagttcaag gggaccagca ggcgtggttt 1020
ggttttgctg gcacacctgg atatctttct ccagaagttt tacgtaaaga tccttatgga 1080
aagccagtgg atatgtgggc atgtggtgtc attctctata ttctacttgt ggggtatcca 1140
cccttctggg atgaagacca acacagactc tatcagcaga tcaaggctgg agcttatgat 1200
tttccatcac cagaatggga cacggtgact cctgaagcca aagacctcat caataaaatg 1260
cttactatca accctgccaa acgcatcaca gcctcagagg cactgaagca cccatggatc 1320
tgtcaacgtt ctactgttgc ttccatgatg cacagacagg agactgtaga ctgcttgaag 1380
aaatttaatg ctagaagaaa actaaagggt gccatcttga caactatgct ggctacaagg 1440
aatttctcag cagccaagag tttgttgaag aaaccagatg gagtaaagga gtcaactgag 1500
agttcaaata caacaattga ggatgaagat gtgaaagcac gaaagcaaga gattatcaaa 1560
gtcactgaac aactgatcga agctatcaac aatggggact ttgaagccta cacaaaaatc 1620
tgtgacccag gccttactgc ttttgaacct gaagctttgg gtaatttagt ggaagggatg 1680
gattttcacc gattctactt tgaaaatgct ttgtccaaaa gcaataaacc aatccacact 1740
attattctaa accctcatgt acatctggta ggggatgatg ccgcctgcat agcatatatt 1800
aggctcacac agtacatgga tggcagtgga atgccaaaga caatgcagtc agaagagact 1860
cgtgtgtggc accgccggga tggaaagtgg cagaatgttc attttcatcg ctcggggtca 1920
ccaacagtac ccatcaagcc accctgtatt ccaaatggga aagaaaactt ctcaggaggc 1980
acctctttgt ggcaaaacat ctaaggcctg aaaaccattc acatatgggt cttctaaatt 2040
tcaacagtgc cacttctgca ttctctgttc tcaaggcacc 2080

<210> 112
<211> 11611
<212> DNA
<213> human

<400> 112
ggcttcagcc ctgcagggaa agaaaagtgc aatgattctg gactgagacg cgcttgggca 60
gaggctatgt aatcgtgtct gtgttgagga cttcgcttcg aggagggaag aggagggatc 120
ggctcgctcc tccggcggcg gcggcggcgg cgactctgca ggcggagttt cgcggcggcg 180
gcaccagggt tacgccagcc ccgcggggag gtctctccat ccagcttctg cagcggcgaa 240
agccccagcg cccgagcgcc tgagccggcg gggagcaagt aaagctagac cgatctccgg 300
ggagccccgg agtaggcgag cggcggccgc cagctagttg agcgcacccc ccgcccgccc 360

cagcggcgcc gcggcgggcg gcgtccaggc ggcatggaga aggacggcct gtgccgcgct 420
gaccagcagt acgaatgcgt ggcggagatc ggggagggcg cctatgggaa ggtgttcaag 480
gcccgcgact tgaagaacgg aggccgtttc gtggcgttga agcgcgtgcg ggtgcagacc 540
ggcgaggagg gcatgccgct ctccaccatc cgcgaggtgg cggtgctgag gcacctggag 600
accttcgagc accccaacgt ggtcaggttg tttgatgtgt gcacagtgtc acgaacagac 660
agagaaacca aactaacttt agtgtttgaa catgtcgatc aagacttgac cacttacttg 720
gataaagttc cagagcctgg agtgcccact gaaaccataa aggatatgat gtttcagctt 780
ctccgaggtc tggactttct tcattcacac cgagtagtgc atcgcgatct aaaaccacag 840
aacattctgg tgaccagcag cggacaaata aaactcgctg acttcggcct tgcccgcatc 900
tatagtttcc agatggctct aacctcagtg gtcgtcacgc tgtggtacag agcacccgaa 960
gtcttgctcc agtccagcta cgccaccccc gtggatctct ggagtgttgg ctgcatattt 1020
gcagaaatgt ttcgtagaaa gcctcttttt cgtggaagtt cagatgttga tcaactagga 1080
aaaatcttgg acgtgattgg actcccagga gaagaagact ggcctagaga tgttgccctt 1140
cccaggcagg cttttcattc aaaatctgcc caaccaattg agaagtttgt aacagatatc 1200
gatgaactag gcaaagacct acttctgaag tgtttgacat ttaacccagc caaaagaata 1260
tctgcctaca gtgccctgtc tcacccatac ttccaggacc tggaaaggtg caaagaaaac 1320
ctggattccc acctgccgcc cagccagaac acctcggagc tgaatacagc ctgaggcctc 1380
agcagccgcc ttaagctgat cctgcggaga acacccttgg tggcttatgg gtccccctca 1440
gcaagcccta cagagctgtg gaggattgct atctggaggc cttccagctg ctgtcttctg 1500
gacaggctct gcttctccaa ggaaaccgcc tagtttactg ttttgaaatc aatgcaagag 1560
tgattgcagc tttatgttca tttgtttgtt tgtttgtctg tttgtttcaa gaacctggaa 1620
aaattccaga agaagagaag ctgctgacca attgtgctgc catttgattt ttctaacctt 1680
gaatgctgcc agtgtggagt gggtaatcca ggcacagctg agttatgatg taatctctct 1740
gcagctgccg ggcctgattt ggtacttttg agtgtgtgtg tgcatgtgtg tgtgtgtgtg 1800
tgtgtgtgtg tgtgtgtatg tgagagattc tgtgatcttt taaagtgtta ctttttgtaa 1860
acgacaagaa taattcaatt ttaaagactc aaggtggtca gtaaataaca ggcatttgtt 1920
cactgaaggt gattcaccaa aatagtcttc tcaaattaga aagttaaccc catgtcctca 1980
gcatttcttt tctggccaaa agcagtaaat ttgctagcag taaaagatga agttttatac 2040
acacagcaaa aaggagaaaa aattctagta tattttaaga gatgtgcatg cattctattt 2100
agtcttcaga atgctgaatt tacttgttgt aagtctattt taaccttctg tatgacatca 2160
tgctttatca tttcttttgg aaaatagcct gtaagctttt tattacttgc tataggttta 2220
gggagtgtac ctcagataga ttttaaaaaa aagaatagaa agcctttatt tcctggtttg 2280
aaattccttt cttccctttt tttgttgttg ttattgttgt ttgttgttgt tattttgttt 2340
ttgtttttag gaatttgtca gaaactcttt cctgttttgg tttggagagt agttctctct 2400

aactagagac aggagtggcc ttgaaatttt cctcatctat tacactgtac tttctgccac 2460
acactgcctt gttggcaaag tatccatctt gtctatctcc cggcacttct gaaatatatt 2520
gctaccattg tataactaat aacagattgc ttaagctgtt cccatgcacc acctgtttgc 2580
ttgctttcaa tgaacctttc ataaattcgc agtctcagct tatggtttat ggcctcgatt 2640
ctgcaaacct aacagggtca catatgttct ctaatgcagt ccttctacct ggtgtttact 2700
tttgctaccc aaataatgag taggatcttg ttttcgtata cccccaccac tcccattgct 2760
accaactgtc accttgtgca ctcctttttt atagaagata ttttcagtgt ctttacctga 2820
gggtatgtct ttagctatgt tttagggcca tacatttact ctatcaaatg atcttttctc 2880
catcccccag gctgtgctta tttctagtgc cttgtgctca ctcctgctct ctacagagcc 2940
agcctggcct gggcattgta aacagctttt cctttttctc ttactgtttt ctctacagtc 3000
ctttatattt cataccatct ctgccttata agtggtttag tgctcagttg gctctagtaa 3060
ccagaggaca cagaaagtat cttttggaaa gtttagccac ctgtgctttc tgactcagag 3120
tgcatgcaac agttagatca tgcaacagtt agattatgtt tagggttagg attttcaaag 3180
aatggaggtt gctgcactca gaaaataatt cagatcatgt ttatgcatta ttaagttgta 3240
ctgaattctt tgcagcttaa tgtgatatat gactatcttg aacaagagaa aaaactagga 3300
gatgtttctc ctgaagagct tttggggttg ggaactattc ttttttaatt gctgtactac 3360
ttaacattgt tctaattcag tagcttgagg aacaggaaca ttgttttcta gagcaagata 3420
ataaaggaga tgggccatac aaatgttttc tactttcgtt gtgacaacat tgattaggtg 3480
ttgtcagtac tataaatgct tgagatataa tgaatccaca gcattcaagg tcaggtctac 3540
tcaaagtctc acatggaaaa gtgagttctg cctttccttt gatcgagggt caaaatacaa 3600
agacattttt gctagggcct acaaattgaa tttaaaaact cactgcactg attcatctga 3660
gctttttggt tagtattcat ggctagagtg aacatagctt tagtttttgc tgttgtaaaa 3720
gtgttttcat aagttcactc aagaaaaatg cagctgttct gaactggaat ttttcagcat 3780
tctttagaat tttaaatgag tagagagctc aactttattt cctagcatct gcttttgact 3840
catttctagg cagtgcttat gaagaaaaat taaagcacaa acattctggc attcaatcgt 3900
tggcagatta tcttctgatg acacagaatg aaagggcatc tcagcctctc tgaactttgt 3960
aaaaatctgt ccccagttct tccatcggtg tagttgttgc atttgagtga atactctctt 4020
gatttatgta ttttatgtcc agattcgcca tttctgaaat ccagatccaa cacaagcagt 4080
cttgccgtta gggcattttg aagcagatag tagagtaaga acttagtgac tacagcttat 4140
tcttctgtaa catatggttt caaacatctt tgccaaaagc taagcagtgg tgaactgaaa 4200
agggcatatt gccccaaggt tacactgaag cagctcatag caagttaaaa tattgtgaca 4260
gatttgaaat catgtttgaa tttcatagta ggaccagtac aagaatgtcc ctgctagttt 4320
ctgtttgatg tttggttctg gcggctcagg cattttggga actgttgcac agggtgcagt 4380
caaaacaacc tacatataaa aattacataa aagaaccttg tccatttagc tttcataaga 4440

aatcccatgg caaagagtaa taaaaaggac ctaatcttaa aaatacaatt tctaagcact 4500
tgtaagaacc cagtgggttg gagcctccca ctttgtccct cctttgaagt ggatgggaac 4560
tcaaggtgca aagaacctgt tttggaagaa agcttggggc catttcagcc ccctgtattc 4620
tcatgatttt ctctcaggaa gcacacactg tgaatggcag acttttcatt tagccccagg 4680
tgacttacta aaaatagttg aaaattattc acctaagaat agaatctcag cattgtgtta 4740
aataaaaatg aaagctttag aaggcatgag atgttcctat cttaaataaa gcatgtttct 4800
tttctataga gaaatgtata gtttgactct ccagaatgta ctatccatct tgatgagaaa 4860
actcttaaat agtaccaaac attttgaact ttaaattatg tatttaaagt gagtgtttaa 4920
gaaactgtag ctgcttcttt tacaagtggt gcctattaaa gtcagtaatg gccattattg 4980
ttccattgtg gaaattaaat tatgtaagct tcctaatatc ataaacatat taaaattctt 5040
ctaaaatatt gcttttcttt taagtgacaa tttgactatt cttatgataa gcacatgaga 5100
gtgtcttaca ttttccaaaa gcaggcttta attgcatagt tgagtctagg aaaaaataat 5160
gttaaaagtg aatatgccac cataattact taattatgtt agtatagaaa ctacagaata 5220
tttaccctgg aaagaaaata ttggaatgtt attataaact cttagatatt tatataattc 5280
aaaagaatgc atgtttcaca ttgtgacaga taaagatgta tgatttctaa ggctttaaaa 5340
attattcata aaacagtggg caatagataa aggaaattct ggagaaaatg aaggtattta 5400
aagggtagtt tcaaagctat atatattttg aaggatatat tctttatgaa caaatatatt 5460
gtaaaaattt atactaaggt catctggtaa ctgtgggatt aatatggtcg aaaacaaatg 5520
ttatggagaa gctgtcccaa gcaaactaaa ttacctgtac ttttttccca tttcaaggga 5580
agaggcaacc acatgaagca atacttctta cacatgccta agaacgttca ttgaaaaaat 5640
aaatttttaa aaggcatgtg tttcctatgc caccaatact tttgaaaaat tgtgaacctt 5700
acccaaaacc atttatcatg tccattaagt atatttgggt atataattag gaagatattt 5760
acatgttcca tctccacagt ggaaaaactt attgaggcta ccaaagtgtg ccaagaaatg 5820
taagtcctta gagtaattag aaatgctgtt ttcctcaaaa gcatgagaaa ctagcatttt 5880
catttcttat ttactccctt tctatatcaa tgcaattcac aacccaattt taatacatcc 5940
ctatatctca agcatttcta tcttgtactt tttcagaaaa taaaccaaaa ataatccttt 6000
ggtctctcta tcttctgacc tttgtaagca acagaaatgt aaaaacagaa ggggtccaat 6060
ttttacacgt ttttttctca agtagccttt ctggggattt ttattttctt aatgaagtgc 6120
caatcagctt ttcaaaatgt tttctatttc tcagcatttc caggaagtga taacgtttag 6180
ctaaatgagt agaagtggac ttccttcaac atattgttac cttgtctagc cttaggaaga 6240
aaacaagagc cacctgaaaa taaatacagg ctcttttcga gcatctgctg aaatactgtt 6300
acagcaattt gaagttgatg tggtaggaaa ggaaggtgac ttttcttgca aaagtctttc 6360
taaacattca cactgtccta agagatgagc tttcttgttt tattccggta tattccacaa 6420
ggtggcactt ttagagaaaa acaaatctga tgaagactaa agaggtactt ctaaaagaga 6480

tttcattcta actttatttt tctgcgcata tttaactctt tcctagcact tgttttttgg 6540
gatgattaat agtctctata atgttctgta acttcaatat tttacttgtt acctaggttc 6600
tgaacaattg tctgcaaata aattgttctt aaggatggat aatacaccca ttttgatcat 6660
ttaagtaaag aaagcctagt cattcattca gtcaagaaaa aatttttgaa gtacccagtt 6720
accttacttt tctagattaa aacaggctta gttactaaaa aggcagtcct catctgtgaa 6780
caggatagtt tcgttagaag tataaaactc ctttagtggc cccagttaaa acacacatac 6840
cctctctgct gctttcaaat tccctagcat ggtggccttt caacattgat taaattttaa 6900
aatcctaatt taaagatcag gtgagcaaaa tgagtagcac atcagtaatt cagtagacaa 6960
aacttttgtc tgaaaaattg ctgtattgaa acagagccct aaaataccaa aagaccaggt 7020
aattttaaca tttgtggaat cacaaatgta aattcataag aagctctaat taaaaaaaaa 7080
aagtctgaag tatatgagca taacaactta ggagtgtgtc tacatactta acttttgaag 7140
ttttttggca actttatata ctttttttaa atttacaagt ctacttaaag acttcttata 7200
ccccaaatga ttaagttaat tttagaggtc acctttctca cagcagtgtc acttgaaatt 7260
tagtagggaa ggatattgca gtatttttca gtttccttag cacagcacca cagaaagcag 7320
cttattcctt ttgagtggca gacactcgac ggtgcctgcc caactttcct cctgagtggc 7380
aagcagatga gtctcagtaa ttcatactga accaaaatgc cacatacact aggggcagtc 7440
agaaactggc tgagaaatcc cccgcctcat tcgcccctct gctcccagga actagagtcc 7500
agttaaagcc cctatgcgaa aggccgaatt ccaccccagg gtttgttata acagtggcca 7560
gtctgaaccc catttgctcg tgctcaaaac ttgattccca cttgaaagcc ttccgggcgc 7620
gctgcctcgt tgccccgccc ctttggcagg agagaggcag tgggcgaggc cgggctgggg 7680
ccccgcctcc cactcacctg ccggtgcctg aaattatgtg cggccccgcg ggctgctttc 7740
cgaggtcaga gtgccctgct gctgtctcag aggcatctgt tctgcaaatc ttaggaagaa 7800
aaatgtccct agtagcaaac gggtgtcttc tgtgcataaa taagtacaac acaattctcc 7860
gaaagttcgg gtaaaaagag atgcggtagc agctgccctg tgtgaagctg tctaccccgc 7920
atctctcagg cgctaagctc agtttttgtt tttgtttttg tttttttaaa gaaaagatgt 7980
ataattgcag gaattttttt ttattttttt attttccatc attctatata tgtgatggtg 8040
aaagatatgc ctggaaaagt tttgttttga aaagtttatt ttctgcttcg tcttcagttg 8100
gcaaaagctc tcaattcttt agcttccagt ttcttttctc tctttttctt tgttaggtaa 8160
ttaaaggtat gtaaacaaat tatctcatgt agcaggggat tttcatgttg agaggaatct 8220
tccgtgtgag ttgtttggtc acacaaataa ccctttctca attttaggag tttggattgt 8280
caaatgtagg tttttctcaa agggggcata taactacata ttgactgcca agaactatga 8340
ctgtagcact aatcagcaca catagagcca cacaattatt taatttctaa ctctctgtgg 8400
tccctagaaa aattccgttg atgtgcttag gttaaagttc tgaagatacc cgttgtaccc 8460
ttacttgaaa gtttctaatc ttaagtttta tgaaatgcaa taatatgtat cagctagcaa 8520

tatttctgtg atcaccaaca actctcagtt tgatcttaaa gtctgaataa taaaacaaat 8580
cccagcagta atacatttct taaacctcac agtgcatgat atatcttttc attctgatcc 8640
tgtgtttgca aaaatataca catgtatatc atagttcctc acttttattt catttgtttt 8700
cctattacct gtagtaaata tattagttag tacatggaat ttatagcatc agctaccccc 8760
aggaacagca cctgacaggc gggggatttt ttttcaagtt gttctacatt tgcataaatt 8820
atttctatta ttattcatgt atgttattta tttctgaatc acactagtcc tgtgaaagta 8880
caactgaagg cagaaagtgt taggattttg catctaatgt tcattatcat ggtattgatg 8940
gacctaagaa aataaaaatt agactaagcc cccaaataag ctgcatgcat ttgtaacatg 9000
attagtagat ttgaatatat agatgtagta ttttgggtat ctaggtgttt tatcattatg 9060
taaaggaatt aaagtaaagg actttgtagt tgtttttatt aaatatgcat atagtagagt 9120
gcaaaaatat agcaaaaata aaaactaaag gtagaaaagc attttagata tgccttaatt 9180
tagaaactgt gccaggtggc cctcggaata gatgccaggc agagaccagt gcctgggtgg 9240
tgcctcctct tgtctgccct catgaagaag cttccctcac gtgatgtagt gccctcgtag 9300
gtgtcatgtg gagtagtggg aacaggcagt actgttgaga ggagagcagt gtgagagttt 9360
ttctgtagaa gcagaactgt cagcttgtgc cttgaggctt ccagaacgtg tcagatggag 9420
aagtccaagt ttccatgctt caggcaactt agctgtgtac agaagcaatc cagtgtggta 9480
ataaaaagca aggattgcct gtataattta ttataaaata aaagggattt taacaaccaa 9540
caattcccaa cacctcaaaa gcttgttgca ttttttggta tttgaggttt ttatctgaag 9600
gttaaagggc aagtgtttgg tatagaagag cagtatgtgt taagaaaaga aaaatattgg 9660
ttcacgtaga gtgcaaatta gaactagaaa gttttatacg attatcattt tgagatgtgt 9720
taaagtaggt tttcactgta aaatgtatta gtgtttctgc attgccatag ggcctggtta 9780
aaactttctc ttaggtttca ggaagactgt cacatacagt aagctttttt ccttctgact 9840
tataatagaa aatgttttga aagtaaaaaa aaaaaatcta atttggaaat ttgacttgtt 9900
agtttctgtg tttgaaatca tggttctaga aatgtagaaa ttgtgtatat cagatactca 9960
tctaggctgt gtgaaccagc ccaagatgac caacatcccc acacctctac atctctgtcc 10020
cctgtatctc ttcctttcta ccactaaagt gttccctgct accatcctgg cttgtccaca 10080
tggtgctctc catcttcctc cacatcatgg accacaggtg tgcctgtcta ggcctggcca 10140
ccactcccaa cttgacctag ccacattcat ctagagatgg ttcctgatgc tgggcacaga 10200
ctgtgctcat ggcacccatt agaaatgcct ctagcatctt tgtatgcatc ttgattttta 10260
aaccaagtca ttgtacagag cattcagttt tggctgtggt accaagagaa aaactaatca 10320
agaatataaa ccacattcca ggctgctgtt ttctctccat ctacaggcca cacttttact 10380
gtatttcttc atacttgaaa ttcattctgc tattttcata tcagggtaca gacttataag 10440
ggtgcatgtt ccttaaaggt gcataattat tcttattccg tttgcttata ttgctacaga 10500
atgctctgtt ttggtgcttt gagttctgca gacccaagaa gcagtgtgga aattcactgc 10560

ctgggacaca gtcttataag aatgttggca ggtgactttg tatcagatgt tgcttctctt 10620
ttctctgtac acagattgag agttaccaca gtggcctgtc gggtccaccc tgtgggtgca 10680
gcacagctct ctgaaagcaa gaaccttcct acctattcta acgtttttgc cctctaagaa 10740
aaatggcctc aggtatggta tagacatagc aagaggggaa gggctgtctc actctagcaa 10800
ccatccctcc attacacaca gaaagccctc ttgaagcaaa agaagaagaa agaaagaaag 10860
cttatctcta aggctactgt cttcagaatg ctctgagctg aatgctcttg ctcctttccc 10920
aagaggcaga tgaaaatata gccagtttat ctataccctt cctatctgag gaggagaata 10980
gaaaagtagg gtaaatatgt aacgtaaaat atgtcattca aggaccacca aaactttaag 11040
taccctatca ttaaaaatct ggttttaaaa gtagctcaag taagggatgc tttgtgaccc 11100
agggtttctg aagtcagata gccattctta cctgcccctt actctgactt attgggaaag 11160
ggagaactgc agtggtgttt ctgttgcagt ggcaaaggta acatgtcaga aaattcagag 11220
ggttgcatac caataatcct ttggaaactg gatgtcttac tgggtgctag aatgaaaatg 11280
taggtattta ttgtcagatg atgaagttca ttgttttttt caaaattggt gttgaaatat 11340
cactgtccaa tgtgttcact tatgtgaaag ctaaattgaa tgaggcaaaa agagcaaata 11400
gtttgtatat ttgtaatacc ttttgtattt cttacaataa aaatattggt agcaaataaa 11460
aataataaaa acaataactt taaactgctt tctggagatg aattactctc ctggctattt 11520
tcttttttac tttaatgtaa aatgagtata actgtagtga gtaaaattca ttaaattcca 11580
agttttagca gaaaaaaaaa aaaaaaaaaa a 11611

<210> 113
<211> 3317
<212> DNA
<213> human

<400> 113
agttctcgcg ggacaccgac ggggagcgga agccaggagg tattgctgct tcggcgaccg 60
ggcggcggca gcggcggcgg cggctgtggc agagtctgtg cctgtggcgg tgacggcggc 120
gggagcaagc gctgccctcg cagagcagcc ttggggtcgc cggccgctcg cagcgttgtg 180
gaggggcggg ccggacgctg agcggagcag ctgcgccacg ggtggcattg tgtgtcccag 240
agtgccggag cgagtcccag aagagaggcg aggctaagcc cagagcgctg ggttgcttca 300
gcagggaaga ctcccttccc cctgcttcag gctgctgagc actgagcagc gctcagaatg 360
gaagccatcg ccaaatatga cttcaaagct actgcagacg acgagctgag cttcaaaagg 420
ggggacatcc tcaaggtttt gaacgaagaa tgtgatcaga actggtacaa ggcagagctt 480
aatggaaaag acggcttcat tcccaagaac tacatagaaa tgaaaccaca tccgtggttt 540
tttggcaaaa tccccagagc caaggcagaa gaaatgctta gcaaacagcg gcacgatggg 600
gcctttctta tccgagagag tgagagcgct cctggggact tctccctctc tgtcaagttt 660
ggaaacgatg tgcagcactt caaggtgctc cgagatggag ccgggaagta cttcctctgg 720

gtggtgaagt tcaattcttt gaatgagctg gtggattatc acagatctac atctgtctcc 780
agaaaccagc agatattcct gcgggacata gaacaggtgc cacagcagcc gacatacgtc 840
caggccctct ttgactttga tccccaggag gatggagagc tgggcttccg ccggggagat 900
tttatccatg tcatggataa ctcagacccc aactggtgga aaggagcttg ccacgggcag 960
accggcatgt ttccccgcaa ttatgtcacc cccgtgaacc ggaacgtcta agagtcaaga 1020
agcaattatt taaagaaagt gaaaaatgta aaacacatac aaaagaatta aacccacaag 1080
ctgcctctga cagcagcctg tgagggagtg cagaacacct ggccgggtca ccctgtgacc 1140
ctctcacttt ggttggaact ttagggggtg ggagggggcg ttggatttaa aaatgccaaa 1200
acttacctat aaattaagaa gagtttttat tacaaatttt cactgctgct cctctttccc 1260
ctcctttgtc ttttttttca tccttttttc tcttctgtcc atcagtgcat gacgtttaag 1320
gccacgtata gtcctagctg acgccaataa taaaaaacaa gaaaccaagt gggctggtat 1380
tctctctatg caaaatgtct gttttagttg gaatgactga aagaagaaca gctgttcctg 1440
tgttcttcgt atatacacac aaaaaggagc gggcagggcc gctcgatgcc tttgctgttt 1500
agcttcctcc agaggagggg acttgtagga atctgccttc cagcccagac ccccagtgta 1560
ttttgtccaa gttcacagta gagtagggta gaaggaaagc atgtctctgc ttccatggct 1620
tcctgagaaa gcccacctgg gctgggcgcg gtggctcacg cctgtaatcc cagcactttg 1680
ggaggccaag gtgggcggat cacaaggtca ggagttcgag accaacctag ccaacatggt 1740
gaaaccccgt ctctactaaa aataagaaat tagccgggtg tggcacgcac ctgtagtccc 1800
agctacttgg gagcctgagg caggagaatc gcttgaacct gggaagtgga ggttgagtga 1860
gccgggaccg tgccattgta ctccagcctg ggtgacagag cgagattccg tctcaaaaaa 1920
aaaaaaaaaa agcccacctg aaagcctgtc tctttccact ttgttggccc ttccagtggg 1980
attatcgagc atgttgtttt ttcatagtgc ctttttcctt atttcaaggg ttgcttctga 2040
gtggtgtttt tttttttttt ttaatttgtt ttgttttaaa ataagttaaa ggcagtccag 2100
agcttttcag ccaatttgtc tcctactctg tgtaaatatt tttccctccg ggcaggggag 2160
ccagggtaga gcaaaggaga caaagcagga gtggaaggtg aggcgttctc ctgcttgtac 2220
taagccagga ggctttaagc tccagcttta agggttgtga gccccttggg ggttcaggga 2280
actgcttgcc cagggtgcag tgtgagtgtg atgggccacc ggggcaagag ggaaggtgac 2340
cgcccagctc tcccacatcc cactggatct ggcttacagg ggggtcggaa gcctgtcctc 2400
accgtctcgg gggttgtggc ccccgccccc tccctatatg cacccctgga accagcaagt 2460
cccagacaag gagagcggag gaggaagtca tgggaacgca gcctccagtt gtagcaggtt 2520
tcactattcc tatgctgggg tacacagtga gagtactcac ttttcacttg tcttgctctt 2580
agattgggcc atggctttca tcctgtgtcc cctgacctgt ccaggtgagt gtgagggcag 2640
cactgggaag ctggagtgct gcttgtgcct cccttcccag tgggctgtgt tgactgctgc 2700
tccccacccc taccgatggt cccaggaagc agggagagtt ggggaaggca agattggaaa 2760

gacaggaaga ccaaggcctc ggcagaactc tctgtcttct ctccacttct ggtcccctgt 2820
ggtgatgtgc ctgtaatctt tttctccacc caaacccctt cccacgacaa aaacaagact 2880
gcctccctct cttccgggag ctggtgacag ccttgggcct ttcagtccca aagcggccga 2940
tgggagtctc cctccgactc cagatatgaa cagggcccag gcctggagcg tttgctgtgc 3000
caggaggcgg cagctcttct gggcagagcc tgtccccgcc ttccctcact cttcctcatc 3060
ctgcttctct tttcctcgca gatgataaaa ggaatctggc attctacacc tggaccattt 3120
gattgtttta ttttggaatt ggtgtatatc atgaagcctt gctgaactaa gttttgtgtg 3180
tatatattta aaaaaaaaat cagtgtttaa ataaagacct atgtacttaa tcctttaact 3240
ctgcggatag catttggtag gtagtgatta actgtgaata ataaatacac aatgaattct 3300
tcaaaaaaaa aaaaaaa 3317

<210> 114
<211> 4772
<212> DNA
<213> human

<400> 114
gctcagttgc cgggcggggg agggcgcgtc cggtttttct caggggacgt tgaaattatt 60
tttgtaacgg gagtcgggag aggacggggc gtgccccgac gtgcgcgcgc gtcgtcctcc 120
ccggcgctcc tccacagctc gctggctccc gccgcggaaa ggcgtcatgc cgcccaaaac 180
cccccgaaaa acggccgcca ccgccgccgc tgccgccgcg gaacccccgg caccgccgcc 240
gccgccccct cctgaggagg acccagagca ggacagcggc ccggaggacc tgcctctcgt 300
caggcttgag tttgaagaaa cagaagaacc tgattttact gcattatgtc agaaattaaa 360
gataccagat catgtcagag agagagcttg gttaacttgg gagaaagttt catctgtgga 420
tggagtattg ggaggttata ttcaaaagaa aaaggaactg tggggaatct gtatctttat 480
tgcagcagtt gacctagatg agatgtcgtt cacttttact gagctacaga aaaacataga 540
aatcagtgtc cataaattct ttaacttact aaaagaaatt gataccagta ccaaagttga 600
taatgctatg tcaagactgt tgaagaagta tgatgtattg tttgcactct tcagcaaatt 660
ggaaaggaca tgtgaactta tatatttgac acaacccagc agttcgatat ctactgaaat 720
aaattctgca ttggtgctaa aagtttcttg gatcacattt ttattagcta aaggggaagt 780
attacaaatg gaagatgatc tggtgatttc atttcagtta atgctatgtg tccttgacta 840
ttttattaaa ctctcacctc ccatgttgct caaagaacca tataaaacag ctgttatacc 900
cattaatggt tcacctcgaa cacccaggcg aggtcagaac aggagtgcac ggatagcaaa 960
acaactagaa aatgatacaa gaattattga agttctctgt aaagaacatg aatgtaatat 1020
agatgaggtg aaaaatgttt atttcaaaaa ttttatacct tttatgaatt ctcttggact 1080
tgtaacatct aatggacttc cagaggttga aaatctttct aaacgatacg aagaaattta 1140
tcttaaaaat aaagatctag atgcaagatt atttttggat catgataaaa ctcttcagac 1200
tgattctata gacagttttg aaacacagag aacaccacga aaaagtaacc ttgatgaaga 1260

ggtgaatgta attcctccac acactccagt taggactgtt atgaacacta tccaacaatt 1320
aatgatgatt ttaaattcag caagtgatca accttcagaa aatctgattt cctattttaa 1380
caactgcaca gtgaatccaa aagaaagtat actgaaaaga gtgaaggata taggatacat 1440
ctttaaagag aaatttgcta aagctgtggg acagggttgt gtcgaaattg gatcacagcg 1500
atacaaactt ggagttcgct tgtattaccg agtaatggaa tccatgctta aatcagaaga 1560
agaacgatta tccattcaaa attttagcaa acttctgaat gacaacattt ttcatatgtc 1620
tttattggcg tgcgctcttg aggttgtaat ggccacatat agcagaagta catctcagaa 1680
tcttgattct ggaacagatt tgtctttccc atggattctg aatgtgctta atttaaaagc 1740
ctttgatttt tacaaagtga tcgaaagttt tatcaaagca gaaggcaact tgacaagaga 1800
aatgataaaa catttagaac gatgtgaaca tcgaatcatg gaatcccttg catggctctc 1860
agattcacct ttatttgatc ttattaaaca atcaaaggac cgagaaggac caactgatca 1920
ccttgaatct gcttgtcctc ttaatcttcc tctccagaat aatcacactg cagcagatat 1980
gtatctttct cctgtaagat ctccaaagaa aaaaggttca actacgcgtg taaattctac 2040
tgcaaatgca gagacacaag caacctcagc cttccagacc cagaagccat tgaaatctac 2100
ctctctttca ctgttttata aaaaagtgta tcggctagcc tatctccggc taaatacact 2160
ttgtgaacgc cttctgtctg agcacccaga attagaacat atcatctgga cccttttcca 2220
gcacaccctg cagaatgagt atgaactcat gagagacagg catttggacc aaattatgat 2280
gtgttccatg tatggcatat gcaaagtgaa gaatatagac cttaaattca aaatcattgt 2340
aacagcatac aaggatcttc ctcatgctgt tcaggagaca ttcaaacgtg ttttgatcaa 2400
agaagaggag tatgattcta ttatagtatt ctataactcg gtcttcatgc agagactgaa 2460
aacaaatatt ttgcagtatg cttccaccag gccccctacc ttgtcaccaa tacctcacat 2520
tcctcgaagc ccttacaagt ttcctagttc acccttacgg attcctggag ggaacatcta 2580
tatttcaccc ctgaagagtc catataaaat ttcagaaggt ctgccaacac caacaaaaat 2640
gactccaaga tcaagaatct tagtatcaat tggtgaatca ttcgggactt ctgagaagtt 2700
ccagaaaata aatcagatgg tatgtaacag cgaccgtgtg ctcaaaagaa gtgctgaagg 2760
aagcaaccct cctaaaccac tgaaaaaact acgctttgat attgaaggat cagatgaagc 2820
agatggaagt aaacatctcc caggagagtc caaatttcag cagaaactgg cagaaatgac 2880
ttctactcga acacgaatgc aaaagcagaa aatgaatgat agcatggata cctcaaacaa 2940
ggaagagaaa tgaggatctc aggaccttgg tggacactgt gtacacctct ggattcattg 3000
tctctcacag atgtgactgt ataactttcc caggttctgt ttatggccac atttaatatc 3060
ttcagctctt tttgtggata taaaatgtgc agatgcaatt gtttgggtga ttcctaagcc 3120
acttgaaatg ttagtcattg ttatttatac aagattgaaa atcttgtgta aatcctgcca 3180
tttaaaaagt tgtagcagat tgtttcctct tccaaagtaa aattgctgtg ctttatggat 3240
agtaagaatg gccctagagt gggagtcctg ataacccagg cctgtctgac tactttgcct 3300

tcttttgtag catataggtg atgtttgctc ttgtttttat taatttatat gtatattttt 3360
ttaatttaac atgaacaccc ttagaaaatg tgtcctatct atcttccaaa tgcaatttga 3420
ttgactgccc attcaccaaa attatcctga actcttctgc aaaaatggat attattagaa 3480
attagaaaaa aattactaat tttacacatt agattttatt ttactattgg aatctgatat 3540
actgtgtgct tgttttataa aattttgctt ttaattaaat aaaagctgga agcaaagtat 3600
aaccatatga tactatcata ctactgaaac agatttcata cctcagaatg taaaagaact 3660
tactgattat tttcttcatc caacttatgt ttttaaatga ggattattga tagtactctt 3720
ggtttttata ccattcagat cactgaattt ataaagtacc catctagtac ttgaaaaagt 3780
aaagtgttct gccagatctt aggtatagag gaccctaaca cagtatatcc caagtgcact 3840
ttctaatgtt tctgggtcct gaagaattaa gatacaaatt aattttactc cataaacaga 3900
ctgttaatta taggagcctt aatttttttt tcatagagat ttgtctaatt gcatctcaaa 3960
attattctgc cctccttaat ttgggaaggt ttgtgttttc tctggaatgg tacatgtctt 4020
ccatgtatct tttgaactgg caattgtcta tttatctttt atttttttaa gtcagtatgg 4080
tctaacactg gcatgttcaa agccacatta tttctagtcc aaaattacaa gtaatcaagg 4140
gtcattatgg gttaggcatt aatgtttcta tctgattttg tgcaaaagct tcaaattaaa 4200
acagctgcat tagaaaaaga ggcgcttctc ccctccccta cacctaaagg tgtatttaaa 4260
ctatcttgtg tgattaactt atttagagat gctgtaactt aaaatagggg atatttaagg 4320
tagcttcagc tagcttttag gaaaatcact ttgtctaact cagaattatt tttaaaaaga 4380
aatctggtct tgttagaaaa caaaatttta ttttgtgctc atttaagttt caaacttact 4440
attttgacag ttattttgat aacaatgaca ctagaaaact tgactccatt tcatcattgt 4500
ttctgcatga atatcataca aatcagttag tttttaggtc aagggcttac tatttctggg 4560
tcttttgcta ctaagttcac attagaatta gtgccagaat tttaggaact tcagagatcg 4620
tgtattgaga tttcttaaat aatgcttcag atattattgc tttattgctt ttttgtattg 4680
gttaaaactg tacatttaaa attgctatgt tactattttc tacaattaat agtttgtcta 4740
ttttaaaata aattagttgt taagagtctt aa 4772

<210> 115
<211> 1260
<212> DNA
<213> human

<400> 115
   aaattgagcc cgcagcctcc cgcttcgctc tctgctcctc ctgttcgaca gtcagccgca 60
   tcttcttttg cgtcgccagc cgagccacat cgctcagaca ccatggggaa ggtgaaggtc 120
   ggagtcaacg gatttggtcg tattgggcgc ctggtcacca gggctgcttt taactctggt 180
   aaagtggata ttgttgccat caatgacccc ttcattgacc tcaactacat ggtttacatg 240
   ttccaatatg attccaccca tggcaaattc catggcaccg tcaaggctga gaacgggaag 300
   cttgtcatca atggaaatcc catcaccatc ttccaggagc gagatccctc caaaatcaag 360
   tggggcgatg ctggcgctga gtacgtcgtg gagtccactg gcgtcttcac caccatggag 420
   aaggctgggg ctcatttgca ggggggagcc aaaagggtca tcatctctgc cccctctgct 480
   gatgccccca tgttcgtcat gggtgtgaac catgagaagt atgacaacag cctcaagatc 540
   atcagcaatg cctcctgcac caccaactgc ttagcacccc tggccaaggt catccatgac 600
   aactttggta tcgtggaagg actcatgacc acagtccatg ccatcactgc cacccagaag 660
   actgtggatg gcccctccgg gaaactgtgg cgtgatggcc gcggggctct ccagaacatc 720
   atccctgcct ctactggcgc tgccaaggct gtgggcaagg tcatccctga gctgaacggg 780
   aagctcactg gcatggcctt ccgtgtcccc actgccaacg tgtcagtggt ggacctgacc 840
   tgccgtctag aaaaacctgc caaatatgat gacatcaaga aggtggtgaa gcaggcgtcg 900
   gagggccccc tcaagggcat cctgggctac actgagcacc aggtggtctc ctctgacttc 960
   aacagcgaca cccactcctc cacctttgac gctggggctg gcattgccct caacgaccac 1020
   tttgtcaagc tcatttcctg gtatgacaac gaatttggct acagcaacag ggtggtggac 1080
   ctcatggccc acatggcctc caaggagtaa gacccctgga ccaccagccc cagcaagagc 1140
   acaagaggaa gagagagacc ctcactgctg gggagtccct gccacactca gtcccccacc 1200
   acactgaatc tcccctcctc acagttgcca tgtagacccc ttgaagaggg gaggggccta 1260

<210> 116
<211> 974
<212> DNA
<213> human

<400> 116
gtttgcttag gtgaggcgca gacggggaag cggagccaac atgccagtgg cccggagctg 60
ggtttgtcgc aaaacttatg tgaccccgcg gagacccttc gagaaatctc gtctcgacca 120
agagctgaag ctgatcggcg agtatgggct ccggaacaaa cgtgaggtct ggagggtcaa 180
atttaccctg gccaagatcc gcaaggccgc ccgggaactg ctgacgcttg atgagaagga 240
cccacggcgt ctgttcgaag gcaacgccct gctgcggcgg ctggtccgca ttggggtgct 300
ggatgagggc aagatgaagc tggattacat cctgggcctg aagatagagg atttcttaga 360
gagacgcctg cagacccagg tcttcaagct gggcttggcc aagtccatcc accacgctcg 420
cgtgctgatc cgccagcgcc atatcaggta ccacctcgga tgggcacctg aatcttcctc 480
cacctgcccc tctgatggtt gccctcacta agcctgctgt ccctatctcc tatgcagccc 540
tcggaggtga tgggtgtgaa ctcacccaga gggtacagat tcacccttgc acacagctca 600
ccagggagct ggggcagcct cttgccccaa tagcccagcg caagggtcac tgcggctcta 660
gccgtacacc ttgtgaaggc ctctgccagg catgtgggca gctggacagg gtccgcaagc 720
aggtggtgaa catcccgtcc ttcattgtcc gcctggattc ccagaagcac atcgacttct 780
ctctgcgctc tccctacggg ggtggccgcc cgggccgcgt gaagaggaag aatgccaaga 840
agggccaggg tggggctggg gctggagacg acgaggagga ggattaagtc cacctgtccc 900

tcctgggctg ctggattgtc tcgttttcct gccaaataaa caggatcagc gctttaaaaa 960
aaaaaaaaaa aaaa 974